# FASCICULE DE BREVET EUROPEEN

(11) **EP 2 931 740 B1**
(45) Date de publication et mention de la délivrance du brevet: **23.08.2017**
(21) Numéro de dépôt: 13820805.3
(22) Date de dépôt: 12.12.2013
(51) Int. Cl.: C07J 1/00, C07J 41/00, C07J 43/00, C07J 51/00, G01N 33/50, G01N 33/74

(54) **DÉRIVÉS DE TESTOSTÉRONE AVEC UNE SUBSTITUTION CARBOXYALKYLE EN POSITION 3 ET LEUR UTILITISATION POUR LA PRODUCTION DES STÉROÏDES MARQUÉS POUR LA DÉTERMINATION DE LA CONCENTRATION DE TESTOSTÉRONE DANS UN ÉCHANTILLON BIOLOGIQUE**
TESTOSTERONDERIVATE MIT EINER CARBOXYALKYL-SUBSTITUTION AN POSITION 3 UND IHRE VERWENDUNG ZUR HERSTELLUNG VON MARKIERTEN STEROIDEN ZUR BESTIMMUNG DER KONZENTRATION VON TESTOSTERON IN EINER BIOLOGISCHEN PROBE
TESTOSTERONE DERIVATIVES WITH A CARBOXYALKYL SUBSTITUTION IN POSITION 3 AND USE THEREOF FOR THE PRODUCTION OF LABELLED STEROIDS FOR DETERMINING THE CONCENTRATION OF TESTOSTERONE IN A BIOLOGICAL SAMPLE

(30) Priorité: 13.12.2012 FR 1261982
(43) Date de publication de la demande: 21.10.2015
(73) Titulaire: Biomérieux, 69280 Marcy l'Étoile (FR)
(72) Inventeur: COSIN, Perrine, 69510 Thurins (FR); GUO, Yuping, 69170 Tarare (FR)
(74) Mandataire: Bitaud, Valérie Marie-Odile
(86) Numéro de dépôt international: PCT/FR2013/053040
(87) Numéro de publication internationale: WO 2014/091158

(56) Documents cités:
- FIET J ET AL: "Development of a highly sensitive and specific new testosterone time-resolved fluoroimmunoassay in human serum", STEROIDS, ELSEVIER SCIENCE PUBLISHERS, NEW YORK, NY, US, vol. 69, no. 7, 1 juillet 2004 (2004-07-01), pages 461-471, XP004520981, ISSN: 0039-128X, DOI: 10.1016/J.STEROIDS.2004.04.008
- RAJKOWSKI K M ET AL: "The conjugation of testosterone with horseradish peroxidase and a sensitive enzyme assay for the conjugate", STEROIDS, ELSEVIER SCIENCE PUBLISHERS, NEW YORK, NY, US, vol. 29, no. 5, 1 mai 1977 (1977-05-01), pages 701-713, XP023444562, ISSN: 0039-128X, DOI: 10.1016/0039-128X(77)90021-6 [extrait le 1977-05-01]
- DHAR T K ET AL: "Homogeneous enzyme immunoassay of estradiol using estradiol-3-0-carboxymethyl ether as hapten", STEROIDS, ELSEVIER SCIENCE PUBLISHERS, NEW YORK, NY, US, vol. 51, no. 5-6, 1 mai 1988 (1988-05-01), pages 519-526, XP025205931, ISSN: 0039-128X, DOI: 10.1016/0039-128X(88)90048-7 [extrait le 1988-05-01]
- FIET J ET AL: "Plasma 17-OH pregnenolone: comparison of a time-resolved fluoroimmunoassay using a new tracer 17-OH pregnenolone-3-oxyacetyl-biotine with a radioimmunoassay using <125>I 17-OH pregnenolone-3-hemisuccinate-histamine", STEROIDS, ELSEVIER SCIENCE PUBLISHERS, NEW YORK, NY, US, vol. 66, no. 2, 1 janvier 2001 (2001-01-01), pages 81-86, XP004902367, ISSN: 0039-128X, DOI: 10.1016/S0039-128X(00)00207-5

## Description

La présente invention concerne le domaine de la détection de la testostérone dans un échantillon biologique. En particulier, elle concerne de nouveaux composés dérivés de testostérone particulièrement utiles dans les immunodosages, leur procédé de préparation, ainsi que leur utilisation pour le dosage de la testostérone.

L'androgène 17 β-hydroxyandrost-4-èn-3-one, communément appelée testostérone, est l'hormone androgène principale chez l'homme. Elle est sécrétée par les cellules de Leydig dans les testicules, sous l'influence d'une hormone hypophysaire: la LH (Hormone Lutéinisante).

La testostérone circule en majeur partie liée à des protéines de transport, principalement la SHBG (Sex Hormone-Binding Globuline) et l'albumine. La testostérone libre représente 1 à 2% de la testostérone totale (Litwack G., 1992).

La formule développée de la testostérone avec la numérotation des atomes est représentée ci-dessous.

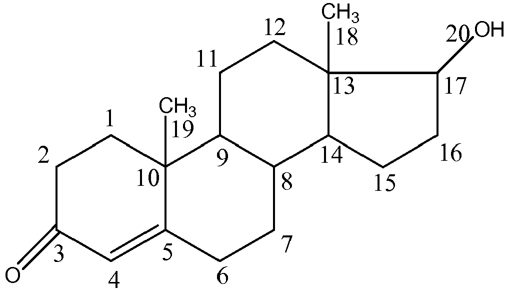

Chez l'homme, la testostérone agit sur la spermatogénèse, sur la maturation des organes génitaux externes, sur les caractères sexuels secondaires (barbe, poils pubiens et axillaires) et sur l'anabolisme protidique. Ainsi, le dosage de la testostérone chez l'homme permet d'évaluer la fonction stéroïdogène testiculaire. Un taux de testostérone sérique supérieur à 3,2 ng/mL est considéré comme normal, un taux inférieur à 2 ng/mL permet de diagnostiquer un hypogonadisme ou une féminisation de l'organisme. La limite supérieure des valeurs normales chez l'homme est 8-9 ng/mL.

Chez la femme, la testostérone est sécrétée en faible quantité par les ovaires et les glandes surrénales. Elle provient essentiellement de la conversion périphérique des précurseurs (notamment l'androstènedione). Ainsi, le dosage de la testostérone chez la femme permet d'explorer la sécrétion androgénique. Des taux élevés en testostérone peuvent être liés à un syndrome d'ovaires polykystiques, une tumeur ovarienne ou surrénalienne, une hyperplasie des surrénales ou un hirsutisme idiopathique.

Il existe plusieurs méthodes disponibles pour la quantification de la testostérone dans des échantillons biologiques. Ces méthodes peuvent être classées en 2 groupes principaux :

### (1) la spectrométrie de masse (MS)

Cette technique d'analyse permet la détermination de la masse moléculaire des composés analysés, et ainsi que leur identification et leur quantification. Appliquée à un mélange complexe comme un fluide biologique, elle nécessite d'être couplée à une technique séparative qui permet d'en réduire la complexité. Il s'agit le plus souvent de la chromatographie en phase gazeuse (GC) ou de la chromatographie en phase liquide (LC). La spectrométrie de masse en tandem (MS/MS) combine 2 analyseurs. Les composés ioniques sélectionnés dans le premier analyseur sont analysées de manière plus fine dans le second. Cette double analyse permet d'augmenter de manière significative la spécificité de la méthode. Ainsi la testostérone peut être dosée par plusieurs méthodes associant une technique séparative et une technique MS :
- chromatographie en phase gazeuse - spectrométrie de masse avec dilution isotopique (ID-GCMS) (Moneti G ; et al., 1987 ; Wudy, S.A., et al., 1992).
- chromatographie en phase liquide - spectrométrie de masse en tandem avec dilution isotopique (ID-LC-MS/MS) (Thienpont L, et al., 2008).

### (2) les immunodosages avec marqueurs

Les procédés d'immunodosage, également appelés dosages immunologiques ou tests immuno-chimiques, impliquent des réactions immunologiques entre l'analyte à détecter, la testostérone, et au moins un premier composé qui est un partenaire de liaison à cet analyte. Comme le dosage de la testostérone se fait par compétition, le procédé implique également un autre composé qui entre en compétition avec la testostérone à doser, lequel est un dérivé de testostérone. L'un des deux composés sera alors marqué pour la visualisation. On appellera ce composé marqué, conjugué marqué.

Bien entendu, le terme « immuno » dans « immunodosage » par exemple, n'est pas à considérer dans la présente demande comme indiquant strictement que le partenaire de liaison est un partenaire immunologique, tel qu'un anticorps. En effet, l'Homme du Métier utilise également largement ce terme lorsque le partenaire de liaison, aussi appelé ligand, n'est pas un partenaire immunologique mais est par exemple un récepteur à l'analyte que l'on veut doser. Ainsi, il est connu de parler du dosage ELISA (Enzyme-Linked Immunosorbent Assay) pour des dosages qui utilisent des partenaires de liaison non immunologiques, appelés plus largement en anglais « Ligand Binding Assay », que l'on pourrait traduire par « Dosage utilisant la liaison à un ligand, alors que le terme « immuno » est inclus dans l'acronyme ELISA. Par souci de clarté, la Demanderesse utilisera dans toute la demande le terme « immuno » pour tout dosage utilisant un partenaire de liaison, même quand il n'est pas un partenaire immunologique.

Selon le type de conjugué marqué qui est utilisé et le type de signal que ce conjugué peut générer, on distingue trois types d'immunodosage :
- les immunodosages radio-isotopiques (RIA) (Cekan, S. Z., 1979), qui peuvent être précédés d'une technique de séparation comme la chromatographie liquide haute performance (Ueschiba, H. et al., 1991),
- les dosages immuno-enzymatiques ou EIA « enzyme linked immunoassay ». Selon le substrat enzymatique choisi, on peut avoir un signal colorimétrique (ELISA) (Rassasie, M.J., et al., 1992), un signal de fluorescence (ELFA) ou un signal chemiluminescent (CLIA) (Stabler T.V., et al., 1991).
- les immunodosages électrochemiluminescentes (Owen, W.E., et al., 2010).

Les deux derniers types d'immunodosage sont alors appelés immunodosages non radio-isotopiques.

Le développement dans les années 60 des méthodes RIA a révolutionné la quantification des stéroïdes. Les traceurs utilisés étaient alors des dérivés de testostérone radioactifs (³H or ¹²⁵I) tels que la testostérone-3-(O-carboxyméthyl)-oxime (ou testostérone-3-O-CMO) marquée (Cook B. et Beastall G.H., 1987). Toutefois, ces méthodes avaient notamment pour inconvénient le traitement des déchets radioactifs et la durée de demi-vie des réactifs marqués relativement courte.

C'est pourquoi les procédés et réactifs d'immunodosage non-radioisotopiques se sont développés au détriment du RIA que l'on n'utilise que rarement aujourd'hui. Toutefois, les immunodosages réalisés sur les plateformes automatisées d'immunoanalyse ne sont pas capables de détecter de façon juste et reproductible des quantités de testostérone inférieures à 1,5 ng/mL en condition d'utilisation de routine (Rosner W. et al., 2007). Ceci pose un problème pour la bonne prise en charge des hommes hypogonadiques qui présentent souvent de tels taux sériques.

De même, il est également établi que la plupart des méthodes actuelles d'immunodosage non-radioisotopiques de la testostérone sont inexactes lorsqu'elles sont utilisées chez la femme car elles ne possèdent pas une sensibilité analytique suffisante pour détecter et quantifier les taux de testostérone présentes dans la circulation sanguine. Les valeurs de référence pour la testostérone totale chez les femmes âgées de 18 à 49 ans sont de 0,04 à 0,44 ng/mL (Demers L.M., 2010), alors que les limites de détection ou encore sensibilité analytique des immunodosages automatisés pour la testostérone sont de l'ordre de 0,1 ng/mL. Ainsi, Shrivastav T. G., 2002 a montré qu'un dosage par ELISA de la testostérone en utilisant comme traceur la testostérone-3-O-CMO conjuguée à la peroxydase de raifort donnait un biais lorsque la quantité de testostérone dans l'échantillon à doser était faible, en ce sens que la concentration obtenue était toujours largement supérieure à celle réellement contenue dans l'échantillon dosé. Fiet J et al., 2004 ont également décrit l'utilisation d'un tel composé 3-O-CMO, également appelé composé 3-CMO, en tant traceur, lequel a été préalablement biotinylé. Toutefois, la sensibilité de dosage de testostérone dans les gammes les plus faibles en utilisant ce composé n'est pas bonne. De plus, un tel composé 3-O-CMO est compliqué à produire dans la mesure où il se trouve sous forme d'un mélange de stéréoisomères, également appelé mélange isomérique. Pour avoir un dosage reproductible, il est alors recommandé d'effectuer une séparation des stéréoisomères avant utilisation dans l'immunodosage, ce qui est compliqué et entraîne une dégradation du rendement de synthèse.

Il existe donc un besoin urgent d'un dosage de testostérone qui soit suffisamment sensible et donc cliniquement utilisable dans les échantillons contenant une concentration faible en testostérone et utilisant des composés pouvant être préparés de façon simplifiée.

La Demanderesse a trouvé, contre toute attente, de nouveaux dérivés de testostérone qui permettent de pallier les inconvénients des immunodosages de l'art antérieur en ce sens que leur utilisation dans un dosage immunologique par compétition, notamment en tant que traceur, permettait d'avoir un dosage hautement sensible et en particulier dans les gammes les plus faibles de testostérone, et dont la préparation est aisée.

Ainsi, un objet de l'invention concerne des composés dérivés de la testostérone de formule générale (I) suivante : 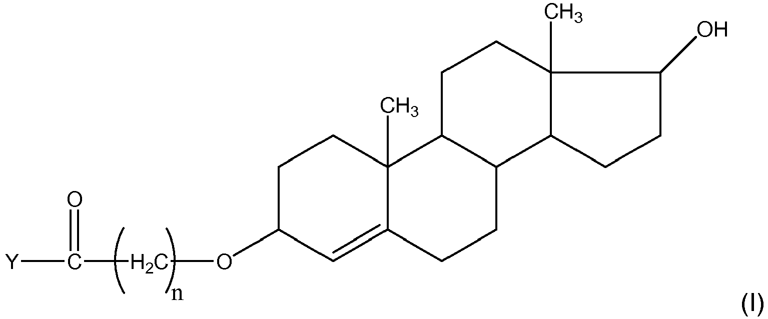 dans laquelle n est un nombre entier compris entre 1 et 10 et Y représente un groupement de couplage activé ou prêt à être activé qui permet la formation d'une liaison amide avec une amine primaire d'une molécule.

Un autre objet de l'invention comprend un conjugué comprenant ou constitué d'un dérivé de testostérone de formule (I) telle que décrite ci-dessus et d'un marqueur, ainsi qu'un kit comprenant de tels composés.

Un autre objet de l'invention comprend l'utilisation dudit conjugué dans un procédé de dosage immunologique de la testostérone, notamment en tant que traceur.

Enfin, un dernier objet concerne un procédé de préparation desdits dérivés de testostérone.

La présente invention concerne donc les composés de formule (I) tels que décrits précédemment, lesquels sont particulièrement utiles pour un test d'immunodosage par compétition, et notamment pour la préparation de traceur.

En effet, la Demanderesse a trouvé contre toute attente que la modification de la testostérone en position 3, par réduction du carbonyle en hydroxyle et introduction d'un bras, permettait de donner un dérivé de la testostérone n'étant pas sous forme de mélange isomérique et étant particulièrement avantageux quand utilisé dans un dosage de la testostérone, et notamment en tant que traceur, en ce sens que le dosage de testostérone est particulièrement sensible et permet de détecter des concentrations de testostérone faibles.

Comme montré par Cook B. et Beastall G.H, 1987, les hormones stéroïdiennes sont structurellement très proches. Ainsi, la testostérone, l'estradiol, l'estrone et l'androstènedione ne diffèrent les uns des autres que par la présence d'une fonction hydroxyle ou cétone dans les positions 3 et 17, ainsi que par la présence ou non d'un groupement méthyle en position 10, comme montré ci-après :

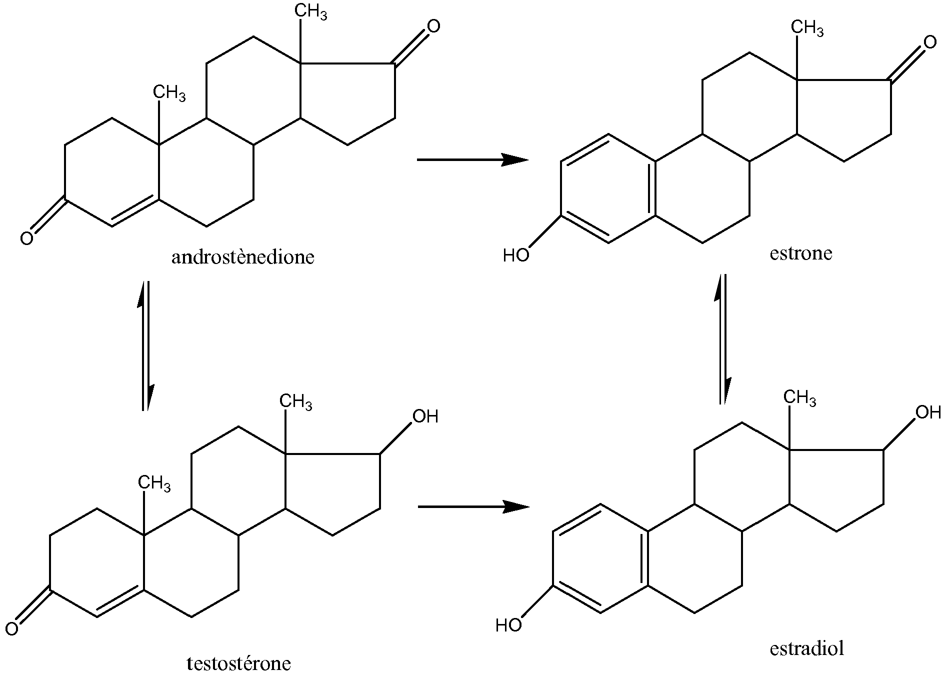

La moindre modification des substituants de la structure de base, et notamment en position 3 ou position 17, fait passer d'une hormone stéroïdienne à une autre.

Contre toute attente, les composés de formule (I) de l'invention, malgré la modification de la structure chimique de base de la testostérone, du fait de la réduction du carbonyle en hydroxyle en position 3 et donc de sa ressemblance structurelle au niveau du groupement fonctionnel en cette position avec l'estradiol, analyte qu'on ne veut pas doser, sont particulièrement bien adaptés pour leur utilisation pour le dosage de la testostérone.

Les composés de l'invention sont donc caractérisés par la formule (I) telle que donnée ci-dessus, dans laquelle n est un nombre entier compris entre 1 et 10 et Y représente un groupement de couplage activé ou prêt à être activé qui permet la formation d'une liaison amide avec une amine primaire d'une molécule.

Ces composés ne doivent pas être trop hydrophobes et la valeur de n doit être contrôlée à cet égard. Selon un mode de réalisation, n est compris entre 1 et 5 et de préférence entre 1 et 3. Selon un mode de réalisation particulier, n est 1.

Les molécules qui peuvent être couplées avec les dérivés de testostérone de l'invention sont toutes molécules qui possèdent naturellement une amine primaire, telles qu'une protéine, ou bien toutes molécules qui ont été modifiées chimiquement pour inclure une telle amine primaire, par exemple une biotine modifiée, présentant une amine primaire.

Par groupement de couplage activé ou prêt à être activé, on entend un groupement fonctionnel adapté, le cas échéant après activation, pour permettre le couplage du dérivé de testostérone de l'invention avec le groupement fonctionnel amine primaire de la molécule de couplage. Ce groupement peut être aussi appelé groupement partant car il n'existera plus dans le conjugué formé entre le dérivé de testostérone et la molécule à amine primaire. Ce groupement partant sera ainsi dissocié du reste de la molécule lors de la réaction de substitution nucléophile avec le groupement nucléophile amine primaire de la molécule de couplage d'intérêt. Le groupement partant sera dissocié directement lorsqu'il est dit déjà activé, ou bien il ne sera dissocié qu'après activation lorsqu'il est dit prêt à être activé.

Par groupement de couplage activé, on entend donc un groupement qui permet de former directement une liaison amide, sans que ce groupement ait besoin d'être modifié. A titre d'exemple, on peut citer les groupements suivants : 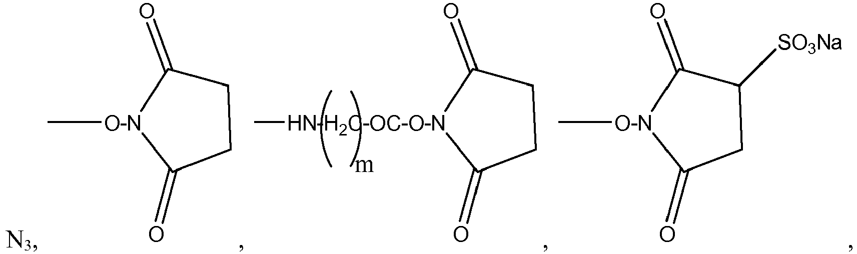 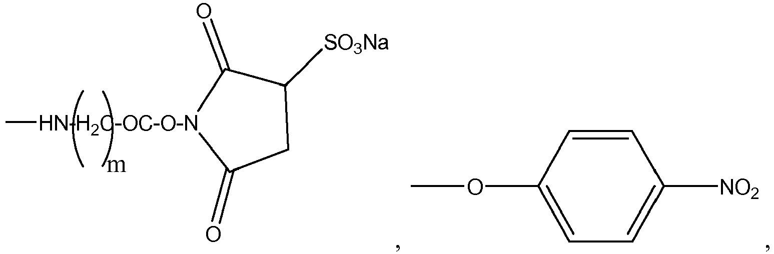 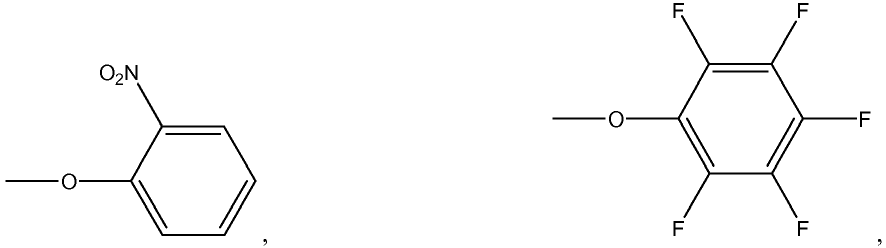 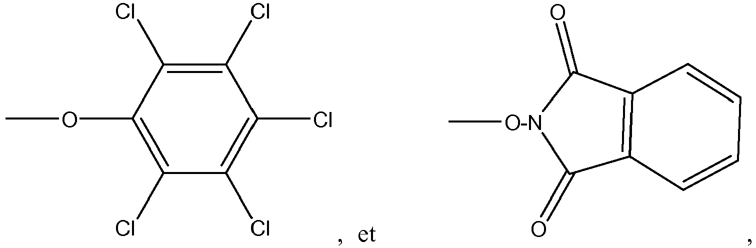 m étant un nombre entier, de préférence compris entre 1 et 10, ce qui constitue un mode de réalisation de l'invention.

Par groupement de couplage prêt à être activé, on entend donc tout groupement qui doit être activé, par des méthodes connues de l'homme du métier, pour être capable de former une liaison amide. De tels groupements possèdent un groupe -OH ou - COOH. A titre d'exemples, on peut citer les groupements -OH et -NH-(CH₂)ₘ-COOH, m étant un nombre entier, de préférence compris entre 1 et 10, ce qui constitue un mode de réalisation de l'invention.

Selon un mode de réalisation particulier de l'invention, m est compris entre 1 et 5, ou encore entre 1 et 3.

Selon un autre mode de réalisation, Y est choisi parmi -OH, -NH-(CH₂)m-COOH, et 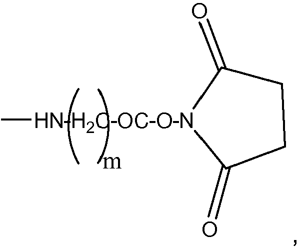 m étant compris entre 1 et 10 et de préférence étant égal à 1.

Les composés particulièrement appropriés pour le dosage de la testostérone, et en particulier pour la préparation de traceur, sont les composés stéréochimiquement purs (isomères α ou β) et ce au niveau du carbone en position 3, puisqu'il n'y a plus de double liaison en position 3, comme dans la testostérone.

En effet, en comparaison avec un composé 3-O-CMO où il est plus difficile de contrôler et de reproduire le mélange isomérique, car le ratio entre les isomères ne sont pas identiques entre les lots de synthèse, les composés stéréochimiquement purs (isomères α ou β) au niveau du carbone 3 sont avantageux en ce sens qu'ils sont davantage reproductibles, de meilleure qualité, et en ce que le dosage dans lequel ils sont utilisés sont plus robustes.

Les dérivés de testostérone de l'invention de formule (I) sous forme d'isomère β en position 3 sont représentés par la formule (Iβ) suivante : 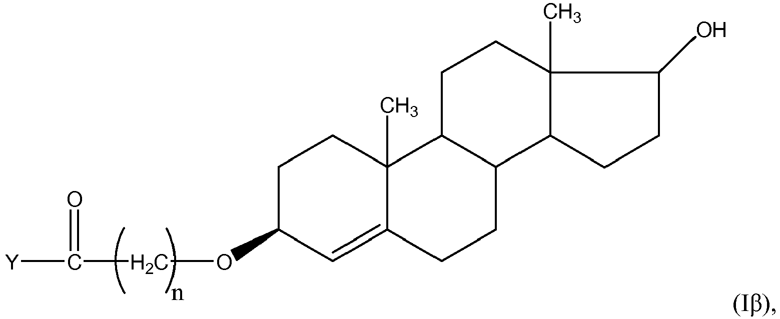 Y et n étant tels que décrits précédemment.

Les dérivés de testostérone de l'invention de formule (I) sous forme d'isomère α en position 3 sont représentés par la formule (Iα) suivante : 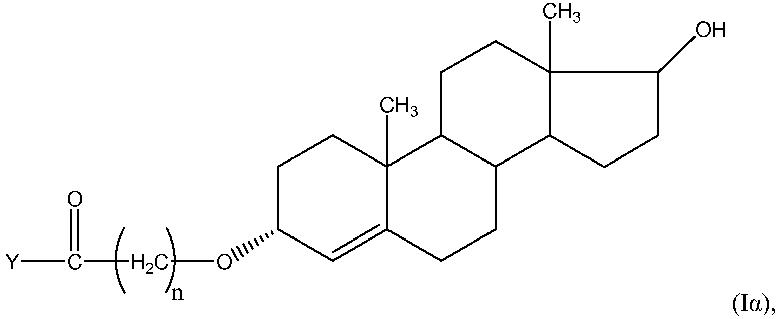 Y et n étant tels que décrits précédemment

En d'autres termes, dans la formule (I), la liaison entre le carbone en position 3 et l'atome d'oxygène du bras, représenté sous forme de segment de droite comme suit : 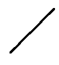 , inclut à la fois une liaison de type β et une liaison de type α.

Ceci sera également applicable à tous les composés décrits ci-après qui comprennent une liaison, entre le carbone en position 3 et l'atome d'oxygène du bras dudit composé, sous forme de segment de droite, et non sous forme de flèche comme suit : 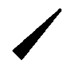 , pour désigner un isomère β, ou 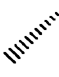 , pour désigner un isomère α.

Cela sera notamment applicable pour les composés utilisés lors de la description des procédés de préparation des dérivés de testostérone de l'invention pour lesquels il n'est pas ajouté le sigle α ou β avec le chiffre désignant la formule.

Les composés de l'invention peuvent être utilisés de deux façons dans les procédés de dosage de la testostérone par immunodosage par compétition, tels que les tests diagnostiques. Soit ils sont utilisés tels quels, soit ils sont utilisés couplés à une autre molécule pour former un conjugué. Ladite autre molécule est soit un marqueur, soit un bras chimique ou « linker », soit un composé chimique dont le couplage avec un dérivé de testostérone présente un intérêt pour la mise en oeuvre d'un dosage de la testostérone par immunodosage par compétition.

Ainsi, la présente invention a également pour objet les conjugués comprenant ou constitués d'un dérivé de testostérone tels que décrits précédemment et d'une autre molécule, en particulier d'un marqueur ou d'un bras chimique.

Par marqueur, on entend toute molécule contenant un groupement amine primaire, directement sans modification chimique, ou après modification chimique pour inclure un tel groupement, laquelle molécule est capable de générer directement ou indirectement un signal détectable. Une liste non limitative de ces marqueurs de détection directe consiste en :
- les enzymes qui produisent un signal détectable par exemple par colorimétrie, fluorescence, luminescence, comme la peroxydase de raifort, la phosphatase alcaline, la β-galactosidase, la glucose-6-phosphate déshydrogénase,
- les chromophores comme les composés fluorescents, luminescents, colorants,
- les molécules radioactives comme le ³²P, le ³⁵S ou le ¹²⁵I, et
- les molécules fluorescentes telles que les Alexa ou les phycocyanines.
- Les sels électrochimiluminescentes tels que des dérivés organo-métalliques à base d'acridinium ou de ruthénium.

Des systèmes indirects de détection peuvent être aussi utilisés, comme par exemple des ligands capables de réagir avec un anti-ligand. Le ligand correspond alors au marqueur pour constituer, avec le dérivé de testostérone, le conjugué de l'invention.

Les couples ligand/anti-ligand sont bien connus de l'homme du métier, ce qui est le cas par exemple des couples suivants : biotine/streptavidine, haptène/anticorps, antigène/anticorps, peptide/anticorps, sucre/lectine, polynucléotide/complémentaire du polynucléotide.

L'anti-ligand peut alors être détectable directement par les marqueurs de détection direct décrits précédemment ou être lui-même détectable par un autre couple ligand/anti-ligand, et ainsi de suite.

Ces systèmes indirects de détection peuvent conduire, dans certaines conditions, à une amplification du signal. Cette technique d'amplification du signal est bien connue de l'homme du métier, et l'on pourra se reporter aux demandes de brevet antérieures FR98/10084 ou WO-A-95/08000 de la Demanderesse.

Selon le type de marquage utilisé, l'homme du métier ajoutera des réactifs permettant la visualisation du marquage ou l'émission d'un signal détectable par tout type d'appareil de mesure approprié comme par exemple un spectrophotomètre, un spectrofluorimètre ou encore une caméra haute définition.

Par bras chimique ou « linker », on entend toute molécule contenant un groupement amine primaire, directement sans modification chimique, ou après modification chimique pour inclure un tel groupement, laquelle molécule est capable de fixation sur une phase solide, de manière covalente ou non-covalente, de manière sélective ou non-sélective.

Comme indiqué précédemment, les dérivés de testostérone ou les conjugués de l'invention sont particulièrement utiles pour la détermination *in vitro* de la concentration en testostérone dans un échantillon biologique, ce qui constitue un autre objet de l'invention.

L'échantillon biologique dans lequel le procédé de l'invention peut être mis en oeuvre est tout échantillon biologique animal, de préférence humain, susceptible de contenir de la testostérone, dans lequel un immunodosage peut être mis en oeuvre. Ces échantillons sont largement connus de l'homme du métier. Les échantillons mis en oeuvre dans le procédé de dosage peuvent être préalablement modifiés ou non avant leur utilisation. A titre d'exemple de tels échantillons non préalablement modifiés, on peut citer les fluides biologiques tels que sang total, urine, liquide céphalo-rachidien, liquide séminal, sperme, les leucorrhées (sécrétions vaginales), glaire cervicale, lavage cervicovaginal, lavage rectal, salive, lavage broncho-alvéolaire et liquide amniotique. A titre d'exemples d'échantillon préalablement modifié, également appelé dérivé d'échantillon, on peut citer le sérum, le plasma, les cellules que l'on récupère à partir d'une biopsie ou suite à une chirurgie et qu'on met en culture *in vitro.* Le dosage de la concentration de testostérone pourra alors être réalisé dans le surnageant de culture ou encore dans le lysat cellulaire.

La détermination de la concentration en testostérone dans l'échantillon biologique peut être mis en oeuvre par un procédé d'immunodosage, également appelé dosage immunologique, par compétition, lequel comprend les étapes consistant à :
a) mettre en présence, au sein dudit échantillon, (i) un partenaire de liaison de la testostérone et (ii) un composé choisi parmi un dérivé de testostérone et un conjugué tels que décrits précédemment, l'un desdits composants (i) et (ii) étant adapté pour émettre un signal,
b) éventuellement laisser un laps de temps suffisant pour permettre la réaction de compétition et
c) mesurer l'intensité du signal et en déduire la concentration en testostérone présente dans ledit échantillon biologique par comparaison à une courbe de calibration établissant une relation entre intensité du signal mesurée et concentration en testostérone.

Le dosage immunologique par compétition est un dosage largement connu de l'homme du métier. Il consiste à doser l'analyte, ici la testostérone, dans l'échantillon, en créant une compétition entre l'analyte de l'échantillon et un dérivé de cet analyte, ici le dérivé de testostérone. La réaction immunologique est alors mise en évidence grâce à la présence d'un traceur.

Le dérivé de l'analyte peut être utilisé dans la réaction de compétition, comme indiqué précédemment, sans couplage préalable ou après couplage à un marqueur pour former un conjugué ou traceur.

Le dosage immunologique nécessite également la mise en oeuvre d'un partenaire de liaison de la testostérone vis-à-vis duquel le dérivé d'analyte et l'analyte entrent en compétition. Lorsque le dérivé d'analyte n'est pas couplé à un marqueur (il n'est pas le traceur mais le partenaire de capture), le partenaire de liaison est alors marqué pour constituer le traceur de la réaction. Lorsque le dérivé d'analyte est couplé à un marqueur (il est alors le traceur), le partenaire de liaison devient alors le partenaire de capture.

Le signal mesuré émis par le traceur est alors inversement proportionnel à la quantité de testostérone de l'échantillon.

Par un partenaire de liaison de la testostérone, on entend toute molécule capable de se lier à la testostérone. A titre d'exemple de partenaire de liaison de la testostérone, on peut citer les anticorps, les fractions d'anticorps, les nanofitines, les récepteurs à la testostérone ou toute autre protéine qui est connue pour avoir une interaction avec la testostérone, comme par exemple la SHBG.

Les anticorps partenaires de liaison sont par exemple soit des anticorps polyclonaux, soit des anticorps monoclonaux.

Les anticorps polyclonaux peuvent être obtenus par immunisation d'un animal avec, comme immunogène, la testostérone cible, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixé un antigène spécifiquement reconnu par les anticorps, notamment l'immunogène.

Les anticorps monoclonaux peuvent être obtenus par la technique des hybridomes largement connue de l'homme du métier. Les anticorps monoclonaux peuvent être également des anticorps recombinants obtenus par génie génétique, par des techniques bien connues de l'homme du métier.

A titre d'exemple de fragments d'anticorps, on peut citer les fragments Fab, Fab', F(ab')₂ ainsi que les chaînes scFv (Single chain variable fragment), dsFv (Double-stranded variable fragment). Ces fragments fonctionnels peuvent notamment être obtenus par génie génétique.

Les nanofitines (nom commercial) sont de petites protéines qui, comme les anticorps, sont capables de se lier à une cible biologique permettant ainsi de la détecter, de la capturer ou tout simplement de la cibler au sein d'un organisme.

Les partenaires de liaison utilisés peuvent être spécifiques ou non de la testostérone. Ils sont dits spécifiques quand ils sont capables de se lier de façon exclusive ou quasi exclusive à la testostérone. Ils sont dits non spécifiques lorsque la sélectivité de liaison la testostérone est faible et qu'ils sont alors capables de se lier à d'autres ligands, tels que d'autres protéines ou anticorps. Selon un mode de réalisation préféré, on préfère les partenaires de liaison spécifiques.

Les partenaires de liaison ou les dérivés de testostérone, lorsqu'ils sont utilisés en capture, peuvent être liés ou non à un support par toute technique largement connue de l'homme du métier.

La deuxième étape b) du procédé de l'invention est une étape classique d'un procédé de dosage par compétition.

La dernière étape c) du procédé de l'invention consiste à déterminer la concentration en testostérone. Comme indiqué précédemment, le signal mesuré est inversement proportionnel à la quantité de testostérone de l'échantillon. Pour déterminer la concentration, le signal est comparé à une courbe de calibration obtenue au préalable par des techniques largement connues de l'homme du métier. Ainsi, par exemple, la courbe de calibration est obtenue en effectuant un dosage immunologique utilisant le même partenaire de liaison ainsi que des quantités croissantes de testostérone. Une courbe est ainsi obtenue en mettant en abscisse la concentration en testostérone et en ordonnée le signal correspondant obtenu après dosage immunologique.

Lorsque le composé (ii) est un conjugué de l'invention tel que décrit précédemment, ce qui constitue un mode de réalisation de l'invention, le signal est obtenu par l'intermédiaire du marquage par le marqueur comme décrit précédemment.

Lorsque le composé (ii) est un dérivé de testostérone de l'invention tel que décrit précédemment, c'est-à-dire qu'il n'est pas lié à un marqueur, le signal consiste en la lecture directe de la liaison partenaire de liaison/dérivé de testostérone, qui peut se faire par exemple par résonance plasmonique de surface ou par voltamétrie cyclique.

Pour mettre en oeuvre le procédé de détermination de la concentration de testostérone, les dérivés de testostérone ou conjugués de l'invention tels que décrits précédemment sont incorporés dans une trousse de diagnostic, ce qui constitue un autre objet de l'invention. Bien entendu, la trousse peut comprendre d'autres constituants nécessaires pour la mise en oeuvre du dosage immunologique, tels que, par exemple, au moins un partenaire de liaison, un moyen de calibration, des tampons de lavage et des réactifs permettant la visualisation du marquage ou l'émission d'un signal détectable.

Les dérivés de testostérone ont été préparés par des procédés particuliers permettant de synthétiser des composés qui sont soit des isomères purs α, soit des isomères purs β. Ces procédés sont nouveaux et constituent un autre objet de l'invention.

Les procédés de préparation de tous les dérivés de testostérone de l'invention de formule (I) passent par la préparation d'un premier composé clé qui est un composé de formule (Ia) comme suit : 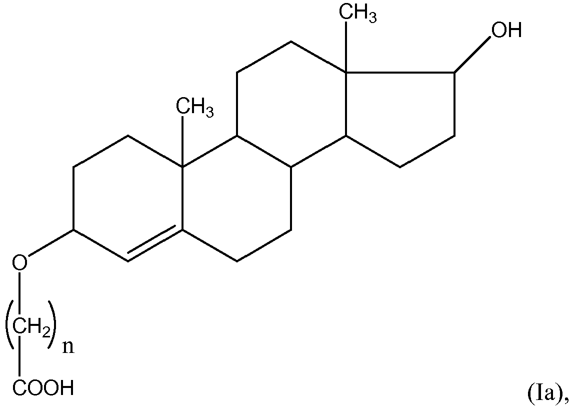 dans laquelle n est tel que défini précédemment. Aussi, son utilisation pour la préparation d'un composé de formule (I) telle que définie précédemment constitue un autre objet de l'invention.

Le composé de formule (Ia) est en fait un dérivé de testostérone de formule (I) dans laquelle Y est le groupement -OH. Ce dérivé de testostérone peut être sous forme d'isomère β en position 3 ; il possède alors la formule (Iaβ) suivante : 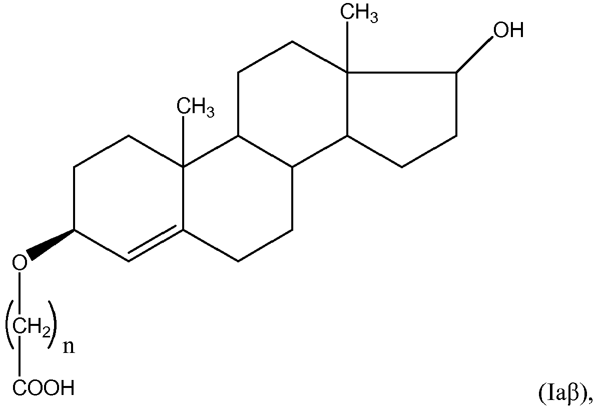 n étant tel que défini précédemment.

Aussi, un autre objet de l'invention consiste en un procédé de préparation d'un dérivé de testostérone, sous forme d'isomère β en position 3, de formule générale (Iaβ), lequel comprend ou consiste en les étapes consistant à :
(1) mettre à réagir la testostérone avec de l'anhydride acétique pour protéger la fonction OH de la position 17, puis avec un agent réducteur, en présence d'un solvant, pour réduire le carbonyle en position 3 et obtenir le composé de formule (IIβ) : 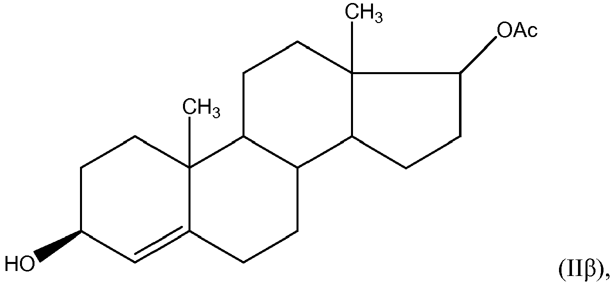 dans laquelle Ac signifie -CO-CH₃,
(2) mettre à réagir le composé de formule (IIβ) ainsi obtenu avec le composé de formule (III) : N₂CH-(CH₂)ₙ₋₁-COOC₂H₅, puis mettre à réagir avec une base, en présence d'un solvant, pour obtenir le composé de formule (Iaβ).

Le dérivé de testostérone de formule (I) dans laquelle Y est -OH peut également être sous forme d'isomère α en position 3 ; il possède alors la formule (Iaα) suivante : 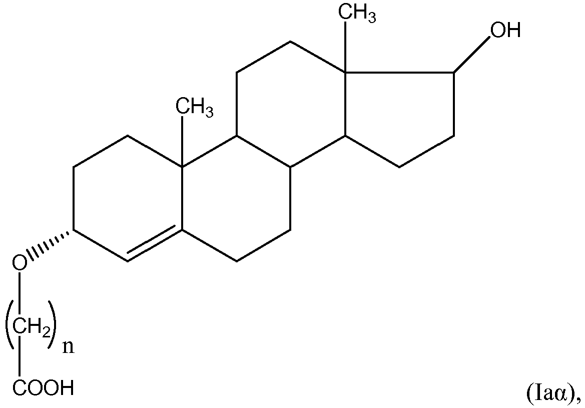 dans laquelle n est tel que décrit précédemment.

Encore un autre objet de l'invention concerne un procédé de préparation d'un dérivé de testostérone, sous forme d'isomère α en position 3, de formule générale (Iaα), lequel procédé comprend ou consiste en les étapes consistant à :
(1) mettre à réagir la testostérone avec du t-butyldiméthylchlorosilane pour protéger la fonction OH de la position 17, puis avec un agent réducteur, en présence d'un solvant, pour réduire le carbonyle 3 et obtenir le composé de formule (VIIIβ) : 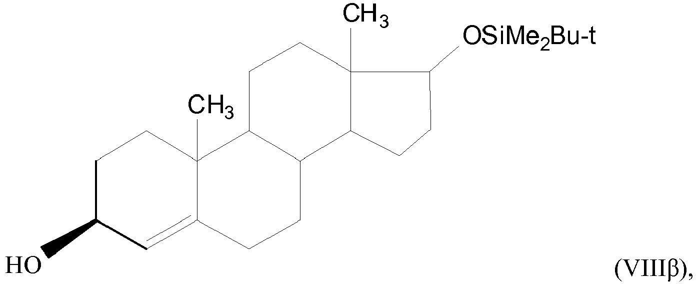 dans laquelle -SiMe₂Bu-t signifie t-butyldiméthylsilanyle,
(2) mettre à réagir le composé de formule (VIIIβ) ainsi obtenu avec de la triphénylphosphine, de l'acide benzoïque et de l'azodicarboxylate de diéthyle, puis avec une base, en présence d'un solvant, pour transformer, en position 3, l'isomère β en isomère α et obtenir ainsi le composé de formule (VIIIα) : 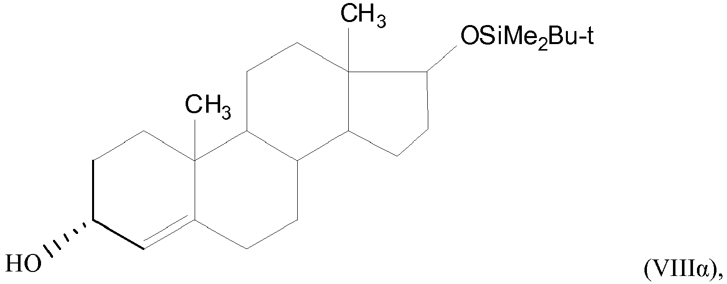 dans laquelle -SiMe₂Bu-t signifie t-butyldiméthylsilanyle, et
(3) mettre à réagir le composé de formule (VIIIα) ainsi obtenu avec le composé de formule (III) : N₂CH-(CH₂)ₙ₋₁-COOC₂H₅, puis mettre à réagir avec une base, en présence d'un solvant, pour obtenir le composé de formule (Iaα).

Les agents réducteurs que l'on peut utiliser ici sont tout agent réducteur connue de l'homme du métier pour réduire stéréosélectivement un carbonyle en hydroxyle. A titre d'exemple, on peut citer le borohydrure de sodium.

Les solvants que l'on peut utiliser ici sont tous solvants qui permettent d'avoir une réaction qui inverse la stéréochimie de l'hydroxyle. A titre d'exemple, on peut citer les alcools tels que le méthanol et l'éthanol.

Les bases que l'on peut utiliser ici sont toutes bases minérales qui permettent la saponification pour le retour à un groupement -OH. A titre d'exemple, on peut citer l'hydroxyde de sodium et l'hydroxyde de potassium.

Comme indiqué précédemment, les composés de formule (Iaβ) ou (Iaα) sont clés pour préparer d'autres dérivés de testostérone de l'invention. Ainsi, par exemple, dans la formule (I), quand Y représente -NH-(CH₂)m-COOH, les dérivés de testostérone étant alors des composés de formules (Ibβ) ou (Ibα) suivantes selon que ce sont respectivement des isomères β ou α en position 3 : 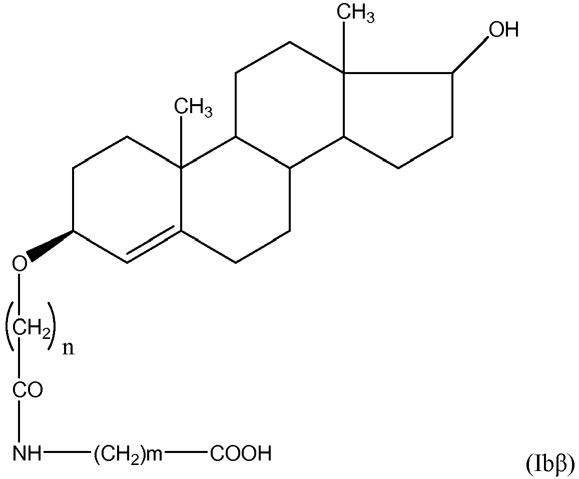 et 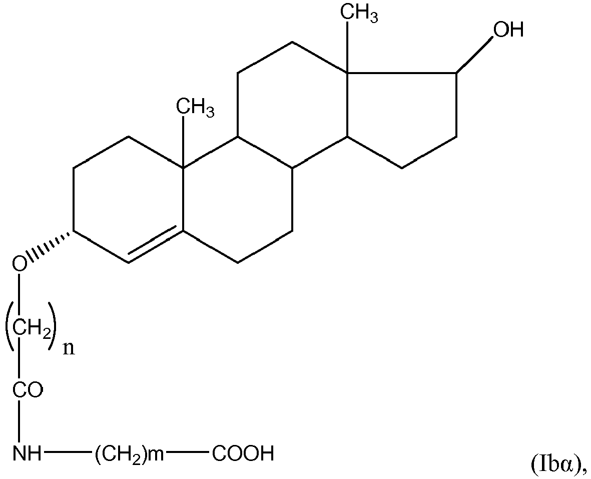 n et m étant tels que définis précédemment, les procédés de préparation comprennent les étapes précédentes (1) et (2) pour les isomères β et (1) à (3) pour les isomères α, ainsi que les 3 étapes suivantes :
1. mettre à réagir le composé de formule (Iaβ) ou (Iaα) ainsi obtenu avec du N-hydroxysuccinimide, en présence d'un dérivé de carbodiimide, pour produire le composé de formule (IV) : 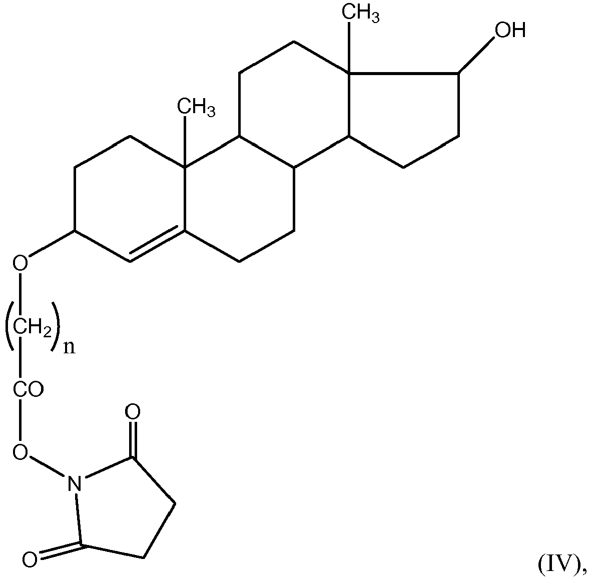
2. mettre à réagir le composé de formule (IV) ainsi obtenu avec le composé de formule (V) : H₂N-(CH₂)m-COOR₁, dans laquelle R₁ est un groupement alkyle ou aryle, pour produire le composé de formule (VI) : 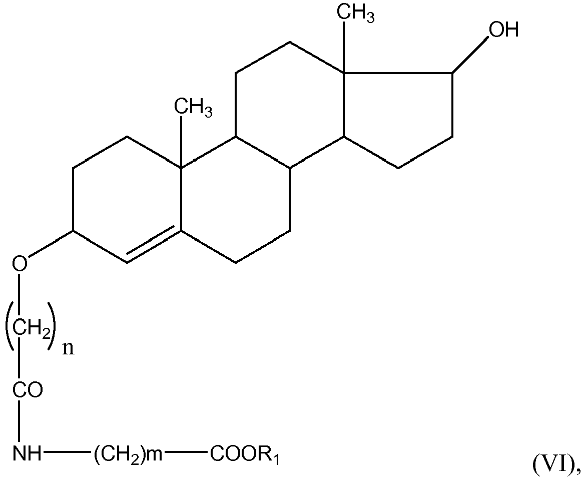
3. mettre à réagir le composé de formule (VI) ainsi obtenu avec une base, en présence d'un solvant, pour obtenir le composé de formule (Ibβ) ou (Ibα).

Ainsi, selon un autre objet, l'invention comprend un procédé de préparation d'un dérivé de testostérone, sous forme d'isomère β en position 3, de formule générale (Ibβ) suivante : 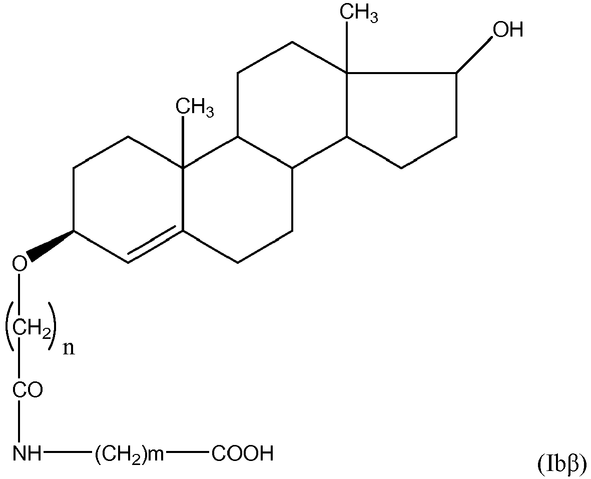 dans laquelle n est un nombre entier compris entre 1 et 10, m est un nombre entier compris entre 1 et 10, comprenant ou consistant en les étapes consistant à :
(1) mettre à réagir la testostérone avec de l'anhydride acétique pour protéger la fonction OH de la position 17, puis avec un agent réducteur, en présence d'un solvant, pour réduire le carbonyle en position 3 et obtenir le composé de formule (IIβ) : 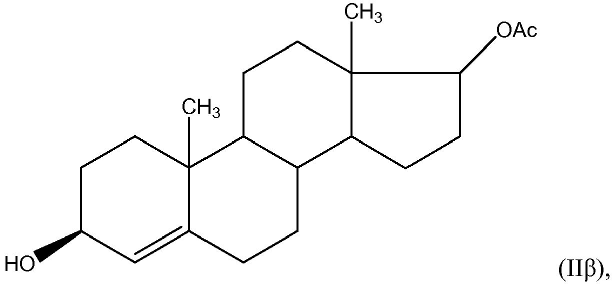 dans laquelle Ac signifie -CO-CH₃,
(2) mettre à réagir le composé de formule (IIβ) ainsi obtenu avec le composé de formule (III) : N₂CH-(CH₂)ₙ₋₁-COOC₂H₅, puis mettre à réagir avec une base, en présence d'un solvant, pour obtenir le composé de formule (Iaβ) : 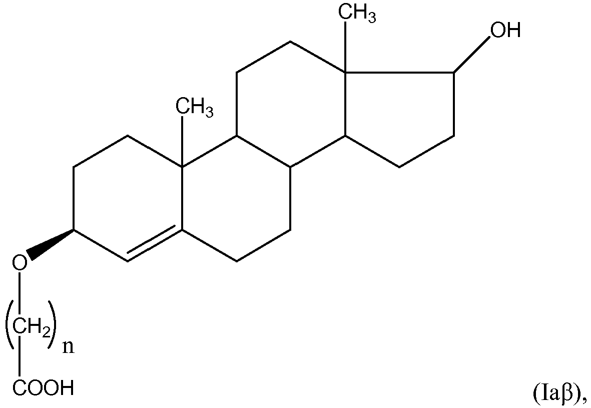
(3) mettre à réagir le composé de formule (Iaβ) ainsi obtenu avec du N-hydroxysuccinimide, en présence d'un dérivé de carbodiimide, pour produire le composé de formule (IVβ) : 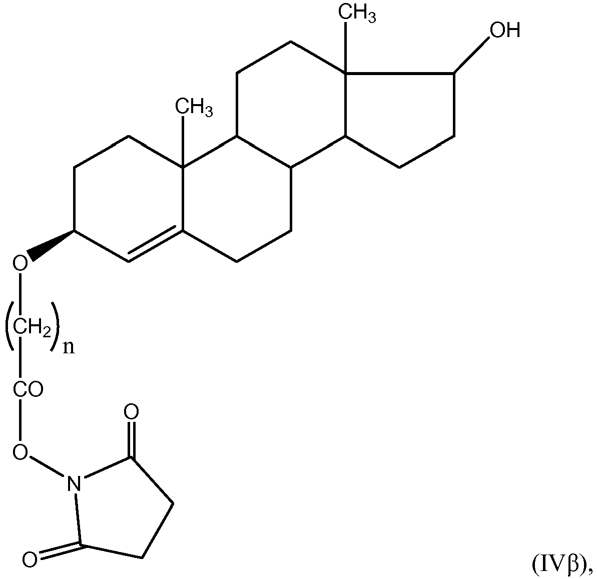
(4) mettre à réagir le composé de formule (IVβ) ainsi obtenu avec le composé de formule (V) : H₂N-(CH₂)m-COOR₁, dans laquelle R₁ est un groupement alkyle ou aryle, pour produire le composé de formule (VIβ) : 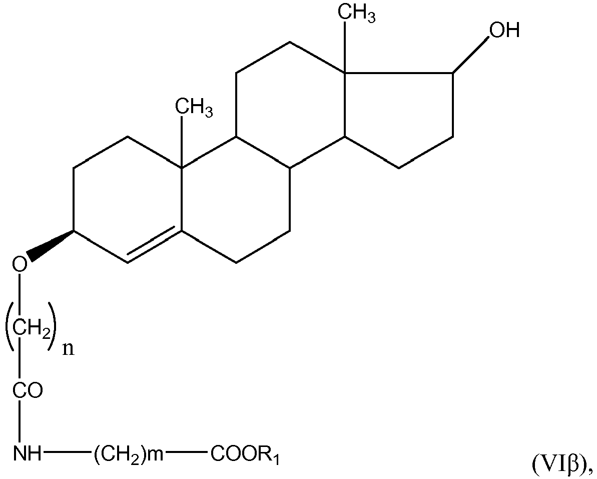
(5) mettre à réagir le composé de formule (VIβ) ainsi obtenu avec une base, en présence d'un solvant, pour obtenir le composé de formule (Ibβ).

Selon encore un autre objet, l'invention comprend un procédé de préparation d'un dérivé de testostérone, sous forme d'isomère α en position 3, de formule générale (Ibα) suivante : 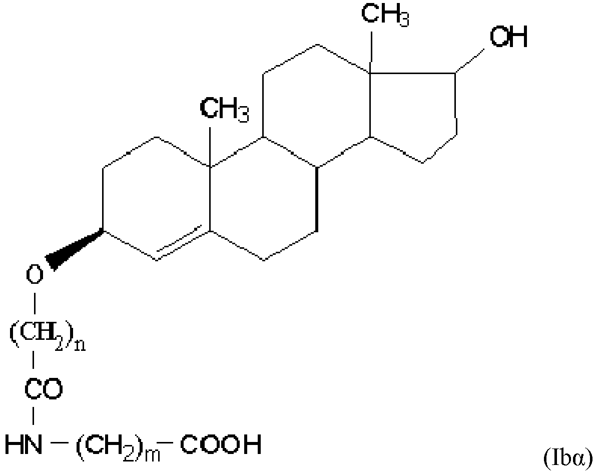 dans laquelle n est un nombre entier compris entre 1 et 10, m est un nombre entier compris entre 1 et 10, comprenant ou consistant en les étapes consistant à :
(1) mettre à réagir la testostérone avec du t-butyldiméthylchlorosilane pour protéger la fonction OH de la position 17, puis avec un agent réducteur, en présence d'un solvant, pour réduire le carbonyle en position 3 et obtenir le composé de formule (VIIIβ) : 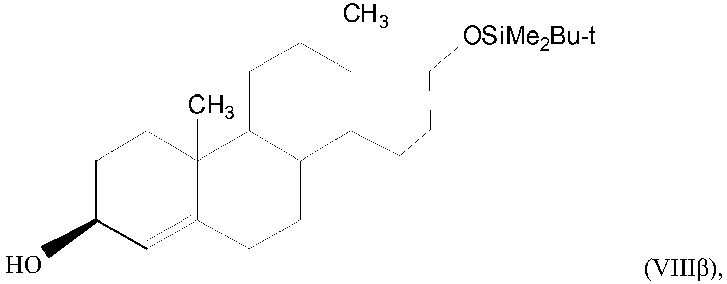 dans laquelle -SiMe₂Bu-t signifie t-butyldiméthylsilanyle,
(2) mettre à réagir le composé de formule (VIIIβ) ainsi obtenu avec de la triphénylphosphine, de l'acide benzoïque et de l'azodicarboxylate de diéthyle, puis avec une base, en présence d'un solvant, pour transformer, en position 3, l'isomère β en isomère α et obtenir ainsi le composé de formule (VIIIα) : 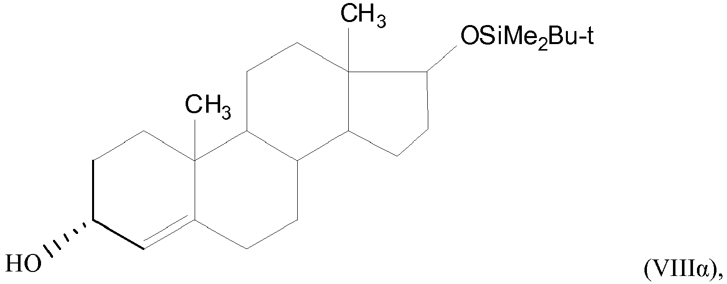 dans laquelle -SiMe₂Bu-t signifie t-butyldiméthylsilanyle,
(3) mettre à réagir le composé de formule (VIIIα) ainsi obtenu avec le composé de formule (III) : N₂CH-(CH₂)ₙ₋₁-COOC₂H₅, puis mettre à réagir avec une base, en présence d'un solvant, pour obtenir le composé de formule (Iaα) : 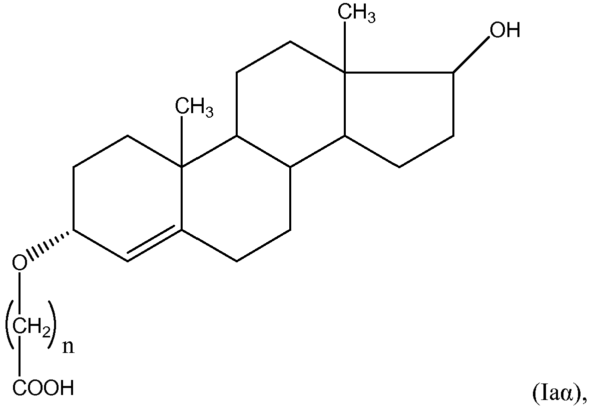
(4) mettre à réagir le composé de formule (Iaα) ainsi obtenu avec du N-hydroxysuccinimide, en présence d'un dérivé de carbodiimide, pour produire le composé de formule (IVα) : 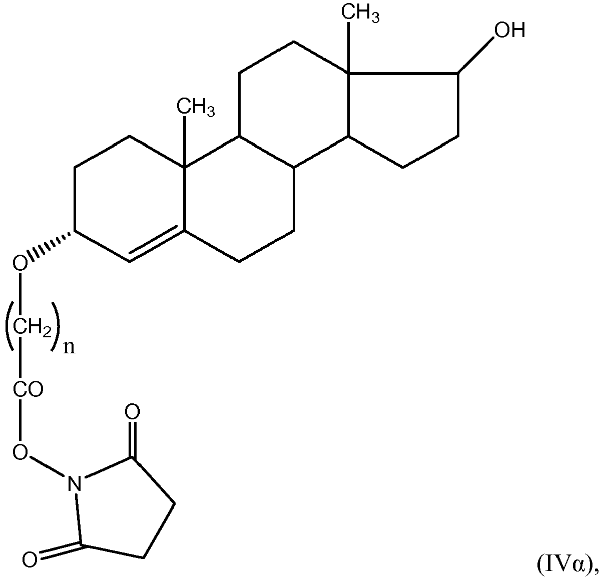
(5) mettre à réagir le composé de formule (IVα) ainsi obtenu avec le composé de formule (V) : H₂N-(CH₂)m-COOR₁, dans laquelle R₁ est un groupement alkyle ou aryle, pour produire le composé de formule (VIα) : 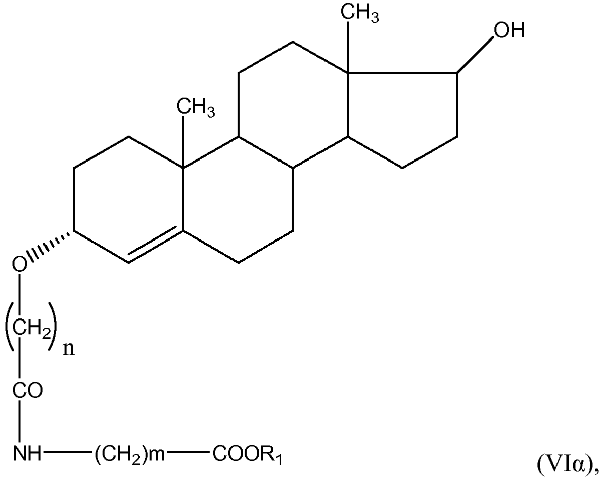
(6) mettre à réagir le composé de formule (VIα) ainsi obtenu avec une base, en présence d'un solvant, pour obtenir le composé de formule (Ibα).

Les bases et solvants utilisés dans les étapes supplémentaires sont tels qu'indiqués précédemment.

Le N-hydroxysuccinimide, de formule 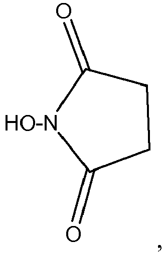 qui, lorsqu'il perd son hydrogène au niveau du groupement hydroxyle, devient un groupement activé Y, permet l'activation en groupement activé du groupement prêt à être activé Y = -OH du composé de formule (Ia), en présence d'un dérivé de carbodiimide. Ce dernier peut être par exemple le dicyclohexylcarbodiimide ou le 1-éthyl-3-(3-diméthyl-aminopropyl)carbodiimide.

Les alkyles et les aryles sont des groupements connus de l'homme du métier. A titre non limitatif de groupement alkyle, on peut citer le méthyle, l'éthyle, le propyle, l'isopropyle, le butyle, le t-butyle, le pentyle. A titre non limitatif de groupement aryle, on peut citer le phényle, le benzyle, le tolyle, le xylyle, le benzylidène, le benzoyle et le naphthyle.

Quand Y représente l'un des groupements suivants : 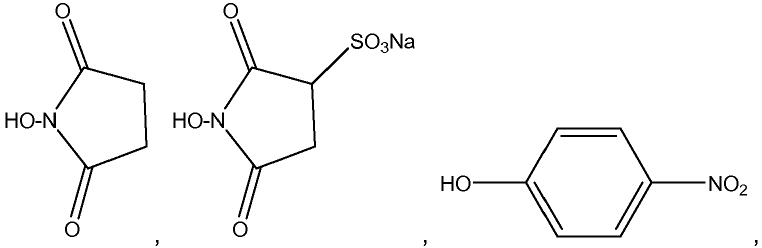 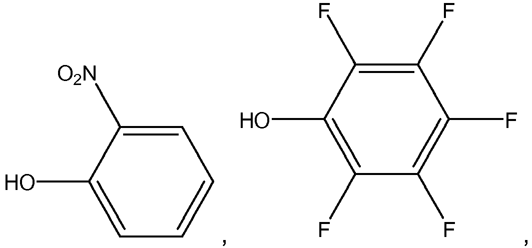 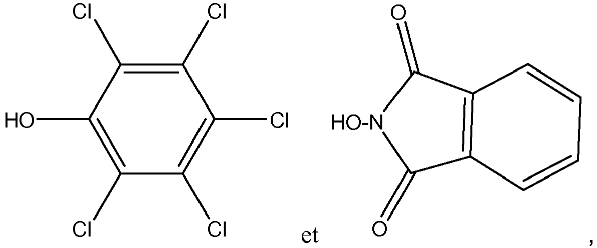 les dérivés de testostérone étant alors des composés de formule (Iβ) ou (Iα) suivantes selon que ce sont respectivement des isomères β ou α en position 3 : 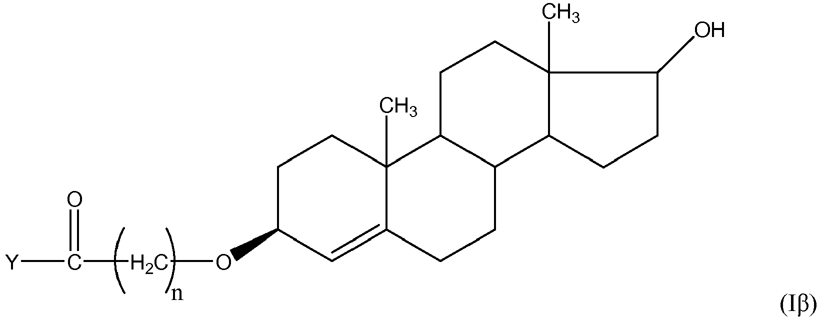 et 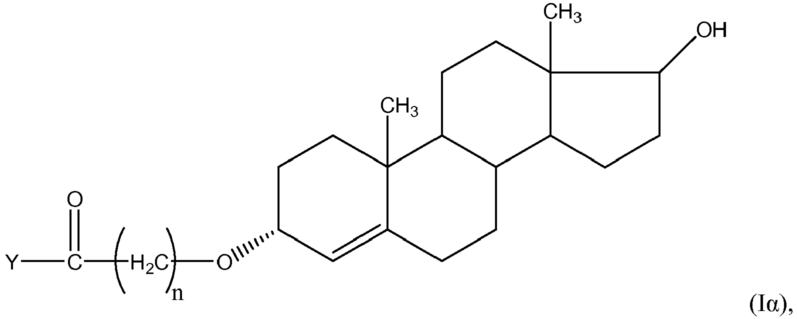 n étant tel que défini précédemment, les procédés de préparation comprennent les étapes précédentes (1) et (2) pour les isomères β et (1) à (3) pour les isomères α, ainsi que l'étape suivante :
1. mettre à réagir le composé de formule (Iaβ) ou (Iaα) ainsi obtenu, en présence d'un dérivé de carbodiimide, avec un réactif choisi, selon le groupement Y final, parmi les composés suivants, pour obtenir les composés de formule (Iβ) ou (Iα) :

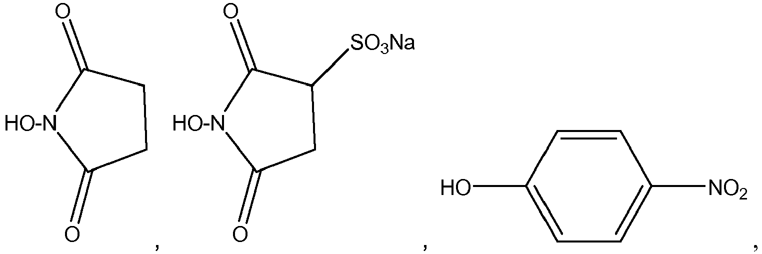

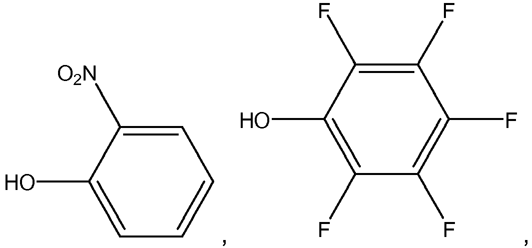

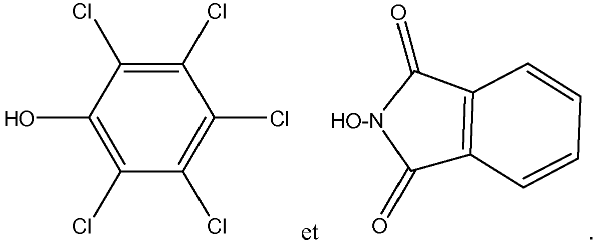

Là encore, l'activation du groupement prêt à être activé du composé (Ia), Y= - OH, est mis en oeuvre en utilisant, en présence d'un dérivé de carbodiimide, l'un des sept composés ci-dessus appropriés qui, lorsqu'ils perdent l'hydrogène du groupement hydroxyle, deviennent des groupements activés Y.

Ainsi, un autre objet de l'invention concerne un procédé de préparation d'un dérivé de testostérone, sous forme d'isomère β en position 3, de formule générale (Iβ) suivante : 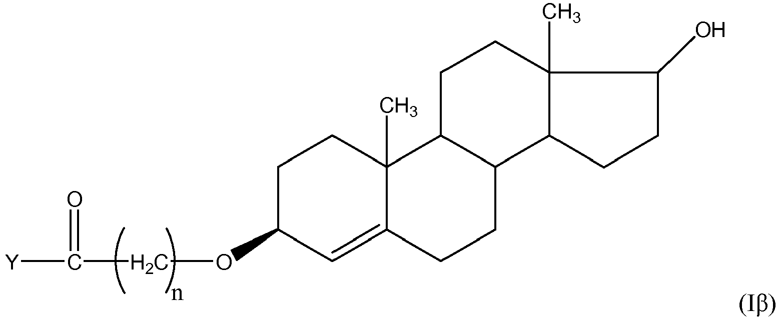 dans laquelle n est un nombre entier compris entre 1 et 10 et Y représente : 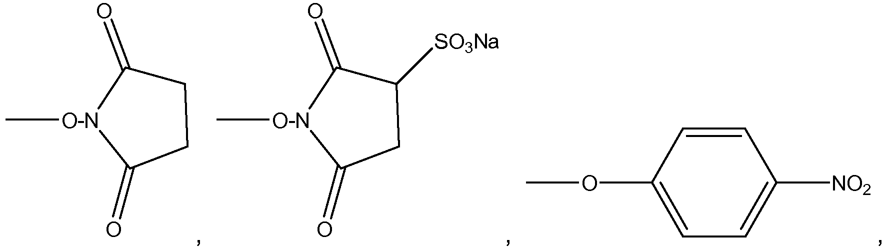 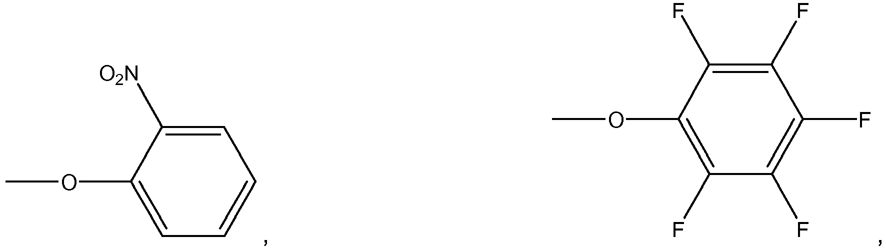 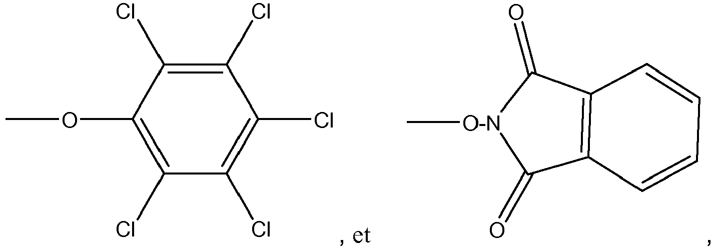 comprenant ou consistant en les étapes consistant à :
(1) mettre à réagir la testostérone avec de l'anhydride acétique pour protéger la fonction OH de la position 17, puis avec un agent réducteur, en présence d'un solvant, pour réduire le carbonyle en position 3 et obtenir le composé de formule (IIβ) : 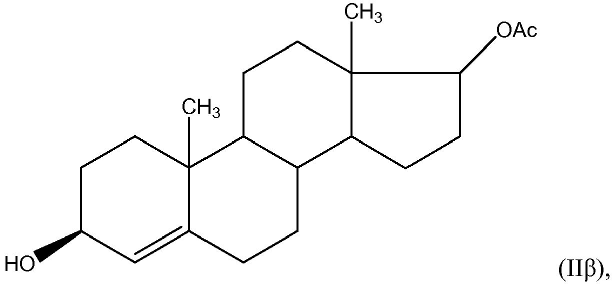 dans laquelle Ac signifie -CO-CH₃,
(2) mettre à réagir le composé de formule (IIβ) ainsi obtenu avec le composé de formule (III) : N₂CH-(CH₂)ₙ₋₁-COOC₂H₅, puis mettre à réagir avec une base, en présence d'un solvant, pour obtenir le composé de formule (Iaβ) : 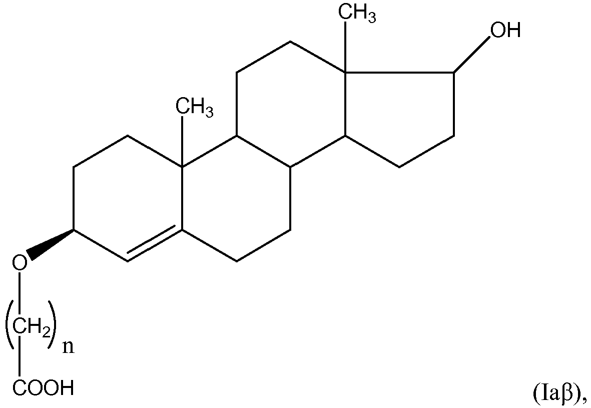
(3) mettre à réagir le composé de formule (Iaβ) ainsi obtenu, en présence d'un dérivé de carbodiimide, avec un réactif choisi, selon le groupement Y final, parmi les composés suivants, pour obtenir les composés de formule (Iβ) :

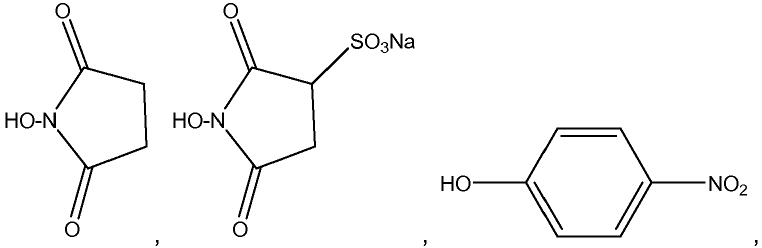

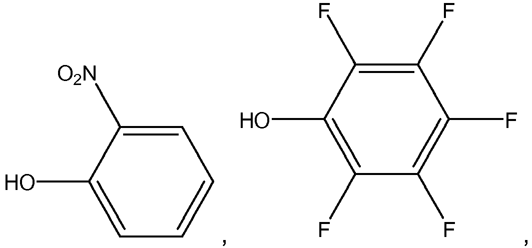

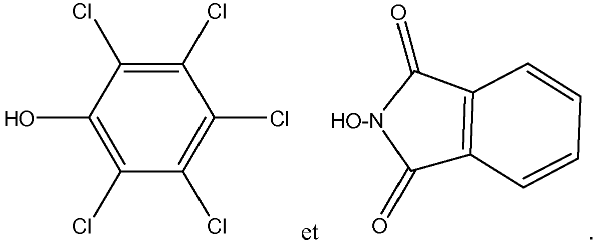

Encore un autre objet de l'invention concerne un procédé de préparation d'un dérivé de testostérone, sous forme d'isomère α en position 3, de formule générale (Iα) suivante : 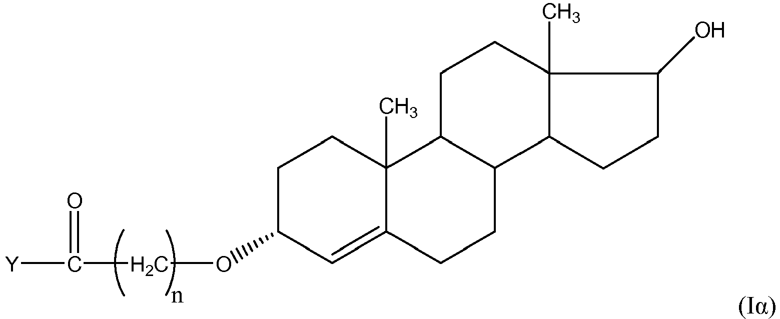 dans laquelle n est un nombre entier compris entre 1 et 10 et Y représente : 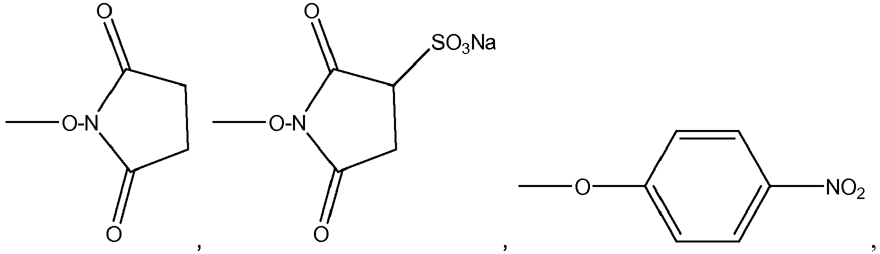 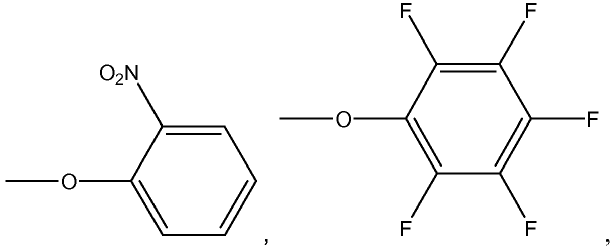 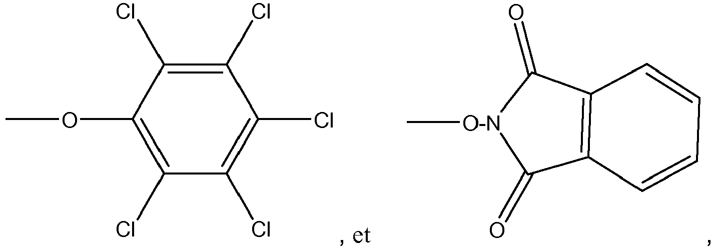 comprenant ou consistant en les étapes consistant à :
(1) mettre à réagir la testostérone avec du t-butyldiméthylchlorosilane pour protéger la fonction OH de la position 17, puis avec un agent réducteur, en présence d'un solvant, pour réduire le carbonyle en position 3 et obtenir le composé de formule (VIIIβ) : 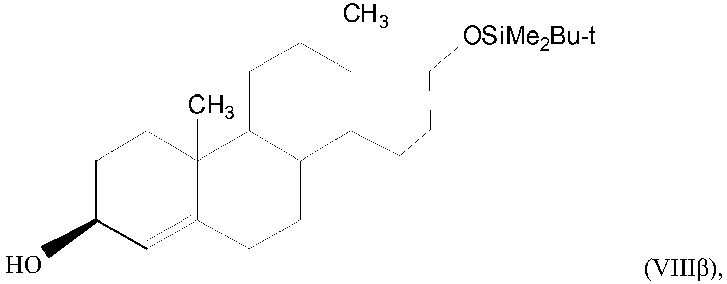 dans laquelle -SiMe₂Bu-t signifie t-butyldiméthylsilanyle,
(2) mettre à réagir le composé de formule (VIIIβ) ainsi obtenu avec de la triphénylphosphine, de l'acide benzoïque et de l'azodicarboxylate de diéthyle, puis avec une base, en présence d'un solvant, pour transformer, en position 3, l'isomère β en isomère α et obtenir ainsi le composé de formule (VIIIα) : 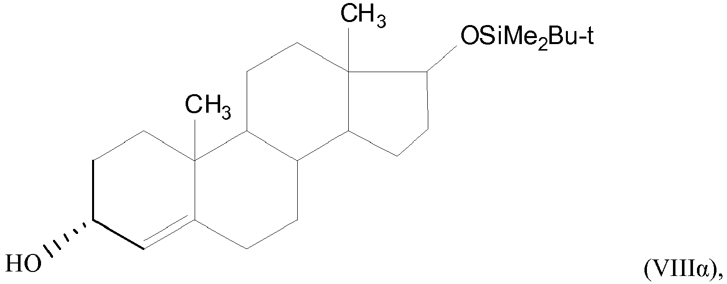 dans laquelle -SiMe₂Bu-t signifie t-butyldiméthylsilanyle,
(3) mettre à réagir le composé de formule (VIIIα) ainsi obtenu avec le composé de formule (III) : N₂CH-(CH₂)ₙ₋₁-COOC₂H₅, puis mettre à réagir avec une base, en présence d'un solvant, pour obtenir le composé de formule (Iaα) : 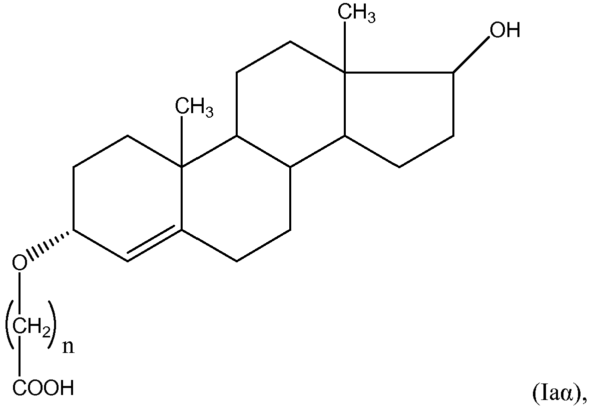
(4) mettre à réagir le composé de formule (Iaα) ainsi obtenu, en présence d'un dérivé de carbodiimide, avec un réactif choisi, selon le groupement Y final, parmi les composés suivants, pour obtenir un composé de formule (Iα) : 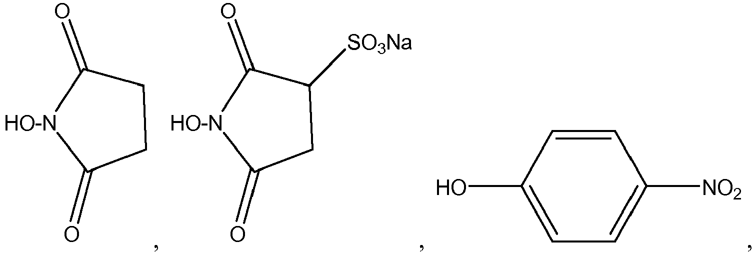 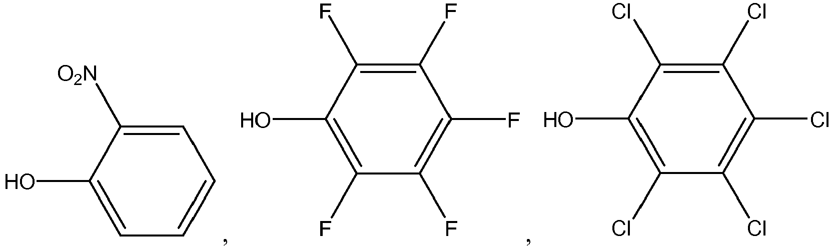 et

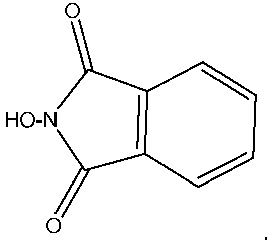

Quand Y représente : 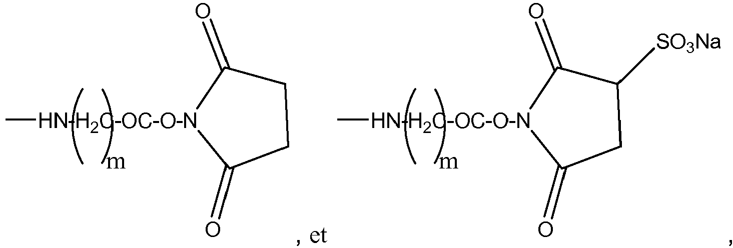 m étant un nombre entier tel que défini précédemment, les dérivés de testostérone étant alors des composés de formule (Iβ) ou (Iα) selon que ce sont respectivement des isomères β ou α en position 3, les procédés de préparation comprennent les étapes précédentes (1) et (2) pour les isomères β et (1) à (3) pour les isomères α, ainsi que les étapes (3) à (5) pour les isomères β et (4) à (6) pour les isomères α pour la préparation des composés de formule (Ibβ) ou (Ibα), ainsi que l'étape suivante :
1. mettre à réagir le composé de formule (Ibβ) ou (Ibα) ainsi obtenu, en présence d'un dérivé de carbodiimide, avec un réactif choisi, selon le groupement Y final, parmi les composés suivants, pour obtenir un composé de formule (Iβ) ou (Iα) :

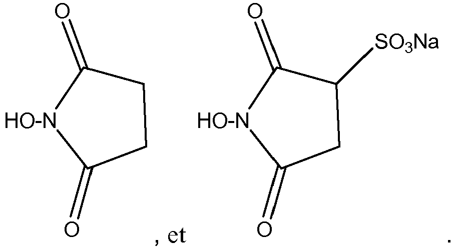

Là encore, l'activation du groupement prêt à être activé du composé (Ib), Y= - COOH, est mis en oeuvre en utilisant, en présence d'un dérivé de carbodiimide, l'un des deux composés ci-dessus qui, lorsqu'ils perdent l'hydrogène du groupement hydroxyle, deviennent des groupements activés Y.

Ainsi, un autre objet de l'invention consiste en un procédé de préparation d'un dérivé de testostérone, sous forme d'isomère β en position 3, de formule générale (Iβ) suivante : 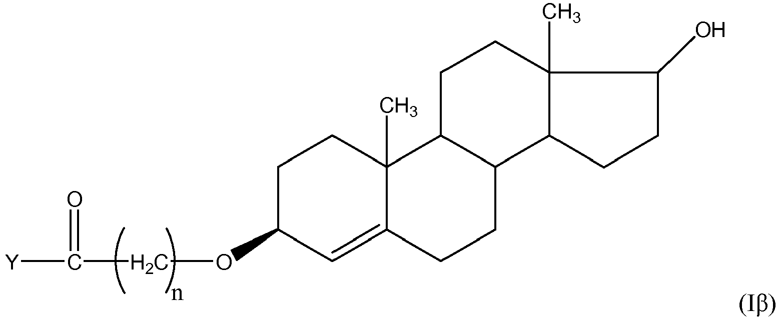 dans laquelle n est un nombre entier compris entre 1 et 10 et Y représente : 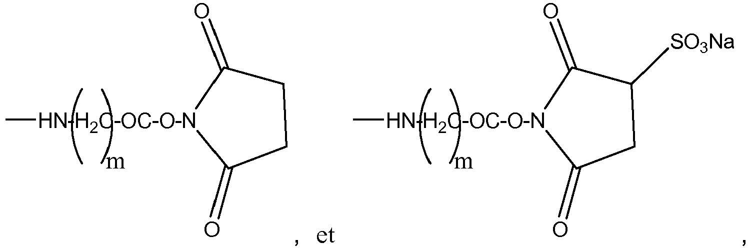 m étant un nombre entier compris entre 1 et 10 , le procédé comprenant ou consistant en les étapes consistant à :
(1) mettre à réagir la testostérone avec de l'anhydride acétique pour protéger la fonction OH de la position 17, puis avec un agent réducteur, en présence d'un solvant, pour réduire le carbonyle en position 3 et obtenir le composé de formule (IIβ) : 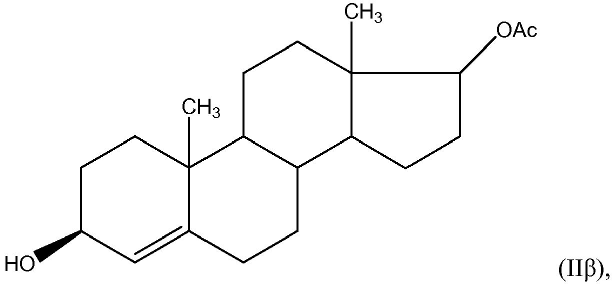 dans laquelle Ac signifie -CO-CH₃,
(2) mettre à réagir le composé de formule (IIβ) ainsi obtenu avec le composé de formule (III) : N₂CH-(CH₂)ₙ₋₁-COOC₂H₅, puis mettre à réagir avec une base, en présence d'un solvant, pour obtenir le composé de formule (Iaβ) : 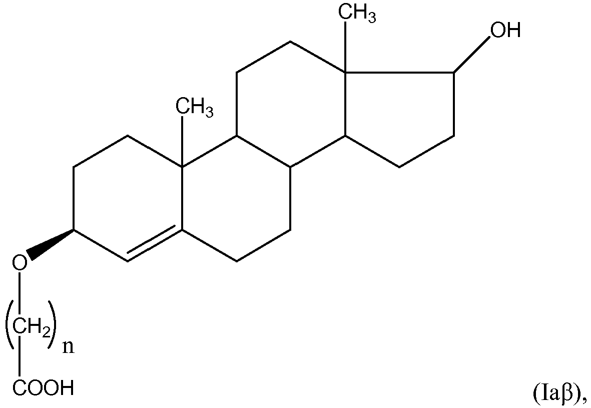
(3) mettre à réagir le composé de formule (Iaβ) ainsi obtenu avec du N-hydroxysuccinimide, en présence d'un dérivé de carbodiimide, pour produire le composé de formule (IVβ) : 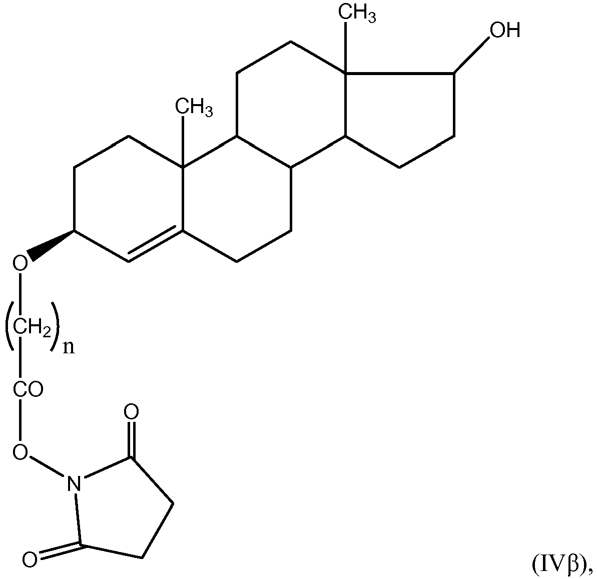
(4) mettre à réagir le composé de formule (IVβ) ainsi obtenu avec le composé de formule (V) : H₂N-(CH₂)m-COOR₁, dans laquelle R₁ est un groupement alkyle ou aryle, pour produire le composé de formule (VIβ) : 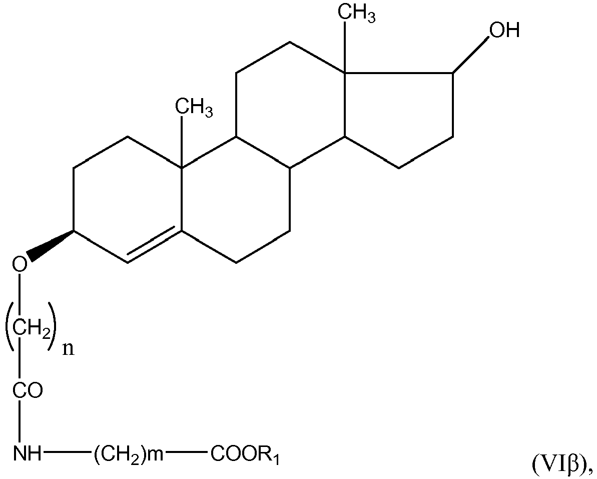
(5) mettre à réagir le composé de formule (VIβ) ainsi obtenu avec une base, en présence d'un solvant, pour obtenir le composé de formule (Ibβ) : 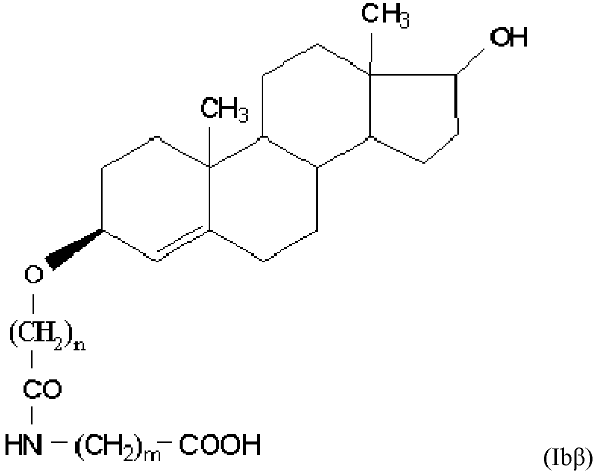
(6) mettre à réagir le composé de formule (Ibβ) ainsi obtenu, en présence d'un dérivé de carbodiimide, avec un réactif choisi, selon le groupement Y final, parmi les composés suivants, pour obtenir les composés de formule (Iβ) :

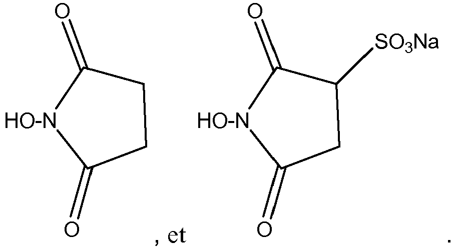

Encore un autre objet de l'invention consiste en un procédé de préparation d'un dérivé de testostérone, sous forme d'isomère α en position 3, de formule générale (Iα) suivante : 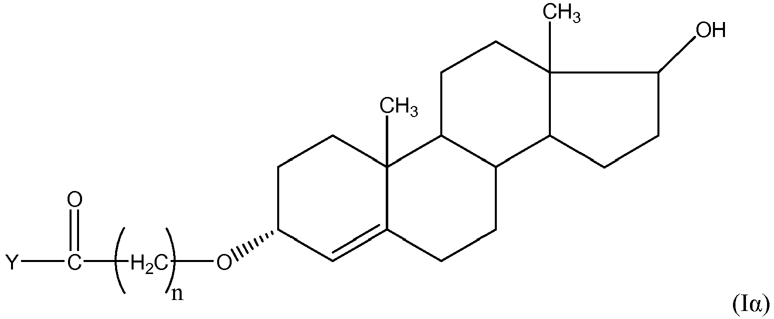 dans laquelle n est un nombre entier compris entre 1 et 10 et Y représente : 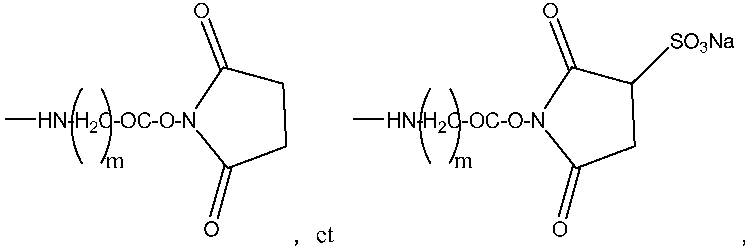 m étant un nombre entier compris entre 1 et 10, le procédé comprenant ou consistant en les étapes consistant à :
(1) mettre à réagir la testostérone avec du t-butyldiméthylchlorosilane pour protéger la fonction OH de la position 17, puis avec un agent réducteur, en présence d'un solvant, pour réduire le carbonyle en position 3 et obtenir le composé de formule (VIIIβ) : 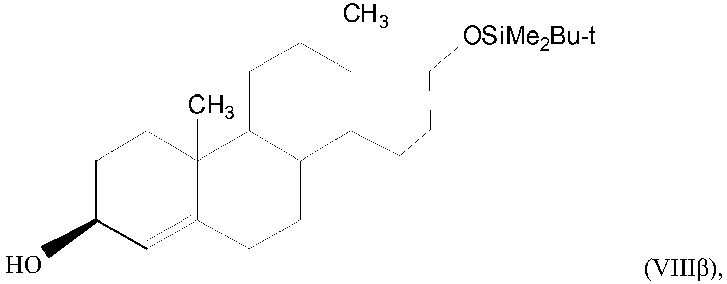 dans laquelle -SiMe₂Bu-t signifie t-butyldiméthylsilanyle,
(2) mettre à réagir le composé de formule (VIIIβ) ainsi obtenu avec de la triphénylphosphine, de l'acide benzoïque et de l'azodicarboxylate de diéthyle, puis avec une base, en présence d'un solvant, pour transformer, en position 3, l'isomère β en isomère α et obtenir ainsi le composé de formule (VIIIα) : 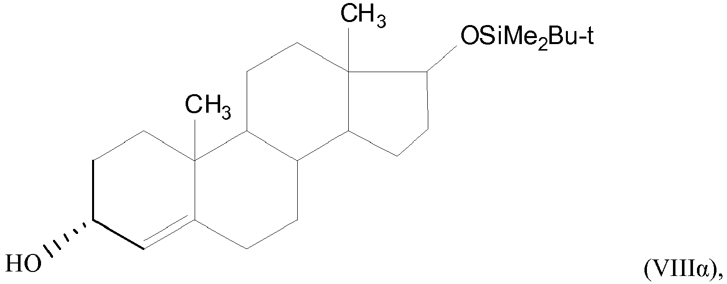 dans laquelle -SiMe₂Bu-t signifie t-butyldiméthylsilanyle,
(3) mettre à réagir le composé de formule (VIIIα) ainsi obtenu avec le composé de formule (III) : N₂CH-(CH₂)ₙ₋₁-COOC₂H₅, puis mettre à réagir avec une base, en présence d'un solvant, pour obtenir le composé de formule (Iaα) : 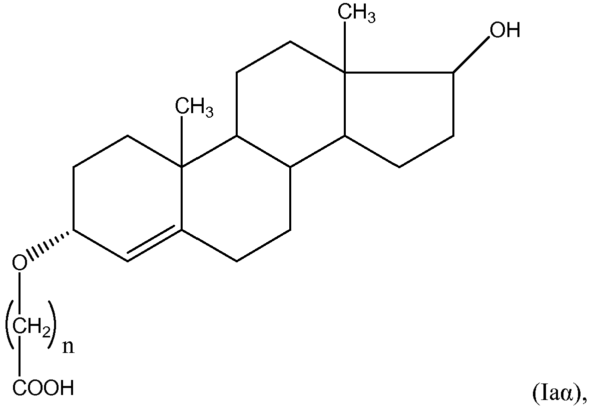
(4) mettre à réagir le composé de formule (Iaα) ainsi obtenu avec du N-hydroxysuccinimide, en présence d'un dérivé de carbodiimide, pour produire le composé de formule (IVα) : 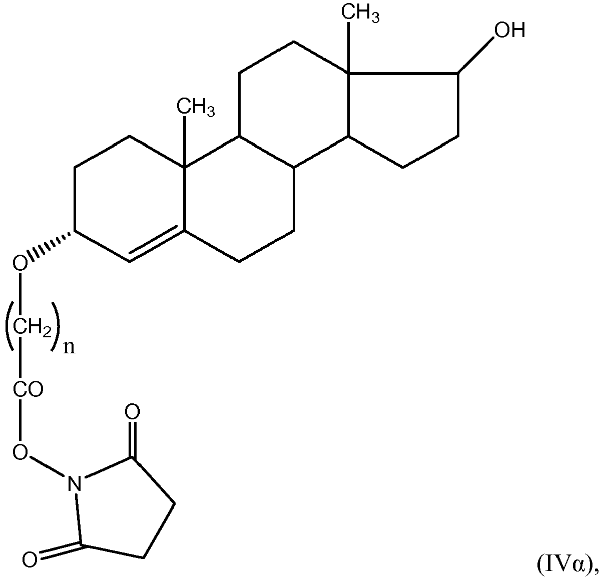
(5) mettre à réagir le composé de formule (IVα) ainsi obtenu avec le composé de formule (V) : H₂N-(CH₂)m-COOR₁, dans laquelle R₁ est un groupement alkyle ou aryle, pour produire le composé de formule (VIα) : 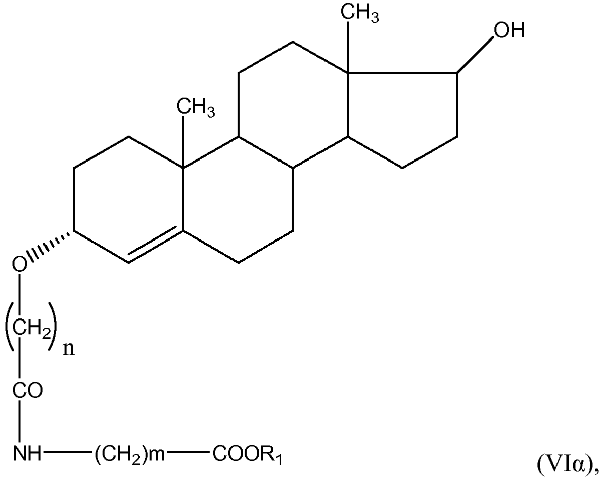
(6) mettre à réagir le composé de formule (VIα) ainsi obtenu avec une base, en présence d'un solvant, pour obtenir le composé de formule (Ibα) : 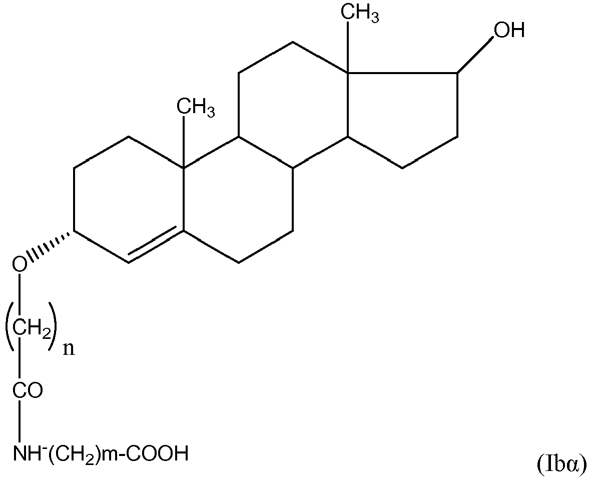
(7) mettre à réagir le composé de formule (Ibα) ainsi obtenu, en présence d'un dérivé de carbodiimide, avec un réactif choisi, selon le groupement Y final, parmi les composés suivants, pour obtenir un composé de formule (Iα) :

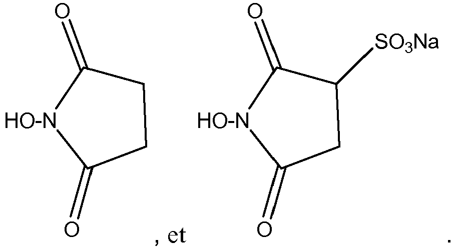

Enfin, quand Y représente N₃, les dérivés de testostérone étant alors des composés de formule (Icβ) ou (Icα) suivantes selon que ce sont respectivement des isomères β ou α en position 3 : 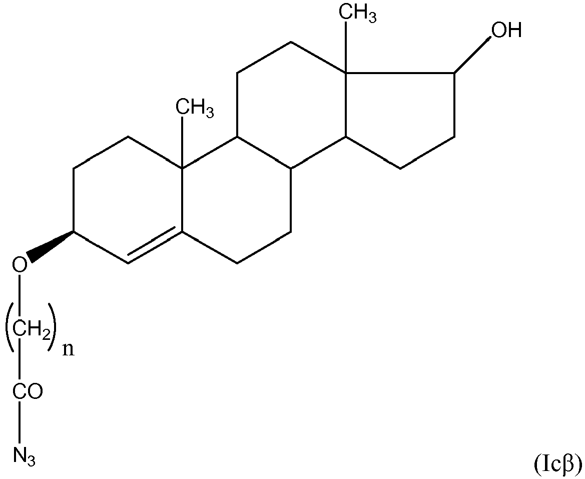 et 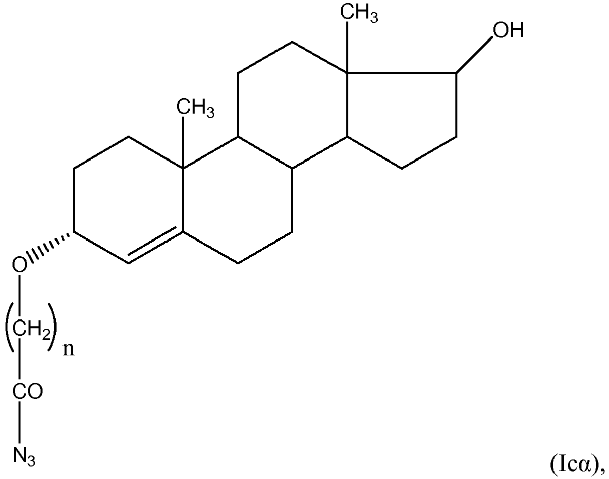 n étant tel que défini précédemment, le procédé de préparation comprend les étapes précédentes (1) et (2) pour les isomères β et (1) à (3) pour les isomères α, ainsi que les deux étapes suivantes :
(1) mettre à réagir le composé de formule (Iaβ) ou (Iaα) ainsi obtenu, en présence d'un dérivé de carbodiimide, avec du H₂NNH₂, pour obtenir le composé de formule (VII) : 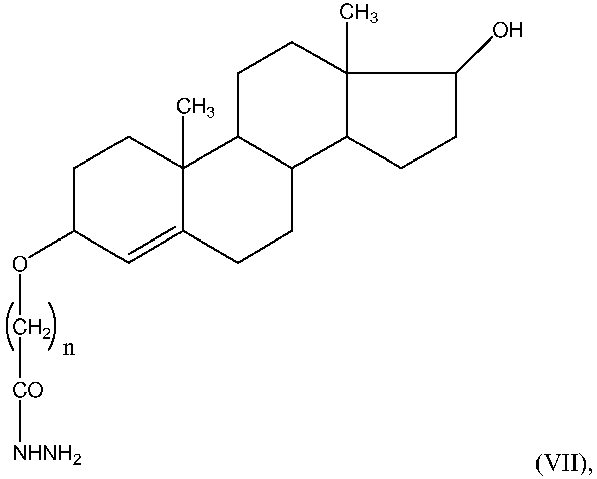
(2) mettre à réagir le composé de formule (VII) ainsi obtenu avec du HONO pour obtenir le composé de formule (Icβ) ou (Icα).

Le dérivé de carbodiimide utilisé dans ce procédé est tel que défini précédemment.

Ainsi, un autre objet de l'invention consiste en un procédé de préparation d'un dérivé de testostérone, sous forme d'isomère β en position 3, de formule générale (Icβ) suivante : 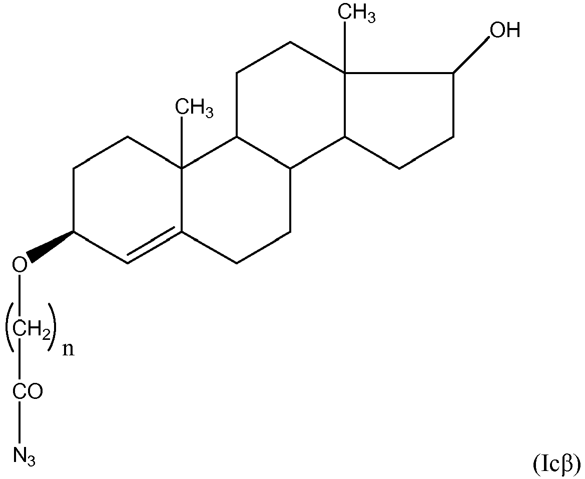 dans laquelle n est un nombre entier compris entre 1 et 10, le procédé comprenant ou consistant en les étapes consistant à :
(1) mettre à réagir la testostérone avec de l'anhydride acétique pour protéger la fonction OH de la position 17, puis avec un agent réducteur, en présence d'un solvant, pour réduire le carbonyle en position 3 et obtenir le composé de formule (IIβ) : 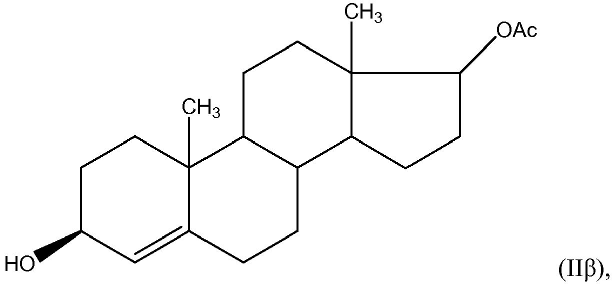 dans laquelle Ac signifie -CO-CH₃,
(2) mettre à réagir le composé de formule (IIβ) ainsi obtenu avec le composé de formule (III) : N₂CH-(CH₂)ₙ₋₁-COOC₂H₅, puis mettre à réagir avec une base, en présence d'un solvant, pour obtenir le composé de formule (Iaβ) : 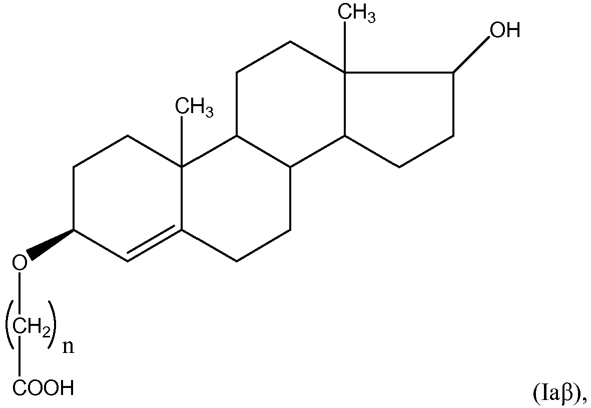
(3) mettre à réagir le composé de formule (Iaβ) ainsi obtenu, en présence d'un dérivé de carbodiimide, avec du H₂NNH₂, pour obtenir le composé de formule (VIIβ) : 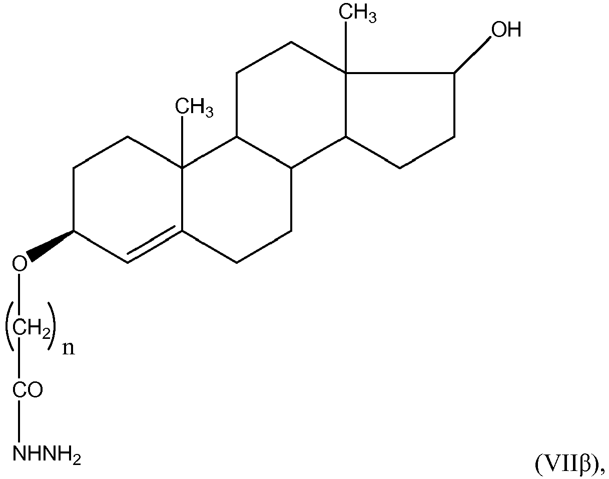
(3) mettre à réagir le composé de formule (VIIβ) ainsi obtenu avec du HONO pour obtenir le composé de formule (Icβ).

Un dernier objet de l'invention concerne un procédé de préparation d'un dérivé de testostérone, sous forme d'isomère α en position 3, de formule générale (Icα) suivante : 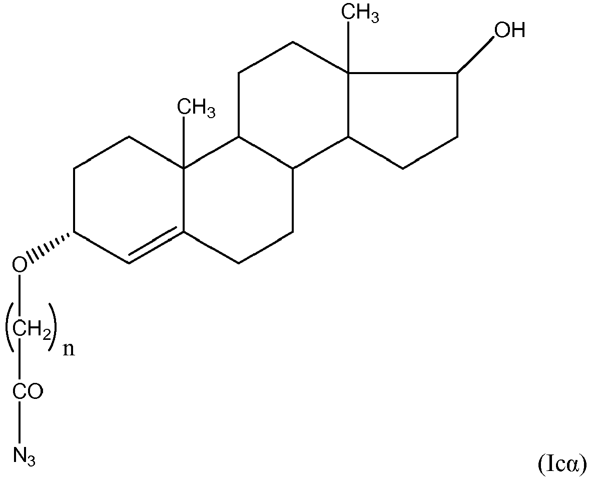 dans laquelle n est un nombre entier compris entre 1 et 10, le procédé comprenant ou consistant en les étapes consistant à :
(1) mettre à réagir la testostérone avec du t-butyldiméthylchlorosilane pour protéger la fonction OH de la position 17, puis avec un agent réducteur, en présence d'un solvant, pour réduire le carbonyle en position 3 et obtenir le composé de formule (VIIIβ) : 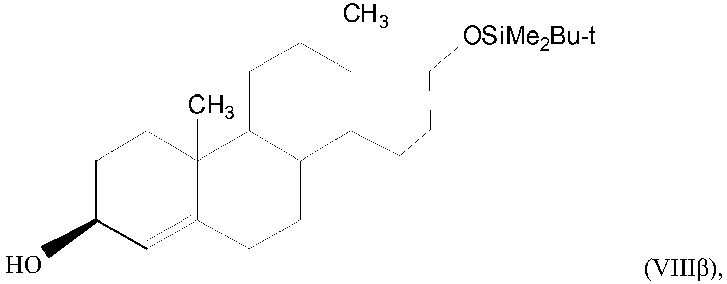 dans laquelle -SiMe₂Bu-t signifie t-butyldiméthylsilanyle,
(2) mettre à réagir le composé de formule (VIIIβ) ainsi obtenu avec de la triphénylphosphine, de l'acide benzoïque et de l'azodicarboxylate de diéthyle, puis avec une base, en présence d'un solvant, pour transformer, en position 3, l'isomère β en isomère α et obtenir ainsi le composé de formule (VIIIα) : 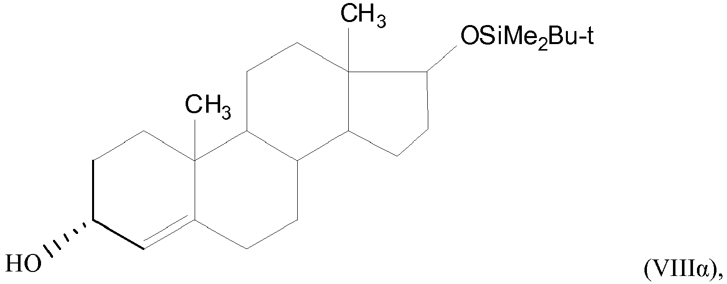 dans laquelle -SiMe₂Bu-t signifie t-butyldiméthylsilanyle,
(3) mettre à réagir le composé de formule (VIIIα) ainsi obtenu avec le composé de formule (III) : N₂CH-(CH₂)ₙ₋₁-COOC₂H₅, puis mettre à réagir avec une base, en présence d'un solvant, pour obtenir le composé de formule (Iaα) : 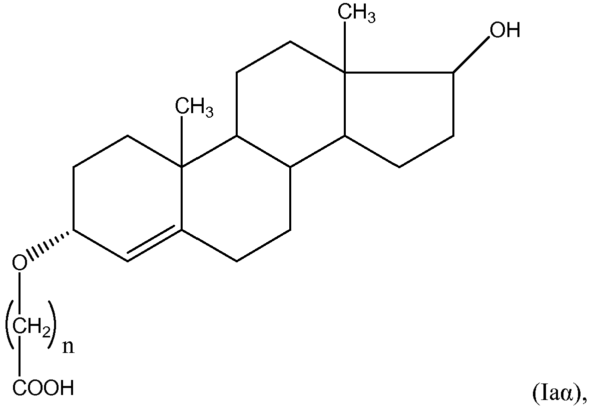
(4) mettre à réagir le composé de formule (Iaα) ainsi obtenu, en présence d'un dérivé de carbodiimide, avec du H₂NNH₂, pour obtenir le composé de formule (VIIα) : 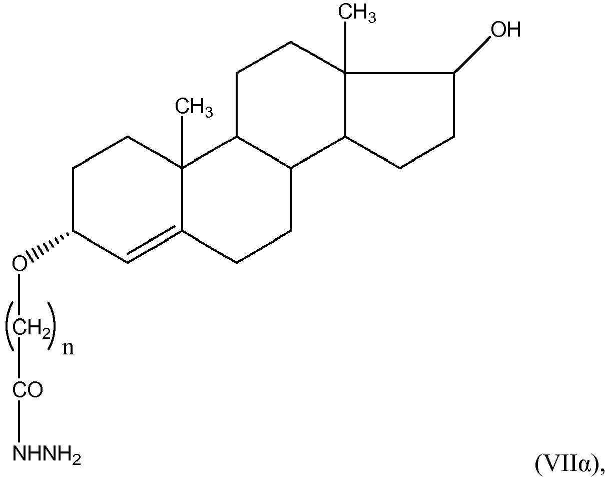
(4) mettre à réagir le composé de formule (VIIα) ainsi obtenu avec du HONO pour obtenir le composé de formule (Icα).

La préparation des conjugués de l'invention est mise en oeuvre par toute technique connue de l'homme du métier permettant de former une liaison amide entre les composés de formule (I) et une amine primaire dans une autre molécule, telle que décrite notamment dans Wong S.S., 1991.

L'invention sera mieux comprise à l'aide des exemples suivants qui sont donnés à titre illustratif et non limitatif, ainsi qu'à l'aide des figures, dans lesquelles :

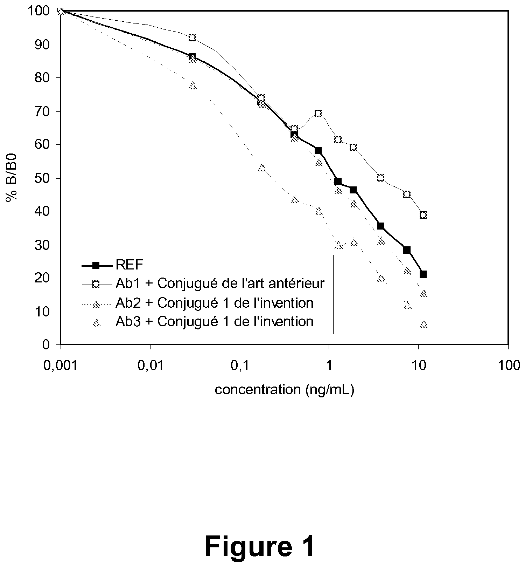

- la Figure 1 montre un graphe donnant le ratio B/B0 en % en fonction de la concentration des points de la gamme étalon (échelle logarithmique), pour un dosage de référence (REF) utilisant un Conjugué de l'art antérieur, un 2^{ème} dosage en utilisant un Conjugué de l'art antérieur (Ab1 + conjugué VIDAS®) et deux dosages utilisant un dérivé de testostérone de l'invention (Ab2 + Conjugué 1 de l'invention, et Ab3 + Conjugué 1 de l'invention),

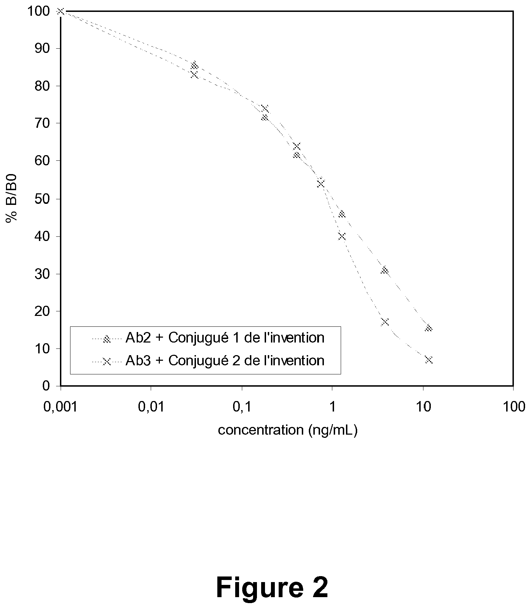

- la Figure 2 montre un graphe donnant le ratio B/B0 en % en fonction de la concentration des points de la gamme étalon (échelle logarithmique), pour un dosage utilisant Ab2 + Conjugué 1 de l'invention et un dosage utilisant Ab3 + Conjugué 2 de l'invention.

### EXEMPLES

L'analyse et le contrôle de la réaction en chromatographie sur couche mince (CCM) sont effectués sur la plaque de gel de silice Merck (type 60, F254, épaisseur 0,2 mm) visualisées à l'aide d'une lampe UV à 254 nm et révélées à la chaleur après pulvérisation de l'acide phosphomolybdique à 5% dans l'éthanol (95%). La purification des produits est réalisée par 'flash chromatography' sur gel de silice Merck, 40-63 µm, pH 6,5 à 7,5.

Les spectres RMN sont enregistrés sur l'appareil Bruker Avance 250. Les déplacements chimiques δ sont exprimés en ppm à partir des références: chloroforme deutérié CDCl₃, δ = 7,26 ppm pour ¹H et 77,14 ppm pour ¹³C ; méthanol deutérié CD₃OD, δ = 4,90 ppm pour ¹H et 49,0 pour ¹³C ; tétraméthylsilane (TMS) : δ = 0 ppm pour ¹H et ¹³C. Les spectres IR sont enregistrés sur l'appareil Thermo-Nicolet FT-IR Avatar 360 et l'option Golden Gate est utilisée. Les analyses en LC-MS sont effectuées avec le système LC-MS PE-Sciex API 100, colonne C₄.

### Exemple 1 : Préparation d'un dérivé de testostérone de l'invention de formule (Iaβ)

17β-hydroxy-3β-carboxyméthoxyandrost-4-ène ou composé de formule (Iβ) dans laquelle n=1 et Y=-OH

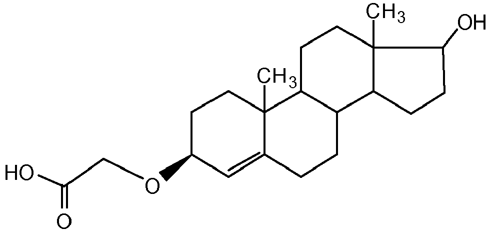

### 1.1 Préparation de 3β-Hydroxy-17β-acétoxy-4-androstène (IIβ)

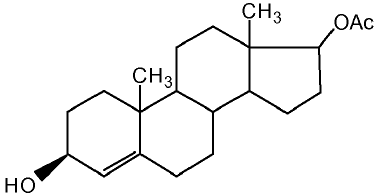

Une solution de 4,23g (14,7 mmole) de testostérone dans 8 ml de pyridine qui a été séchée sur l'hydroxyde de potassium (KOH) pendant 1 nuit (5 g de KOH pour 250 ml de pyridine) est préparée dans un ballon de 250 ml muni d'une agitation magnétique. La solution est refroidie à 0°C avec un mélange eau/glace. 4 ml d'anhydre acétique y sont ajoutés goutte à goutte. On laisse revenir le milieu réactionnel à température ambiante. La solution est agitée pendant une nuit à température ambiante. On ne trouve plus le produit de départ sur la CCM analytique (éluant : acétate d'éthyle / éther de pétrole, 3/1, v/v). 30 ml d'eau distillée et 30 ml d'acétate d'éthyle sont versés dans le ballon et le mélange est agité. Les phases sont séparées avec une ampoule à décanter et la phase aqueuse est extraite avec 3x20ml d'acétate d'éthyle. Les phases organiques sont réunies et la solution obtenue est lavée avec 20 ml d'eau distillée, séchée sur du sulfate de magnésium (MgSO₄) anhydre et évaporée à l'évaporateur rotatif. Le produit ainsi obtenu est séché sous pression réduite à l'aide d'une pompe à vide pendant 4 heures pour enlever tous les solvants. 4,8 g (rendement = 99,0%) du produit intermédiaire 17β-acétoxy-4-androstène sont obtenus.

¹HRMN(CDCl₃) : δ 5,70(1H, H₄), 4,60(1H, H₁₇), 2,07(3H, s), 1,16(3H, s), 0,80(3H, s). ¹³CRMN (CDCl₃): δ 199,5, 171,0(2C), 123,9, 82,5, 53,7, 50,2, 42,4, 38,6, 36,6, 35,7, 35,4, 33,9,32,8, 31,5, 27,5, 23,5, 21,2, 20,5, 17,4, 12,1.

Dans un ballon de 250 ml muni d'une agitation magnétique, 4,7 g (14,2 mmole) de 17β-Acétoxy-4-androstène sont dissouts dans 20 ml de méthanol et 3 ml de tétrahydrofurane (THF). La solution est ensuite refroidie à 0°C avec un mélange eau/glace. 0.8 g (21,1 mmole) du borohydrure de sodium (NaBH₄) sont ajoutés dans la solution. La réaction est suivie sur plaque CCM (éluant: acétate d'éthyle/éther de pétrole, 1/1, v/v) en contrôlant la disparition du produit de départ. Une fois que tout le produit de départ a été consommé, 10 ml d'acétone sont ajoutés pour arrêter la réaction. On laisse revenir la solution à température ambiante et la solution est évaporée à l'évaporateur à 30°C. 30 ml d'eau distillée et 30 ml d'acétate d'éthyle sont versés dans le ballon. Le mélange est agité et les phases sont décantées. La phase aqueuse est extraite avec 3x30 ml d'acétate d'éthyle. Les phases organiques sont réunies, lavées avec 30 ml d'eau distillée, séchées sur du MgSO₄ anhydre et enfin évaporées à sec sous pression réduite à 30°C. 4,6 g (rendement = 97,3%) du produit (IIβ) sont obtenus après une purification chromatographique sur gel de silice (éluant: acétate d'éthyle / éther de pétrole, 111, v/v).

¹HRMN (CDCl₃) : δ 5,30(1H, H₄), 4,61(1H, t, H₁₇), 4,18(1H, H₃), 2,04(3H, s), 1,05(3H, s), 0,80(3H, s). ¹³CRMN(CDCl₃): δ 171,3, 147,3, 123,7, 82,8, 67,9, 54,4, 50,5, 42,6, 37,4, 36,8, 35,8, 35,5, 32,6, 32,1, 29,5, 27,5, 23,6, 21,3, 20,6, 19,0, 12,1.

### 1.2 Préparation du dérivé de testostérone de l'invention de formule (Iaβ)

Le solvant sec réactionnel dichlorométhane (CH₂Cl₂) est préparé par distillation de 250 ml de CH₂Cl₂ sur 5 g du pentoxyde de phosphore (P₂O₅).

Un tricol de 250 ml muni d'une agitation magnétique, d'une ampoule de coulée et d'une arrivée de gaz inerte est purgé avec l'azote sec pendant 10 min. 2,2 g (6,6 mmole) de 3β-Hydroxy-17β-acétoxy-4-androstène (IIβ), 10 ml de dichlorométhane sec et 20 mg (0,045 mmole) d'acétate de Rhodium(II) dimère [Rh(OAc)₂]₂ sont introduits sous azote. Le mélange obtenu est refroidi à 0°C. Une solution de 2,5 ml (2,71g, 23,8 mmole) de diazoacétate d'éthyle (N₂CHCOOEt) dans 5 ml de dichlorométhane sec est préparée et introduite dans l'ampoule de coulée. La solution est ajoutée dans le tricol sous azote, sous agitation, pendant 70 min. On laisse revenir le milieu réactionnel à température ambiante et l'agitation est maintenue pendant 4 heures. Sur la CCM (éluant : acétate d'éthyle / éther de pétrole, 1/1, v/v), on peut trouver encore la présence du produit de départ en faible quantité. Afin de totaliser la réaction, 2,5 mg (0,006 mmole) de [Rh(OAc)₂]₂ sont ajoutés. Une solution de 0,5 ml (4,8 mmole) de N₂CHCOO Et dans 2 ml de dichlorométhane sec est introduite dans le tricol par l'ampoule de coulée pendant 10 min à température ambiante. 20 ml d'eau distillée et 20 ml de dichlorométhane sont versés et le mélange est agité pendant 10 min. Les phases sont séparées et la phase aqueuse est extraite avec 2x20 ml de dichlorométhane. Les phases organiques sont rassemblées, séchées sur MgSO₄ anhydre et évaporées à sec à l'aide d'un évaporateur rotatif à 25°C pour donner le produit brut sous forme d'huile jaune. La purification est réalisée en chromatographie sur gel de silice (éluant : acétate d'éthyle / éther de pétrole, 1/3, v/v). 2,4 g (rendement = 86,6%) du produit 3β-Carboxyméthoxy-17β-acétoxy-4-androstène éthyle ester sont obtenus.

¹HRMN (CDCl₃) : δ 55,39(1H), 4,54(1H, t), 4,23(2H, q), 4,13(1H), 2,03(s), 1,29(t), 1,05(s), 0,80(s). ¹³CRMN(CDCl₃): δ 171,2, 170,9,148,1, 120,4, 82,7, 76,0, 65,5, 61,2, 54,3, 50,4, 42,5, 37,5, 36,8, 35,6, 35,2, 32,5, 32,1, 27,5, 25,2, 23,5, 21,2, 20,4, 18,8, 14,0, 12,0. MS (LC-MS) : 347,0 (M-H)⁻.

2,0 g (4,8 mmole) de 3β-Carboxyméthoxy-17β-acétoxy-4-androstène éthyle ester et 20 ml de méthanol sont introduits dans un ballon de 250 ml muni d'une agitation magnétique. Le mélange est refroidi à 0°C. 10 ml (1,0M, 10 mmole) de solution d'hydroxyde de sodium (NaOH) sont ajoutés. L'agitation est maintenue à 0°C pendant 3 heures. On laisse revenir le milieu réactionnel à température ambiante et la réaction de saponification se complète après 20 heures d'agitation. Le mélange réactionnel est neutralisé avec l'acide chlorhydrique (HCl, 1,0N) à pH 6 et ensuite évaporé à 25°C avec un évaporateur rotatif. Le résidu est purifié sur une colonne de gel de silice pour donner 1,3 g (rendement = 78,0%) du produit (Iaβ).

¹HRMN (CD₃OD) : δ 5,32(1H, H₄), 3,83(2H), 3,50(1H, t), de 2,40 à 1,10 (m), 1,01(3H, s), 0,69(3H, s). ¹³CRMN(CD₃OD) : δ 174,5, 148,9, 119,8, 81,9, 76,3, 64,8, 54,5, 50,7, 42,9, 37,6, 36,6, 35,9, 35,2, 32,6, 32,2, 30,3, 25,2, 23,4, 20,6, 18,9, 11,1. IR (cm⁻¹) : 3383, 2928, 2869, 2847, 1721, 1529, 1432, 1377, 1336, 1243, 1213, 1110, 1050.

### Exemple 2 : Préparation d'un dérivé de testostérone de l'invention de formule (Iβ)

17β-hydroxy-3β-carboxyméthoxy-androst-4-ène N-hydroxysuccinimide ester ou composé de formule (I) dans laquelle n=1 et Y=

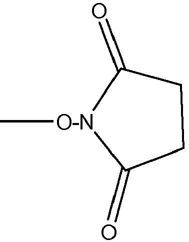

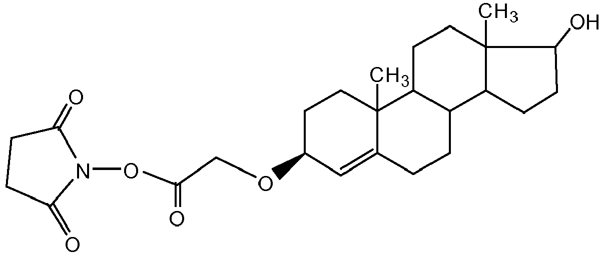

On a répété le mode opératoire décrit dans l'exemple 1, puis on a poursuivi comme suit.

10 ml de 1,2-diméthoxyéthane (DME) distillé sur de l'hydrure de calcium (CaH₂), 100 mg (0,287 mmole) du composé de formule (Iaβ) préparé dans l'exemple 1, et 33 mg (0,287 mmole) de N-hydroxysuccinimide (HOSu) sont introduits dans un ballon de 25 ml muni d'une agitation magnétique. Le mélange est refroidi à 0°C. 59 mg (0,287 mmole) de 1,3-dicyclohexylcarbodiimide (DCC) sont ajoutés. L'agitation est maintenue à température pendant 15 heures. On trouve, sur la CCM (éluant : chloroforme/acétone/acide acétique, 70/25/1, v/v/v), la disparition presque totale du produit de départ. Le mélange réactionnel est filtré et le filtrat est évaporé à température ambiante sous pression réduite. 6,0 ml de dichlorométhane anhydre sont ajoutés dans le résidu et le mélange ainsi obtenu est ensuite filtré. La solution est évaporée à sec à température ambiante avec un évaporateur rotatif. Le produit est séché sous vide pendant 4 heures. 64 mg (50,0%) du produit (IVβ, n=1) sont obtenus.

¹HRMN (CDCl₃) : δ 5,38(1H, H₄), 4,47(2H), 4,40(1H, t, H₁₇), de 4,20 à 3,40(4H, m), 2,85(4H, s), de 2,60 à 1,10 (m), 1,05(3H, s), 0,74(3H, s). ¹³CRMN(CDCl₃) : δ 168,9, 166,6, 149,2, 119,7, 81,9, 76,9, 63,2, 54,5, 50,7, 42,9, 37,6, 36,6, 36,0, 35,1, 32,6, 32,2, 30,5, 25,7, 25,2, 23,4, 20,6, 18,9, 11,2. IR (cm⁻¹) : 3537, 3323, 2929, 2849, 1784, 1732, 1203, 1071. IR (cm⁻¹): 3536, 3507, 3322, 2928, 2849, 1783, 1731, 1624, 1573, 1442, 1426, 1376, 1203, 1071. MS (LC-MS) : 446 (M + H)⁺, 908 (2M + H₂O)⁺.

### Exemple 3 : Préparation d'un dérivé de testostérone de l'invention de formule (Ibβ)

Composé de formule (Iβ) dans laquelle n=1 et Y=-NH-CH₂-COOH

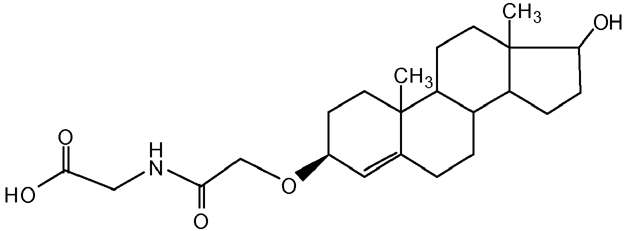

On a répété le mode opératoire décrit dans l'exemple 1, puis on a poursuivi comme suit.

### 3.1 Préparation du composé de formule (Viβ) dans laquelle m = n = 1

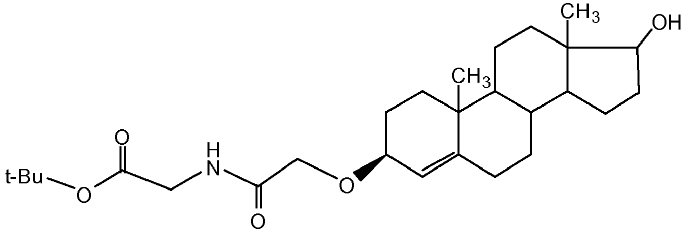

Le produit de l'exemple 1 (Iaβ) (500 mg, 1,43 mmole) et 30 ml de DME anhydre sont introduits dans un ballon de 100 ml. Du N-hydroxysuccinimide (HOSu, 247,7 mg, 2,15 mmole) est ajouté. Le mélange est agité et du 1,3-dicyclohexylcarbodiimide (DCC, 440 mg, 2,15 mmole) est ensuite ajouté. L'agitation est maintenue à température ambiante pendant 1 nuit. Le milieu réactionnel est filtré et le filtrat, contenant le composé (IVβ, n=1) est directement utilisé pour la suite de la synthèse.

Une solution de bicarbonate de sodium (NaHCO₃, 1,0N, 10 ml) et 1,0 ml de produit tert-butyle glycine ester (H₂NCH₂COOBu^{t}) sont ajoutés dans le filtrat. Le mélange formé est agité à température ambiante pendant 12 heures. 70 ml d'eau distillée et 50 ml d'acétate d'éthyle sont mélangés avec le milieu réactionnel. Les phases sont décantées et la phase est extraite avec de l'acétate d'éthyle (3x50 ml). La solution organique réunie est séchée sur MgSO₄ anhydre puis évaporée à l'aide d'un évaporateur rotatif à 30°C. Le résidu est purifié en chromatographie (éluant : acétate d'éthyle 100%). 401 mg (rendement = 60,2%) du produit (VIβ, m=n=1) sont formés sous forme de cristaux blanc.

¹HRMN (CDCl₃) : δ 7,19(1H), 5,32(1H), 4,35(1H), 4,01(2H, s), 3,94(2H, d), 3,64(1H, t), de 2,30 à 0,80 (m), 1,45(s), 1,04(s), 0,73(s). ¹³CRMN(CDCl₃) : δ 170,6, 168,8, 148,8, 120,0, 82,3, 81,8, 76,3, 67,0, 54,5, 50,7, 42,9, 41,4, 37,6, 36,6, 36,0, 35,1, 32,6, 32,2, 30,5, 28,1, 25,5, 23,4, 20,6, 18,9, 11,1.

### 3.2 Préparation du dérivé de testostérone de formule (Ibβ) dans laquelle m=n=1

400 mg (0,87 mmole) de l'ester de tert-butyle (VIβ, n=1) préparé sont dissouts dans 30 ml de méthanol. 2,0 ml de solution d'hydroxyde de sodium (NaOH, 1,0N) sont ajoutés. L'agitation est maintenue à température ambiante pendant 15 heures. Le mélange réactionnel est neutralisé avec de l'acide chlorhydrique (HCl, l,0N) à pH = 6. La solution ainsi formée est évaporée à 30°C sous pression réduite. Le produit brut obtenu est purifié en chromatographie avec un éluant méthanol/chloroforme (20/80, v/v). 340 mg (rendement = 96,8%) du dérivé de formule (Ibβ, m=n=1) sont obtenus sous forme de cristaux blancs.

¹HRMN (CD₃OD) : δ 5,35(1H), 3,96(2H, s), 3,86(2H, s), 3,50(1H, t), 3,25 (1H), de 2,30 à 0,70 (m), 1,02(3H, s), 0,69(3H, s). ¹³CRMN(CD₃OD) : δ 173,5, 173,4, 149,5, 121,5, 82,3, 77,5, 67,7, 56,1, 52,0, 44,0, 42,0, 38,6, 37,9, 37,3, 36,4, 34,7, 33,9, 33,2, 30,5, 24,3, 21,7, 19,3, 11,6. IR (cm⁻¹): 3371, 3322, 2922, 2848, 1714, 1622, 1537, 1437, 1420, 1230, 1077. MS (LC-MS) : 406,6 (M + H)⁺, 405,4 (M - H)⁻.

### Exemple 4 : Préparation d'un dérivé de testostérone de l'invention de formule (Iβ)

Composé Iβ de formule (I) dans laquelle m = n = 1 et Y=

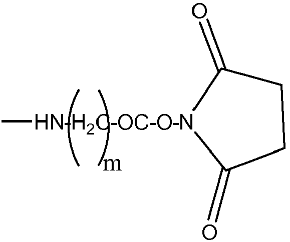

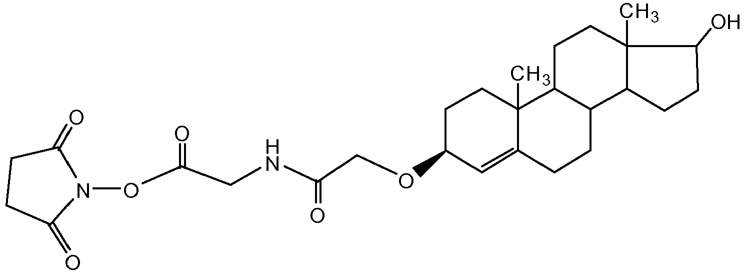

On a répété le mode opératoire décrit dans l'exemple 3, puis on a poursuivi comme suit.

Le produit de formule (Ibβ) obtenu dans l'exemple 3 (220 mg, 0,54 mmole) est dissout dans 15 ml de tétrahydrofurane (THF) anhydre dans un ballon muni d'une agitation magnétique. 62,5 mg (0,54 mmole) du N-hydroxysuccinimide (HOSu) sont introduits et une solution transparente est formée. 112 mg (0,54 mmole) de 1,3-dicyclohexylcarbodiimide (DCC) sont ajoutés. Le milieu est agité à température ambiante pendant 4 heures 30 min. Le mélange réactionnel est filtré et le filtrat est évaporé sans chauffer à l'aide d'un évaporateur rotatif. Le résidu obtenu est lavé avec 6,0 ml de dichlorométhane anhydre, puis séché sous vide. 190 mg (rendement = 70,1 %) de l'ester (1β) sont produits. ¹HRMN (CDCl₃) : δ 7,20(IH), 5,35(lH), 4,45(H, d), 4,05(2H), 3,98(1H), 3,64(1H, t), 2,84(4H, m), de 2,60 à 0,80 (m), 1,04(3H, s), 0,73(3H, s). ¹³CRMN(CDCl₃) : δ 171,0, 168,7, 165,6, 149,1, 119,8, 81,8, 76,5, 66,8, 54,5, 50,7, 42,9, 38,3, 37,6, 36,6, 36,0, 35,0, 33,8, 32,6, 32,3, 30,4, 25,6, 23,4, 20,6, 18,9, 11,1. MS (Fab, Na) : 501 (M - 1)⁺,525 (M + Na)⁺.

### Exemple 5 : Préparation d'un dérivé de testostérone de l'invention de formule (Iaα)

17β-hydroxy-3α-carboxyméthoxy-androst-4-ène ou composé de formule (Iα) dans laquelle n=1 et Y=-OH

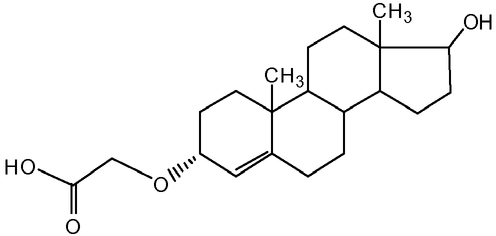

### 5.1 Préparation de 3β-Hydroxy-17β-O-(t-butyldiméthylsilyl)-4-androstène (VIIIβ)

2,88 g (10 mmole) de testostérone, 12 ml de N,N'-diméthylformamide (DMF) anhydre et 1,0 g (14,7 mmole) d'imidazole sont introduits dans un ballon de 250 ml sous azote. 2,26 g (15 mmole) de tert-butyldiméthylchlorosilane (C1TBDMS) sont ajoutés. Le mélange est agité sous azote à température ambiante pendant 4 heures 30 min. 100 ml d'eau distillée et 100 ml d'acétate d'éthyle sont versés dans le milieu réactionnel et le mélange est agité. Les phases sont séparées et la phase aqueuse est extraite avec de l'acétate d'éthyle (3x70 ml). La solution organique réunie est lavée avec 100 ml d'eau distillée, séchée sur le MgSO₄ anhydre et évaporée. Le résidu est purifié en chromatographie de gel de silice (éluant : acétate d'éthyle/éther de pétrole, 1/5, v/v) pour donner 3,67 g du produit réactionnel sous forme de cristaux blancs.

3,66 g de ce produit obtenu sont mélangés avec 40 ml de méthanol, 10 mg de cérium(III) chlorure hydraté (CeCl₃.7 H₂O) dans un ballon de 250 ml muni d'une agitation magnétique. Une suspension est formée. 0,40 g (10,6 mmole) de borhydrure de sodium (NaBH₄, 98%) sont ajoutés en 3 fois pendant 5 min. L'agitation est maintenue à température pendant 10 min. Le milieu réactionnel est évaporé à 30°C à l'aide d'un évaporateur rotatif sous pression réduite. Le résidu est mélangé avec 50 ml d'acétate d'éthyle et 50 ml d'eau distillée. Les phases sont décantées et la phase aqueuse est extraite avec de l'acétate d'éthyle (2x50 ml). La solution organique rassemblée est séchée sur du MgSO₄ anhydre et évaporée pour donner le produit brut qui est ensuite purifié en chromatographie de gel de silice (éluant : acétate d'éthyle/éther de pétrole, 1/4, v/v). 3,5 g (rendement = 86,5%) du produit (VIIIβ) sont formés sous forme de cristaux blancs.

¹HRMN (CDCl₃) : δ 5,29(1H, H₄), 4,18 (1H, H₃), 3,56(1H, t, H₁₇), de 2,40 à 0,80 (m), 1,08(3H, s, H₁₉), 0,89(9H, s, t-Bu), 0,74(3H, s, H₁₈), 0,02(6H, s, Me₂Si). ¹³CRMN(CDCl₃) : δ 147,8, 123,5, 81,8, 68,0, 54,8, 50,5, 43,3, 37,2, 37,1, 36,1, 35,5, 32,8, 32,2, 31,0, 29,6, 25,9, 23,6, 20,8, 19,1, 18,9, 11,4, -4,3, -4,6.

### 5.2 Préparation de 3α-Hydroxy -17β-O-(t-butyldiméthylsilyl)-4-androstène (IXα)

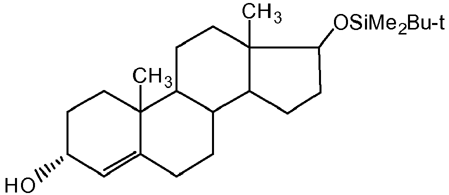

Dans un ballon de 500 ml, 4,46 g (11,0 mmole) du produit (VIIIβ) préparé, 50 ml de benzène anhydre, 5,78 g (22,0 mmole) de triphénylphosphine et 2,69 g (22,0 mmole) d'acide benzoïque sont introduits sous azote. 3,84 g (22,0 mmole) de diéthyle azodicarboxylate (DEAD) sont dissouts dans 10 ml de benzène anhydre et la solution obtenue est ajoutée goutte à goutte dans le ballon sous protection d'azote pendant 10 min. Le milieu réactionnel est agité à température ambiante pendant 1 heure. 50 ml de solution de bicarbonate de sodium (NaHCO₃, 1,0 M) sont versés dans le milieu et les phases sont séparées. La phase aqueuse est extraite avec de l'acétate d'éthyle (3x30 ml) et la solution organique réunie est lavée avec 50 ml de solution de NaHCO₃ (1,0 M), puis avec 50 ml d'eau distillée, séchée sur du MgSO₄ anhydre et évaporée. Le résidu est repris dans 150 ml d'éthanol et 30 ml de solution d'hydroxyde de sodium (NaOH, 1,0 N) sont ajoutés. Le mélange est agité à température ambiante pendant 1 nuit et à 70°C pendant 3 heures. Il n'y a plus de produit de départ sur la CCM (éluant : acétate d'éthyle /éther de pétrole, 1/9, v/v). Le milieu réactionnel est évaporé jusqu'à 50 ml avec un évaporateur rotatif. 50 ml d'eau distillée et 100 ml d'acétate d'éthyle sont introduits. Les phases sont séparées après agitation. La phase aqueuse est extraite avec de l'acétate d'éthyle (2x70 ml). La solution organique obtenue est lavée avec de l'eau distillée (70 ml), séchée sur le MgSO₄ anhydre et évaporée sous pression réduite pour donner le produit brut. La purification est effectuée à l'aide d'une colonne chromatographique de gel de silice (éluant : acétate d'éthyle /éther de pétrole, 1/7 , v/v). 2,19 g (rendement = 47,1 %) du produit (Ixα) sont obtenus.

¹HRMN (CDCl₃) : δ 5,45(1H, H₄), 4,07(1H, H₃), 3,52(1H, t, H₁₇), de 2,40 à 0,80 (m), 0,97(3H, s, H₁₉), 0,86(9H, s, t-Bu), 0,71(3H, s, H₁₈), 0,00(6H, s, Me₂Si). ¹³CRMN(CDCl₃) : δ 150,2, 120,8, 81,7, 64,2, 54,3, 50,4, 43,3, 37,6, 37,1, 35,9, 32,4(2C), 31,7, 30,9, 27,9, 25,9, 23,6, 21,2, 18,2, 18,1, 11,4, -4,3, -4,7.

### 5.3 Préparation du dérivé de testostérone de formule (Iaα)

Dans un tricol de 250 ml muni d'une agitation magnétique, 3,95 g (9,8 mmole) du produit (Ixα) sont dissouts dans 65 ml de dichlorométhane anhydre sous azote. 100 mg d'acétate de Rhodium(II) dimère sont ajoutés. La solution est refroidie à 0°C. Une solution de 4,0 g (35 mmole) de diazoacétate d'éthyle dans 25 ml du dichlorométhane anhydre est préparée et ajoutée goutte à goutte dans le tricol sous azote pendant 1 heure. On laisse revenir le milieu réactionnel à température ambiante et l'agitation est maintenue pendant 4 heures. 100 ml d'eau distillée sont ajoutés et les phases sont séparées. La phase aqueuse est extraite avec de l'éther éthylique (3x100 ml). La solution organique réunie est séchée sur le MgSO₄ anhydre et évaporée. Le résidu obtenu est purifié avec une colonne chromatographique de gel de silice (éluant : acétate d'éthyle/éther de pétrole, 1/6, v/v) pour donner 3,2 g de produit qui est directement utilisé pour la suite de la synthèse.

3,0 g du produit ainsi obtenu sont dissouts dans 20 ml de tétrahydrofurane (THF) anhydre et la solution est refroidie à 0°C. 15 ml de solution de tétra-n-butylammonium fluorure (Bn₄NF, 1,0 M dans THF) sont ajoutés sous azote. On laisse revenir le milieu réactionnel à température ambiante et l'agitation est maintenue pendant 22 heures. Le mélange réactionnel est évaporé à l'aide d'un évaporateur rotatif. 100 ml d'eau distillée et 100 ml d'éther éthylique sont mélangés avec le résidu. Les phases sont décantées et la phase est extraite avec de l'éther éthylique (3x50 ml). La solution organique est séchée sur du MgSO₄ anhydre et évaporée. Une purification chromatographique du résidu formé sur gel de silice (éluant : acétate d'éthyle/ éther de pétrole, 1/1, v/v) donne 1,0 g de produit (huile transparente) qui est ensuite dissout dans 30 ml d'éthanol. 6,0 ml de solution d'hydroxyde de sodium (NaOH, 1,0 N) sont introduits dans la solution à température ambiante et le mélange est agité pendant 30 min. La CCM (éluant : chloroforme/acétone/acide acétique, 70/25/2, v/v/v) est utilisée pour contrôler l'évaluation de la saponification. Le milieu réactionnel est neutralisé avec de l'acide chlorhydrique (HCl, 1,0 N) à pH 6 et évaporé à sec à température ambiante sous pression réduite. Le produit brut ainsi formé est purifié sur une colonne chromatographique de gel de silice (éluant : chloroforme/méthanol, 4/1, v/v). 950 mg du produit (Iaα) sont obtenus.

¹HRMN (CDCl₃) : δ 5,44(1H, H₄), 4,11(2H, s), 3,84 (1H, H₃), 3,63(1H, t, H₁₇), de 2,40 à 0,80 (m), 1,01(3H, s, H₁₉), 0,78(3H, s, H₁₈). ¹³CRMN(CD₃OD): δ 175,8, 152,2, 119,2, 82,4, 73,5, 66,7, 55,3, 52,0, 44,0, 38,7, 37,9, 37,2, 33,6, 33,4, 33,1, 30,5, 25,0, 24,2, 22,2, 18,7, 11,6. IR (cm⁻¹): 3375, 2925, 2869, 2846, 1725, 1589, 1434, 1213, 1105, 1072. MS (LC-MS): 347,2 (M-H)⁻.

### Exemple 6 : Préparation d'un dérivé de testostérone de l'invention de formule (Iα)

17β-hydroxy-3α-carboxyméthoxy-androst-4-ène N-hydroxysuccinimide ester ou composé de formule (Iα) dans laquelle n=1 et Y=

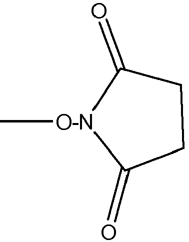

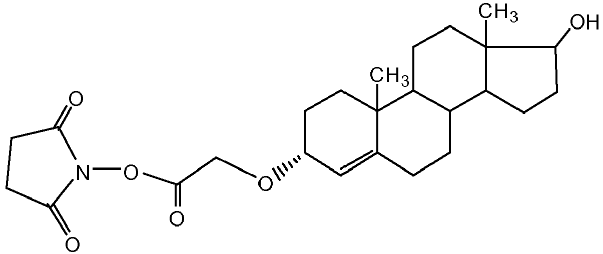

On a répété le mode opératoire décrit dans l'exemple 5, puis on a poursuivi comme suit.

100 mg (0,29 mmole) du produit (Iaα) synthétisé dans l'exemple 5, 34,1 mg (0,29 mmole) de N-hydroxysuccinimie (HOSu) et 18 ml de tétrahydrofurane (THF) anhydre sont introduits dans un ballon de 50 ml muni d'une agitation magnétique. Une suspension est obtenue. 59,8 mg (0,29 mmole) de 1,3-dicyclohexylcarbodiimide (DCC) sont ajoutés. Le milieu est agité à température ambiante pendant 1 nuit. Le mélange réactionnel est filtré et le filtrat est évaporé à 25°C. 5 ml de diméthoxyméthane anhydre sont introduits et le mélange est filtré. Le filtrat est évaporé à 25°C à l'aide d'un évaporateur rotatif. Le résidu est séché à pompe à vide, lavé avec 5 ml d'hexane anhydre et séché sous pression réduite. 86 mg (66,5%, pureté: 90% en HPLC) du dérivé de testostérone (Iα) sont obtenus.

¹HRMN (CDCl₃) : δ 5,50(1H, H₄), 4,44(2H, s), 3,85 (1H, H₃), 3,61(1H, t, H₁₇), 2,84(4H, s), de 2,40 à 0,80 (m), 0,97(3H, s, H₁₉), 0,74(3H, s, H₁₈). ¹³CRMN(CDCl₃) : δ 169,0, 166,7, 152,2, 117,2, 81,9, 73,3, 63,6, 53,9, 50,6, 43,0, 37,7, 36,6, 35,8, 32,4, 32,2, 31,9, 30,4, 25,6, 24,2, 23,4, 21,1, 18,1, 11,1. IR (cm⁻¹) : 3517, 3324, 2917, 2848, 1780, 1729, 1703, 1427,1379,1204,1066. MS (LC-MS) : 447,2 (M+H)⁺.

### Exemple 7 : Préparation d'un dérivé de testostérone de formule (Ibα)

Composé de formule (Iα) dans laquelle n=1 et Y=-NH-CH₂-COOH

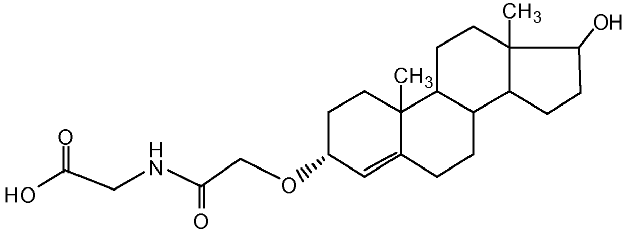

On a répété le mode opératoire décrit dans l'exemple 5, puis on a poursuivi comme suit.

### 7.1 Préparation du composé (VIα) dans lequel m=n=1

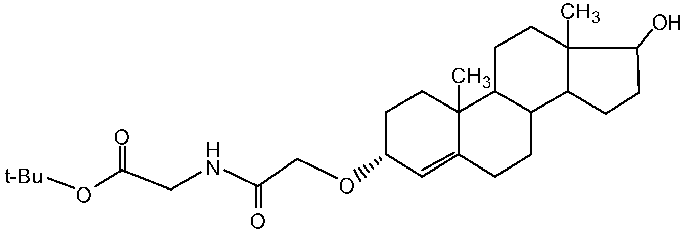

380 mg (1,09 mmole) du produit (Iaα), 125,5 mg (1,09 mmole) de N-hydroxysuccinimide (HOSu) et 30 ml de tétrahydrofurane (THF) anhydre sont mélangés dans un ballon de 100 ml sous protection d'azote. 225 mg (1,09 mmole) de 1,3-dicyclohexylcarbodiimide (DCC) sont introduits. Le milieu est agité à température ambiante pendant 4 heures. La CCM (éluant : chloroforme/méthanol, 5/1, v/v) est utilisée pour contrôler la réaction. Le mélange contenant le produit (IVα, n=1) est filtré.

6 ml de la solution de bicarbonate de sodium (NaHCO₃, 1,0 M) et 142 mg (1,09 mmole) de glycine tert-butyle ester sont rapidement introduits dans le filtrat. Le mélange est agité à 25°C pendant 30 min. puis concentré jusqu'à 10 ml sous pression réduite. 50 ml d'eau distillée et 50 ml d'acétate d'éthyle sont ajoutés. Les phases sont décantées et la phase aqueuse est extraite avec de l'acétate d'éthyle (3x50 ml). La solution organique rassemblée est séchée sur du MgSO₄ et évaporée. Le produit brut est ensuite purifié sur une colonne chromatographique de gel de silice (éluant : acétate d'éthyle 100%) pour donner 440 mg du produit (VIα, m=n=1).

¹HRMN (CDCl₃) : δ 7,12(1H, NH), 5,48(1H, H₄), 4,01(2H, m), 3,80(1H, H₃), 3,66(1H, t, H₁₇), de 2,40 à 0,70 (m), 1,48(s, t-Bu), 0,99(3H, s, H₁₉), 0,76(3H, s, H₁₈). ¹³CRMN(CDCl₃): δ 170,8, 168,6, 151,6, 117,5, 82,2, 81,6, 72,8, 67,6, 53,8, 50,5, 42,9, 41,3, 37,6, 36,6, 35,8, 32,3, 32,2, 32,0, 30,3, 28,0, 24,3, 23,3, 21,0, 18,1, 11,1.

### 7.2 Préparation du dérivé de testostérone de formule (Ibα)

440 mg (0,95 mmole) du produit (VIα, m=n=1) synthétisé sont dissouts dans 30 ml de méthanol et une solution transparente est formée. 3 ml de solution d'hydroxyde de sodium (NaOH, 1,0 N) sont ajoutés à température ambiante. L'agitation est maintenue pendant 4 heures. Il n'y a plus de présence du produit de départ (VIα) sur la CCM (éluant : chloroforme/méthanol, 4/1, v/v). Le milieu réactionnel est neutralisé avec de l'acide chlorhydrique (HCl, 1,0 N) à pH = 6 et évaporé à 25°C sous pression réduite. Le résidu est purifié avec une colonne de chromatographie de gel de silice (éluant : chloroforme/méthanol, 2,5/1, v/v). 380 mg (rendement = 98,6%) du produit (Iα) sont obtenus.

¹HRMN (CD₃OD) : δ 7,88(1H, t, ²J = 3,13Hz, NH), 5,50(1H, d, ²J = 4,06Hz, H₄), 4,00(2H, d, ²J = 3,13Hz), 3,90(2H, s), 3,80(1H, t, H₃), 3,56(1H, t, H₁₇), de 2,40 à 0,70 (m), 1,02(3H, s, H₁₉), 0,76(3H, s, H₁₈). ¹³CRMN(CD³OD): δ 174,2, 173,5, 152,5, 119,0, 82,4, 74,0, 68,3, 55,4, 52,0, 44,0, 42,5, 38,7, 37,9, 37,2, 33,7, 33,4, 33,2, 30,6, 25,3, 24,3, 22,2, 18,6, 11,6. IR (cm⁻¹): 3369, 2921, 2847, 1715, 1624, 1538, 1436, 1422, 1233, 1078. MS (LC-MS) : 404,4 (M-H)⁻, 809,9 (2M-H)⁻.

### Exemple 8 : Préparation d'un dérivé de testostérone de l'invention de formule (Iα)

composé de formule (Iα) dans laquelle m=n=1 et Y=

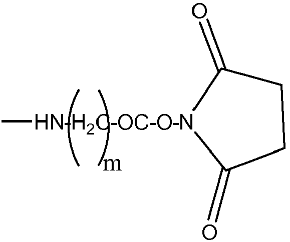

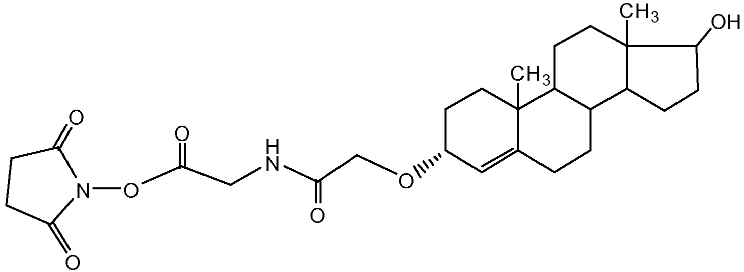

On a répété le mode opératoire décrit dans l'exemple 7, puis on a poursuivi comme suit.

Le produit de formule (Ibα) préparé dans l'exemple 7 (120 mg, 0,295 mmole) est dissout dans 10 ml de 1,2-diméthoxyéthane (DME) anhydre dans un ballon muni d'une agitation magnétique. 35,1 mg (0,295 mmole) de N-hydroxysuccinimide (HOSu) sont introduits et une solution transparente est formée. 61,7 mg (0,295 mmole) de 1,3-dicyclohexylcarbodiimide (DCC) sont ajoutés. Le milieu est agité à température ambiante pendant 4 heures 30 min. Le mélange réactionnel est filtré et le filtrat est évaporé sans chauffer à l'aide d'un évaporateur rotatif. Le résidu obtenu est lavé avec 6,0 ml de dichlorométhane anhydre, puis séché sous vide. 90,4 mg (rendement = 61,0 %, pureté 85% en HPLC) du produit (Iα) sont obtenus. Le produit est conservé à -20°C sous azote.

¹HRMN (CDCl₃) : δ 7,17(1H, NH), 5,42(1H, H₄), 4,45(2H, d), 4,03(2H, s), 3,76(1H, H₃), 3,62(1H, t, H₁₇), 2,84(4H, s), de 2,30 à 0,80 (m), 1,05(3H, s, H₁₉), 0,75(3H, s, H₁₈). ¹³CRMN(CDCl₃): δ 171,2, 168,7, 165,7, 152,0, 117,4, 81,9, 73,1, 67,5, 53,9, 50,6, 43,0, 38,3, 37,7, 36,6, 35,8, 32,4, 32,3, 32,1, 30,5, 25,6, 24,3, 23,4, 21,1,18,2, 11,1.

### Exemple 9: Préparation d'un conjugué 1 de l'invention 17β-hydroxy-3β-carboxyméthoxyandrost-4-ène/phosphatase alcaline

0,3 mL d'une solution de phosphatase alcaline (PAL) recombinante à 20 mg/mL (Roche, Ref. 03-535-452), sont dialysés en boyau Spectra/Por® (seuil de coupure 6000-8000 Da, Spectrum Laboratories, USA) contre 300 mL de tampon borate 50 mM pH 7,6, sous agitation magnétique, pendant une nuit, à 2-8°C. En sortie de dialyse, la concentration de la protéine est déterminée par lecture de la densité optique à 280 nm et cette concentration est ajustée à 8 mg/mL.

L'ester 17-β-hydroxy-3-β-carboxyméthoxyandrost-4-ène-N-hydroxysuccinimide (ou NHS) obtenu dans l'exemple 2 est repris en diméthylformamide (DMF) à une concentration de 1 mg/mL.

Pour un couplage de type (1-3) (1 mole de phosphatase alcaline-3 moles de dérivé de testostérone), 312,5 µL de la solution de PAL sont mélangés avec 30 µL de la solution d'ester 17-β-hydroxy-3-β-carboxyméthoxyandrost-4-ène-NHS. Pour un couplage de type (1-5), 312,5 µL de la solution de PAL sont mélangés avec 50 µL de la solution d'ester. Les mélanges sont incubés pendant 1h au bain-marie à 30°C, sous agitation magnétique douce.

Ensuite, la réaction est bloquée par addition de lysine 1 mM diluée dans l'eau. La quantité de lysine ajoutée est équimolaire à la quantité d'ester utilisée pour le couplage. On ajoute donc 68 µL de la solution de lysine pour le couplage de type (1-3) et 112 µL pour le couplage de type (1-5). Les mélanges sont incubés pendant 20 min sur une roue, à 18/25°C.

Après arrêt de la réaction, les conjugués sont dialysés en boyau Spectra/Por® (seuil de coupure environ 7000 Da) pendant 1h à 18/25°C contre 250 mL de tampon Tris 50 mM pH 7,4, NaCl 9 g/L, MgCl₂ 5 mM, ZnCl₂ 0,1 mM, azide 0,9 g/L, sous agitation magnétique. Au bout de 1h, les boyaux sont transférés dans de nouveaux bains contenant toujours 250 mL du même tampon. La dialyse est poursuivie sur la nuit à 2/8°C, sous agitation magnétique.

En sortie de dialyse, le volume des conjugués est complété à 1 mL avec du tampon de dialyse. Les conjugués sont ensuite purifiés par chromatographie d'interactions hydrophobes en utilisant une colonne RESOURCE Phenyl (Cat No. 17-1186-01, GE Healthcare Lifesciences) montée sur une chaîne de chromatographie ÄKTA. Le débit de la pompe est réglé à 0,5 ml/min. Le tampon TA est du Tris 50 mM pH 7,4, NaCl 9 g/L, MgCl₂ 5 mM, ZnCl₂ 0,1 mM, azide 0,9 g/L, (NH₄)₂SO₄ 0,8M. Le tampon TB est Tris 50 mM pH 7,4, NaCl 9 g/L, MgCl₂ 5 mM, ZnCl₂ 0,1 mM, azide 0,9 g/L, c'est le tampon de dialyse.

La colonne RESOURCE Phenyl est équilibré en tampon TA. Le conjugué à purifier est mélangé volume pour volume (475 µL conjugué et 475 µL tampon) avec le tampon Tris 50 mM pH 7,4, NaCl 9 g/L, MgCl₂ 5 mM, ZnCl₂ 0,1 mM, azide 0,9 g/L, (NH₄)₂SO₄ 1,6 M. Cette étape permet d'avoir le conjugué dans le tampon TA. L'injection du conjugué est suivie d'un lavage de 30 mL en tampon TA. Ensuite un gradient de 0 à 100% de TB est appliqué pendant 30 mL, puis un lavage en tampon TB pendant 10 mL et un lavage en eau pendant 10 mL. Le déroulement de la chromatographie est suivie par mesure de la densité optique à 280 nm. Les fractions à partir de 42 mL d'élution jusqu'à 51 mL (soit au total 10 mL) sont récupérées, rassemblées puis concentrées par diafiltration en utilisant une cellule Amicon (Amicon stirred cells, Millipore), une membrane Amicon PM avec un seuil de coupure de 10 000 Da et le tampon TB. Lors de cette étape, le volume de la solution de conjugué est réduit à environ 1 mL. Les conjugués sont conservés à 2/8°C jusqu'à leur utilisation dans un immunodosage.

### Exemple 10: Dosage de la testostérone en utilisant le Conjugué 1 de l'invention 17-β-hydroxy-3-β-carboxyméthoxyandrost-4-ène/phosphatase alcaline et comparaison avec des dosages utilisant un Conjugué de l'art antérieur

Le kit VIDAS® (bioMérieux) du dosage de la testostérone (Cat. No 30418) a été utilisé comme immunodosage de référence, il s'agit d'un kit commercial avec un marquage CE. Ce dosage est décrit dans la notice du coffret réf. 09345 F-fr-2010/08 et il est réalisé en utilisant l'automate d'immunoanalyse VIDAS®.

Le cône à usage unique sert à la fois de phase solide pour la réaction et de système de pipetage. La cartouche est composée de 10 puits recouverts d'une feuille d'aluminium scellée et étiquetée. Le premier puits comporte une partie prédécoupée pour faciliter l'introduction de l'échantillon. Le dernier puits est une cuvette optique dans laquelle la fluorescence du substrat est mesurée. Les différents réactifs nécessaires à l'analyse sont contenus dans les puits intermédiaires.

Le cône contenu dans le kit VIDAS® du dosage de la testostérone (bioMérieux Cat. No 30418) a été sensibilisé par un anticorps polyclonal de mouton anti-IgG de lapin, puis par un anticorps polyclonal de lapin anti-testostérone. La surface est passivée et il est prêt à l'emploi.

L'échantillon à doser est introduit dans le premier puits de la cartouche. Ensuite, toutes les étapes de la réaction de dosage sont réalisées automatiquement par l'appareil d'analyse VIDAS®. L'échantillon à doser est mélangé avec le conjugué qui est un dérivé de testostérone marqué à la phosphatase alcaline, mais dont la position 3 n'est pas modifiée comme dans la présente invention. On appellera ce conjugué « Conjugué VIDAS® » ou Conjugué de l'art antérieur.

Il s'effectue alors une compétition entre la testostérone présente dans l'échantillon et le dérivé testostérone du conjugué vis-à-vis des sites de l'anticorps anti-testostérone fixé sur le cône. Les étapes de lavage s'effectuent avec le tampon Tris-NaCl (0,05 mol/l) pH 7,4 ou de la diéthanolamine (1,1 mol/l) pH 9,8 et permettent d'éliminer les composés non fixés. Lors de l'étape finale de révélation, le substrat 4-Méthylombelliferyl phosphate est aspiré puis refoulé dans le cône ; l'enzyme du conjugué catalyse la réaction d'hydrolyse de ce substrat en 4-Méthylombelliferone dont la fluorescence émise est mesurée à 450 nm. La valeur du signal de fluorescence est inversement proportionnelle à la concentration de testostérone présente dans l'échantillon. Cette concentration est calculée par rapport à une courbe de calibration.

Pour les autres dosages, nous avons utilisé les réactifs du kit VIDAS® testostérone (Cat. No 30418) et le protocole de dosage indiqué dans la notice du kit, avec les modifications suivantes :
- Des cônes ont été sensibilisés avec d'autres anticorps anti-testostérone : 2 immunoglobulines G (IgG) monoclonales de souris, les clones 19E10H6 et 15H9H10 (bioMérieux), qu'on appellera respectivement Ab1 et Ab2 , et 1 IgG monoclonale de mouton, le clone testo3.6A3 (Bioventix), qu'on appellera Ab3. Comme dans le cône du format de test de référence, les anticorps anti-testostérone ont été capturés par des anticorps polyclonaux anti-espèces adsorbés sur le cône au préalable. Il s'agit d'un anticorps polyclonal de mouton anti-IgG de souris pour Ab1 et Ab2, et d'un anticorps polyclonal d'âne anti-IgG de mouton pour Ab3. Les anticorps anti-espèces sont dilués à une concentration entre 1 et 12 µg/mL dans la solution de sensibilisation. Après 6 h d'incubation à +18/25°C, un lavage est effectué avec une solution saline. Ensuite, la solution d'anticorps anti-testostérone contenant un agent de saturation de type protéique ou peptidique est ajoutée (concentration de l'anticorps : 0,01-0,2 µg/mL). La sensibilisation / passivation se poursuit à +18/25°C sur la nuit. Puis, les cônes sont vidés et séchés.

- Les cônes sensibilisés avec l'anticorps Ab1 sont testés avec le conjugué VIDAS®, dilué au 1/2,5^{ème} pour les essais avec l'anticorps monoclonal. Les cônes sensibilisés avec les anticorps Ab2 et Ab3 sont testés avec le conjugué 1 de l'invention préparé dans l'exemple 9. Ce dernier conjugué est utilisé à une concentration de 3 ng/mL pour les essais avec les cônes sensibilisés à l'anticorps Ab2 et 2,5 ng/mL pour les cônes sensibilisés à l'anticorps Ab3.
- La conversion en dose est réalisée grâce à une gamme étalon de 10 points fabriquée en supplémentant du sérum de femme avec de la testostérone en solution éthanolique. Les concentrations nominales de chacun des points sont : 0,001 ng/mL - 0,03 ng/mL - 0,18 ng/mL - 0,41 ng/mL - 0,76 ng/mL - 1,29 ng/mL - 1,91 ng/mL - 3,82 ng/mL - 7,48 ng/mL - 11,55 ng/mL.
- Le volume de prise d'échantillon pour chaque dosage est de 200 µL.

La gamme étalon a été mesurée avec chaque format de dosage. Le Tableau 1 ci-dessous résume les résultats obtenus en signal RFV (=relative fluorescence value) et ratio B/B0%, pour les dilutions de la gamme étalon. Le ratio B/B0 est le signal obtenu pour le point de gamme testé divisé par le signal obtenu pour le point de gamme 0,001 ng/mL de testostérone, multiplié par 100.

**Tableau 1**

| | REF = Kit VIDAS® | | Ab1 + Conjugué VIDAS® | | Ab2 + Conjugué 1 de l'invention | | Ab3 + Conjugué 1 de l'invention | |
|---|---|---|---|---|---|---|---|---|
| [c] Testostérone (ng/mL) | Signal (RFV) | B/B0% | Signal (RFV) | B/B0% | Signal (RFV) | B/B0% | Signal (RFV) | B/B0% |
| 0,001 | 4202 | 100 | 4799 | 100 | 4077 | 100 | 3209 | 100 |
| 0,03 | 3630 | 86 | 4412 | 92 | 3490 | 86 | 2492 | 78 |
| 0,18 | 3056 | 73 | 3537 | 74 | 2935 | 72 | 1700 | 53 |
| 0,41 | 2645 | 63 | 3093 | 64 | 2527 | 62 | 1403 | 44 |
| 0,76 | 2440 | 58 | 3320 | 69 | 2238 | 55 | 1286 | 40 |
| 1,29 | 2057 | 49 | 2940 | 61 | 1880 | 46 | 960 | 30 |
| 1,91 | 1939 | 46 | 2836 | 59 | 1729 | 42 | 990 | 31 |
| 3,82 | 1493 | 36 | 2391 | 50 | 1264 | 31 | 644 | 20 |
| 7,48 | 1183 | 28 | 2163 | 45 | 905 | 22 | 380 | 12 |
| 11,55 | 880 | 21 | 1849 | 39 | 632 | 16 | 205 | 6 |

Les valeurs de ratio B/B0% sont représentées sur la Figure 1 par un graphe qui donne ces valeurs en fonction du logarithme de la concentration des points de la gamme étalon pour chaque format, à savoir deux formats utilisant un conjugué de l'art antérieur ((i) REF qui est le dosage de référence de l'art antérieur qui correspond au kit commercial VIDAS® du dosage de la testostérone (bioMérieux, Cat. No 30418), et (ii) Ab1 + Conjugué VIDAS®), et deux formats utilisant un conjugué 1 de l'invention (Ab2 + Conjugué 1 de l'invention, et Ab3 + Conjugué 1 de l'invention).

Le graphe sur la Figure 1 montre que les deux formats de dosage les plus sensibles sont ceux utilisant utilisent le conjugué de l'invention. En effet, le format de test le plus sensible est le format associant l'anticorps Ab3 et le Conjugué 1 de l'invention. Une diminution du signal de 50% est observée dès 0,2 ng/mL de testostérone environ. Pour le format associant l'anticorps Ab2 et le Conjugué 1 de l'invention, ainsi que le dosage de référence sur VIDAS®, il faut environ 1 ng/mL de testostérone pour atteindre un ratio B/B0 de 50%. Le format Ab2 + Conjugué 1 de l'invention est légèrement plus sensible que le format de référence. Finalement, avec le format Ab1 + Conjugué VIDAS®, il faut 3,82 ng/mL de testostérone pour atteindre les 50%. C'est le format le moins sensible.

Dans un deuxième temps, pour confirmer que le format Ab2 + Conjugué 1 de l'invention est bien plus sensible que le format de référence REF, kit VIDAS® Testostérone, dont le domaine de mesure préconisé dans la notice s'étend de 0,1 à 13 ng/mL, ces deux formats ont été comparés sur des échantillons de sérum obtenus auprès des Etablissements Français du Sang. Il s'agit de 4 échantillons recueillis chez des femmes (codes en F) chez qui la dose de testostérone sérique est inférieure à 1 ng/mL et de 2 échantillons recueillis chez des hommes (codes en H). Les résultats sont présentés dans le Tableau 2. La concentration théorique a été déterminée par un laboratoire tiers, avec une technique qui permet de mesurer de façon juste les concentrations inférieures à 1 ng/mL comme la spectrométrie de masse ID/GC-MS. Le ratio [c] mesurée / [c] théorique X 100 présenté dans le Tableau 2 pour chaque dosage permet d'estimer la justesse de chaque dosage, [c] signifiant concentration. Plus ce ratio est proche de 100%, plus le dosage est juste.

**Tableau 2**

| | | REF = Kit VIDAS® | | Ab2 + Conjugué 1 de l'invention | |
|---|---|---|---|---|---|
| Code échantillon | [c] théorique | [c] mesurée | [c] me / [c] th x 100 | [c] mesurée | [c] me / [c] th x100 |
| F37 | 0,11 | 0,25 | 223 | 0,13 | 118 |
| F38 | <0,025 | 0,09 | 340 | 0,03 | 100 |
| F45 | 0,07 | 0,20 | 279 | 0,07 | 100 |
| F46 | 0,21 | 0,41 | 193 | 0,30 | 140 |
| H29 | 2,38 | 3,20 | 134 | 2,97 | 125 |
| H34 | 3,94 | 3,35 | 85 | 4,05 | 103 |

Les résultats dans le Tableau 2 montrent que le format utilisant un conjugué de l'invention est plus juste que le dosage de référence sur VIDAS®, surtout pour le dosage des faibles taux de testostérone sérique trouvés chez les femmes.

### Exemple 11 : Préparation du Conjugué 2 de l'invention 17-β-hydroxy-3-β-carboxyméthoxyandrost-4-ène/biotine

La molécule sera appelée ici de façon abrégée testostérone-3β-EMC-DAP-biotine, DAP étant le diaminopropyle et présente la formule suivante.

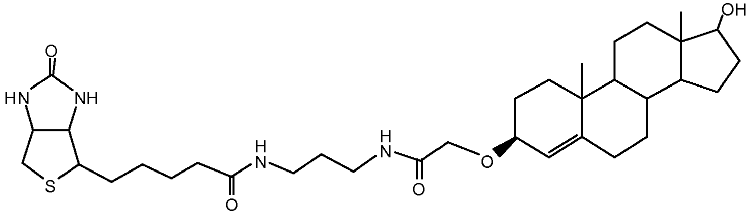

Dans un ballon de 50 mL muni d'une agitation magnétique, 100 mg (0,287 mmole) d'acide 17-β-hydroxy-3β-carboxyméthoxyandrost-4-ène obtenus dans l'exemple 1 sont dissouts dans 7 mL de diméthoxyéthane (DME) anhydride. 50 mg de N-hydroxysuccinimide (NHS) sont ajoutés et l'agitation est maintenue à température ambiante pendant 3 min. 69 mg (0,334 mmole) de N, N'-dicyclohexylcarbodiimide (DCC) sont introduits et une solution transparente est obtenue. Le milieu de réaction est agité à température ambiante pendant 1 nuit.

Le mélange de réaction est filtré et le filtrat ainsi obtenu est directement utilisé. 100 mg (0,241 mmole) de N-(+)-Biotinyl-3-aminopropylammonium trifluoroacetate (biotine-NH-DAP, sel de TFA, Sigma-Aldrich Cat. No. 71776) sont mélangés avec 1,5 mL de solution de NaHCO₃ à 1N puis ajoutés dans le filtrat. L'agitation est maintenue à température ambiante pendant 14 h.

Le milieu de réaction est séché à température ambiante sous pression réduite. Le résidu obtenu est purifié en chromatographie sur gel de silice 60 (0,040-0,063 mm, Merck Cat. No. 109385) avec l'éluant : dichlorométhane/méthanol, 8/1, v/v pour commencer et dichlorométhane méthanol, 5/1, v/v pour finir la purification. 125 mg de produit sont obtenus, ce qui correspond à un rendement de 82%. La testostérone-3β-EMC-DAP-biotine est une poudre blanche..

Le produit est analysée en chromatographie liquide haute pression (HPLC). La colonne utilisée est une Thermokromasil C18, 150x4,6mm et l'éluant est un mélange acétonitrile, eau (0,1% acide trifluoroacétique) en gradient. La chromatographie est suivie par la mesure de l'absorbance à 214 nm. La pureté du produit ainsi estimée est de 90,1%.

### Exemple 12 : Dosage de la testostérone en utilisant le Conjugué 2 de l'invention testostérone-3β-EMC-DAP/biotine

Dans une microplaque 96 puits (Nunc Maxisorp F96) sont distribués 100 µL/puits de l'anticorps monoclonal de souris anti-Fc IgG de chèvre (clone 9A4A5, bioMérieux) dilué à 10 µg/mL en tampon PBS 1x. La microplaque est incubée sur la nuit à température ambiante afin d'obtenir l'adsorption de l'anticorps. La microplaque est vidée, puis 300 µL/puits de tampon de passivation (tampon Tris 0,2 M pH 6,2) contenant un agent de saturation de type protéique ou peptidique, et l'anticorps anti-testostérone monoclonal de mouton clone 3.6A3 (Ab3 - bioVentix) dilué à 0,2 µg/mL sont ajoutés. La microplaque est incubée pendant 1 h à 37°C. Trois lavages TBS (Tris buffered saline)-Tween^{®}-20 0,05%. sont effectués. Les points de gamme utilisés dans l'Exemple 10 sont dilués au ½ dans un tampon Tris ; 100 µL de ces dilutions sont distribués dans les puits de la plaque. Après 1 h d'incubation à 37°C, les puits sont vidés et 0,2 ng/puits du Conjugué 2 de l'invention testostérone-3β-EMC-DAP-biotine en tampon Tris sont ajoutés. La réaction avec le conjugué est effectuée pendant 15 minutes à 37°C, et est suivie de 3 lavages. 100 µL/puits d'une solution de streptavidine-peroxydase (Jackson Imunoresearch Cat. No. 016-30-084) diluée au 1/20,000 en TBS-Tween^{®}-20 0,05%., BSA 2% sont ajoutés et la microplaque est incubée pendant 30 min à 37°C. Après 3 lavages, 100 µL/puits du substrat 1-step Ultra TMB (Thermo Scientific, Cat. No. 34028) sont ajoutés et la microplaque est incubée pendant 5 min à température ambiante, à l'abri de la lumière. La réaction est ensuite arrêtée par ajout de 100 µL d'acide sulfurique 2M. La densité optique (DO) à 450 nm et à 630 nm est mesurée dans un lecteur de microplaque. Pour chaque puits, on soustrait la densité optique à 630 nm de la densité optique à 450 nm. Les résultats obtenus en appliquant ce protocole sont présentés dans le Tableau 3.

**Tableau 3**

| [c] Testostérone (ng/mL) | DO₄₅₀-DO₆₃₀ | B/B0% |
|---|---|---|
| 0,001 | 2,52 | 100 |
| 0,03 | 2,10 | 83 |
| 0,18 | 1,88 | 74 |
| 0,41 | 1,61 | 64 |
| 0,76 | 1,36 | 54 |
| 1,29 | 1,00 | 40 |
| 3,82 | 0,42 | 17 |
| 11,55 | 0,17 | 7 |

Les valeurs de ratio B/B0% du Tableau 3 sont représentées sur la Figure 2 par un graphe qui donne ces valeurs en fonction du logarithme de la concentration des points de la gamme étalon. La Figure 2 reprend les valeurs obtenues dans l'exemple 10 pour le format utilisant Ab2 + Conjugué 1 de l'invention.

Ce graphe montre que l'allure de la gamme de dosage du testostérone obtenu avec l'anticorps Ab3 + le Conjugué 2 de l'invention testostérone-3β-EMC-DAP-biotine sur microplaque est comparable à celle obtenue par le format Ab2 + Conjugué 1 de l'invention sur VIDAS® dans l'Exemple 10. Ce dosage en microplaque avec un conjugué de l'invention est donc plus sensible que les dosages VIDAS® utilisant le Conjugué VIDAS®.

### Références Bibliographiques

Cekan, S. Z., 1979, J. Steroid Biochem., II : 1629.
Cook B and Bestall G.H., 1987, Steroid hormones a practical approach, Ed Green B. and Leake R.E., Chapter 1.
Demers L.M., 2010, Maturitas, 67 : 39-45.
Fiet J et al., 2004, Steroids, 69(7) : 461-471.
Litwack G., 1992, Biochemistry of Hormones II: Steroid hormones . In DEVLIN T. M., Textbook of Chemistry with clinical correlations, 3,d Edition, Wiley J. and Sons, 901-925.
Moneti G ; et al., 1987, J. Steroid Biochem., 27(1-3) : 53.
Rassasie, M.J., et al., 1992, Steroids, 57: 112.
Rosner W. et al., 2007, JCEM, 92(2) : 405-413.
Owen, W.E., et al., 2010, Clinica Chimica Acta, 411: 1073.
Stabler T.V., et al., 1991, Clin. Chem., 37(11): 1987.
Thienpont, L.M., et al., 2008, Clin. Chem., 54(8):1290.
Ueschiba, H. et al., 1991, Clin. Chem., 37(8) : 1329.
Vingler P., et al., 1991, J. of Chromatography, 571 : 73.
Wong, S.S., 1991, Reactive groups of proteins and their modifying agents. In Chemistry of protein conjugation and cross-linking, CRC Press Inc, p33-39.
Wudy, S.A., et al., 1992, Steroids, 57 : 319.

## Revendications

1. Dérivé de testostérone de formule générale (I) suivante : 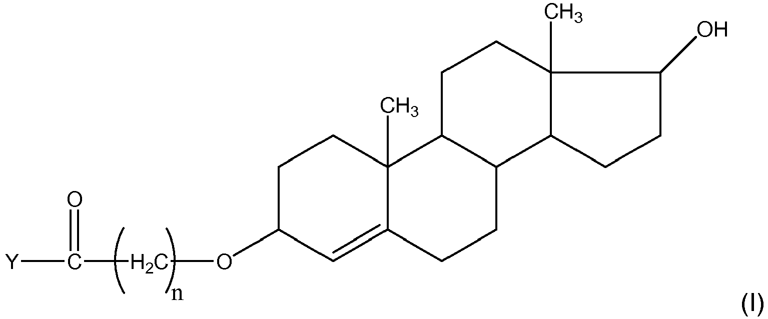 dans laquelle n est un nombre entier compris entre 1 et 10 et Y représente un groupement activé ou prêt à être activé qui permet la formation d'une liaison amide avec une amine primaire d'une molécule.

2. Dérivé de testostérone selon la revendication 1, **caractérisé en ce que** n est compris entre 1 et 5

3. Dérivé de testostérone selon la revendication 1 ou 2, **caractérisé en ce que** Y est choisi parmi choisi parmi -OH, -NH-(CH₂)ₘ-COOH, -N₃, 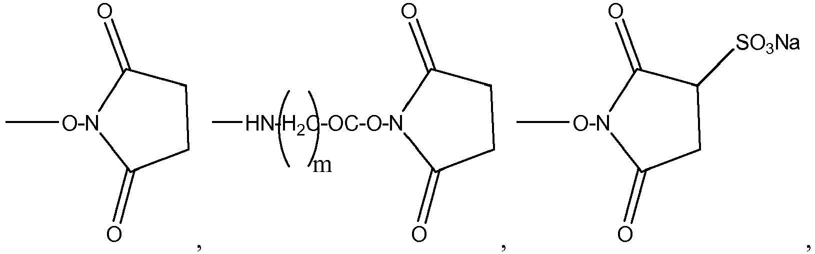 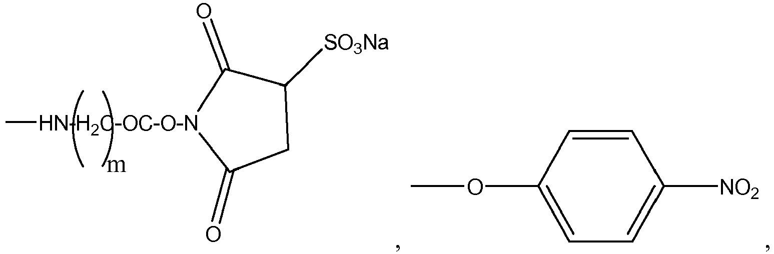 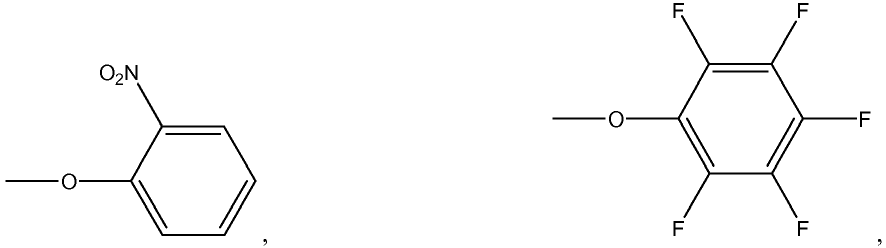 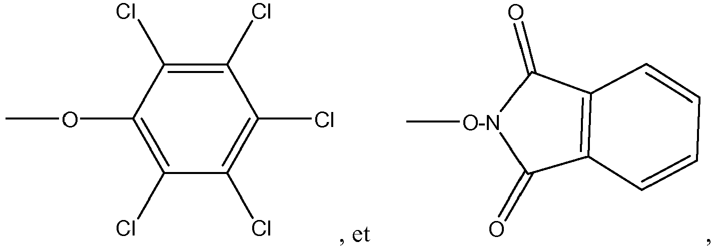 m étant un nombre entier compris entre 1 et 10.

4. Dérivé de testostérone selon la revendication 3, **caractérisé en ce que** m est compris entre 1 et 5.

5. Dérivé de testostérone selon la revendication 1 ou 2, **caractérisé en ce que** Y est choisi parmi -OH et -NH-(CH₂)ₘ-COOH et 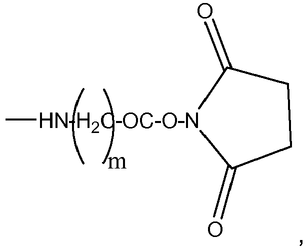 m étant compris entre 1 et 10 et de préférence m étant égal à 1.

6. Dérivé de testostérone selon l'une quelconque des revendications 1 à 5, **caractérisé en ce qu'**il est sous forme de dérivé β au niveau du carbone en position 3.

7. Dérivé de testostérone selon l'une quelconque des revendications 1 à 5, **caractérisé en ce qu'**il est sous forme de dérivé α au niveau du carbone en position 3.

8. Conjugué constitué d'un dérivé de testostérone tel que décrit dans l'une quelconque des revendications 1 à 7, et d'un marqueur.

9. Utilisation d'un dérivé de testostérone tel que décrit dans l'une quelconque des revendications 1 à 7 ou d'un conjugué tel que décrit dans la revendication 8 pour la détermination de la concentration en testostérone dans un échantillon biologique.

10. Procédé de détermination de la concentration en testostérone par immunodosage par compétition dans un échantillon biologique, comprenant les étapes consistant à :
a) mettre en présence, au sein dudit échantillon, (i) un partenaire de liaison de la testostérone et (ii) un composé choisi parmi un dérivé de testostérone tel que décrit dans l'une quelconque des revendications 1 à 7 et un conjugué tel que décrit dans la revendication 8, l'un desdits composants (i) et (ii) étant adapté pour émettre un signal,
b) éventuellement laisser un laps de temps suffisant pour permettre la réaction de compétition et
c) mesurer l'intensité du signal et en déduire la concentration en testostérone par comparaison à une courbe de calibration établissant une relation entre intensité du signal mesurée et concentration en testostérone.

11. Procédé de détermination de la concentration en testostérone selon la revendication 10, **caractérisé en ce que** le composé (ii) est un conjugué tel que décrit dans la revendication 8.

12. Trousse de diagnostic permettant la mise en oeuvre du procédé selon la revendication 10 ou 11, comprenant un dérivé de testostérone tel que décrit dans l'une quelconque des revendications 1 à 7 ou un conjugué tel que décrit dans la revendication 8.

13. Utilisation d'un composé de formule (Ia) pour la préparation d'un dérivé de testostérone de formule (I) tel que décrit dans l'une quelconque des revendications 1 à 7, la formule (Ia) étant : 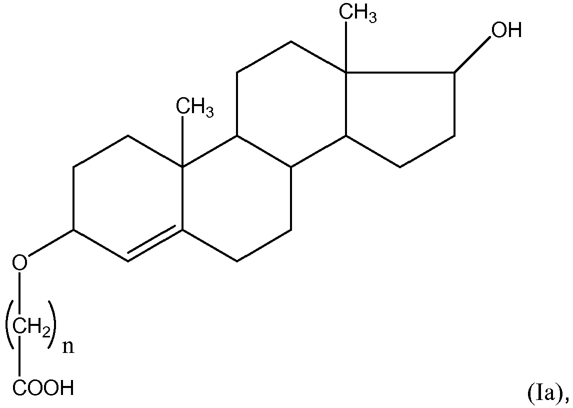 n étant un nombre entier compris entre 1 et 10.

14. Procédé de préparation d'un dérivé de testostérone, sous forme d'isomère β en position 3, de formule générale (Iaβ) suivante : 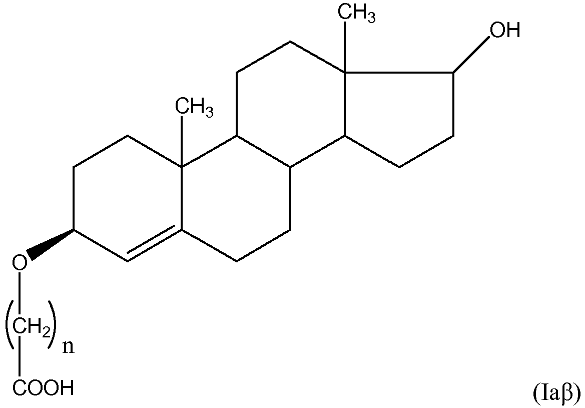 dans laquelle n est un nombre entier compris entre 1 et 10, comprenant les étapes consistant à :
(1) mettre à réagir la testostérone avec de l'anhydride acétique pour protéger la fonction OH de la position 17, puis avec un agent réducteur, en présence d'un solvant, pour réduire le carbonyle en position 3 et obtenir le composé de formule (IIβ) : 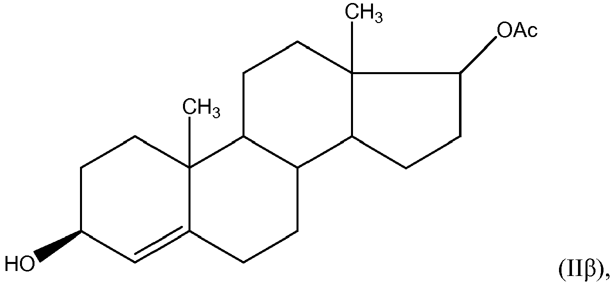 dans laquelle Ac signifie -CO-CH₃,
(2) mettre à réagir le composé de formule (IIβ) ainsi obtenu avec le composé de formule (III) : N₂CH-(CH₂)ₙ₋₁-COOC₂H₅, puis mettre à réagir avec une base, en présence d'un solvant, pour obtenir le composé de formule (Iaβ).

15. Procédé de préparation d'un dérivé de testostérone, sous forme d'isomère β en position 3, de formule générale (Ibβ) suivante : 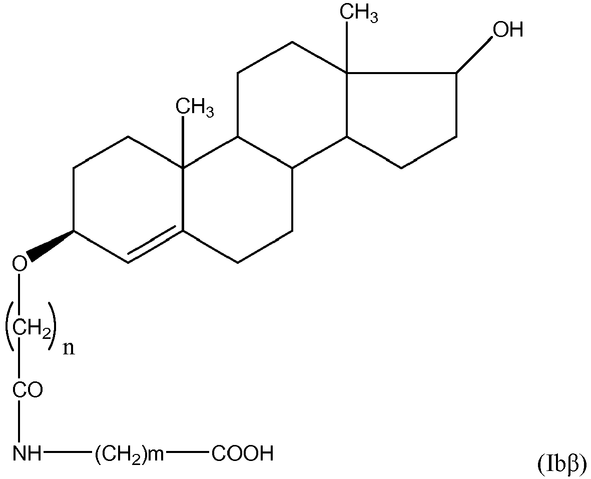 dans laquelle n est un nombre entier compris entre 1 et 10, m est un nombre entier compris entre 1 et 10, comprenant les étapes consistant à :
(1) mettre à réagir la testostérone avec de l'anhydride acétique pour protéger la fonction OH de la position 17, puis avec un agent réducteur, en présence d'un solvant, pour réduire le carbonyle en position 3 et obtenir le composé de formule (IIβ) : 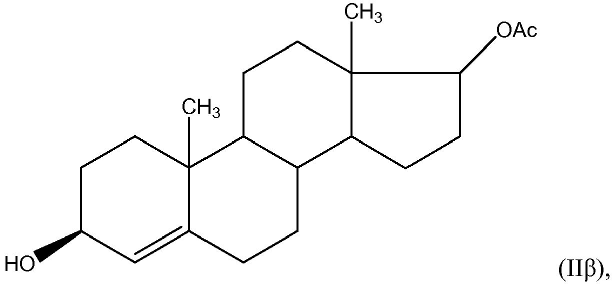 dans laquelle Ac signifie -CO-CH₃,
(2) mettre à réagir le composé de formule (IIβ) ainsi obtenu avec le composé de formule (III) : N₂CH-(CH₂)ₙ₋₁-COOC₂H₅, puis mettre à réagir avec une base, en présence d'un solvant, pour obtenir le composé de formule (Iaβ) : 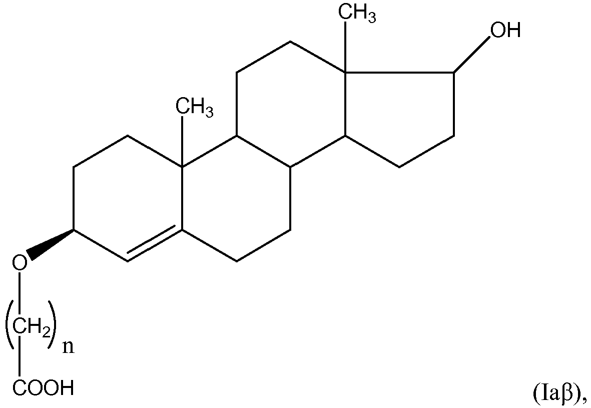
(3) mettre à réagir le composé de formule (Iaβ) ainsi obtenu avec du N-hydroxysuccinimide, en présence d'un dérivé de carbodiimide, pour produire le composé de formule (IVβ) : 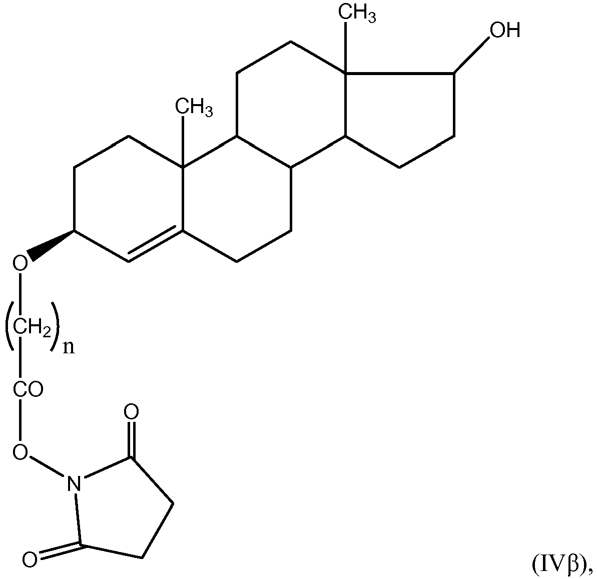
(4) mettre à réagir le composé de formule (IVβ) ainsi obtenu avec le composé de formule (V) : H₂N-(CH₂)m-COOR₁, dans laquelle R₁ est un groupement alkyle ou aryle, pour produire le composé de formule (VIβ) : 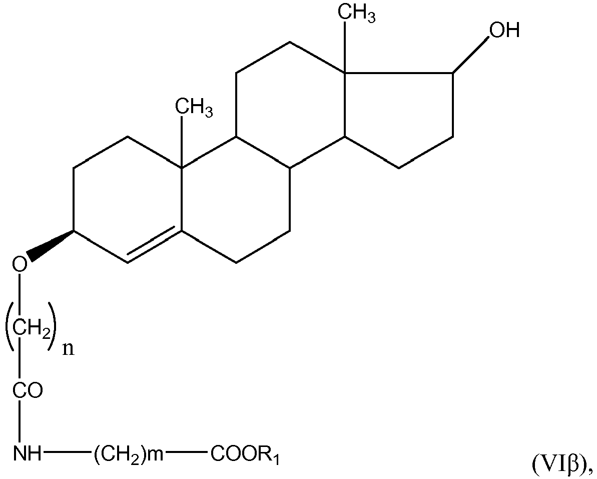
(5) mettre à réagir le composé de formule (VIβ) ainsi obtenu avec une base, en présence d'un solvant, pour obtenir le composé de formule (Ibβ).

16. Procédé de préparation d'un dérivé de testostérone, sous forme d'isomère β en position 3, de formule générale (Iβ) suivante : 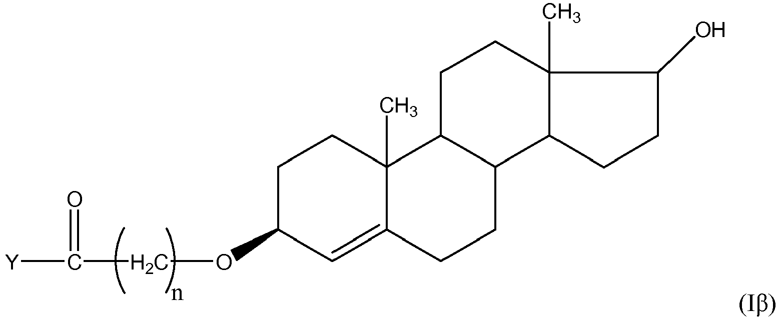 dans laquelle n est un nombre entier compris entre 1 et 10 et Y représente : 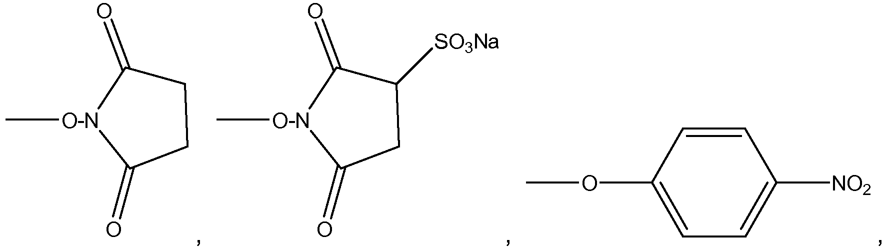 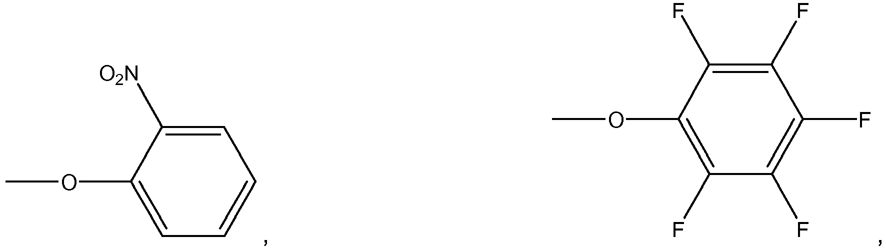 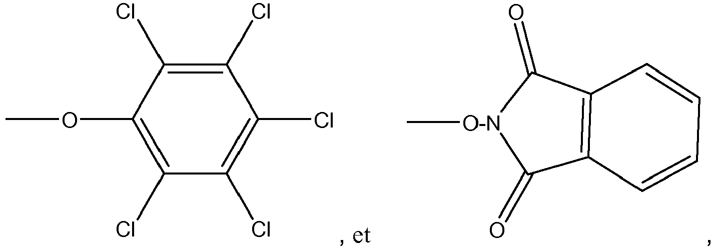 comprenant les étapes consistant à :
(1) mettre à réagir la testostérone avec de l'anhydride acétique pour protéger la fonction OH de la position 17, puis avec un agent réducteur, en présence d'un solvant, pour réduire le carbonyle en position 3 et obtenir le composé de formule (IIβ) : 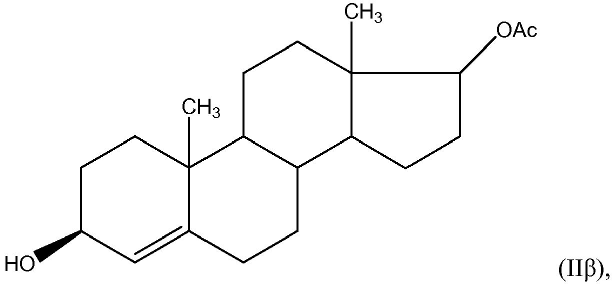 dans laquelle Ac signifie -CO-CH₃
(2) mettre à réagir le composé de formule (IIβ) ainsi obtenu avec le composé de formule (III) : N₂CH-(CH₂)ₙ₋₁-COOC₂H₅, puis mettre à réagir avec une base, en présence d'un solvant, pour obtenir le composé de formule (Iaβ) : 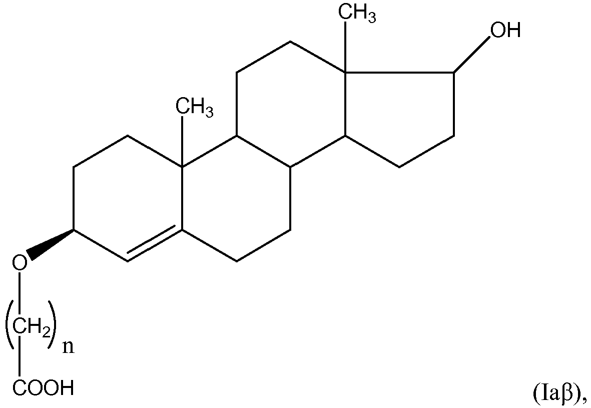
(3) mettre à réagir le composé de formule (Iaβ) ainsi obtenu, en présence d'un dérivé de carbodiimide, avec un réactif choisi, selon le groupement Y final, parmi les composés suivants, pour obtenir les composés de formule (Iβ) :

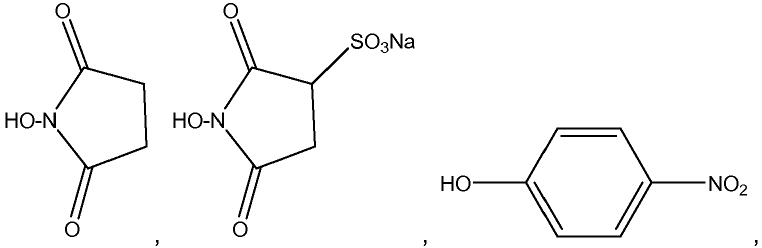

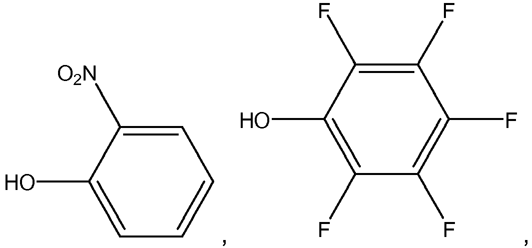

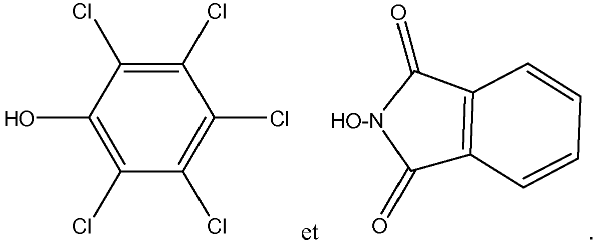

17. Procédé de préparation d'un dérivé de testostérone, sous forme d'isomère β en position 3, de formule générale (Iβ) suivante : 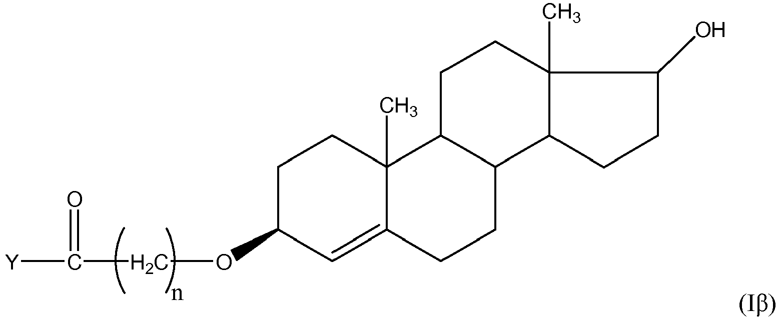 dans laquelle n est un nombre entier compris entre 1 et 10 et Y représente : 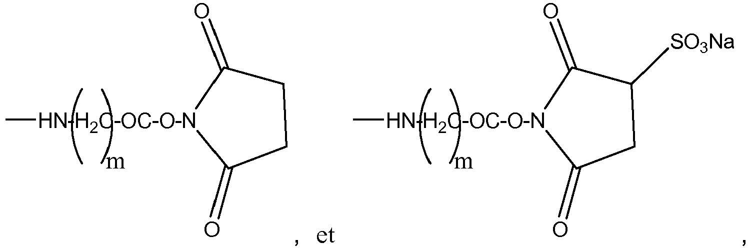 m étant un nombre entier compris entre 1 et 10 , comprenant les étapes consistant à :
(1) mettre à réagir la testostérone avec de l'anhydride acétique pour protéger la fonction OH de la position 17, puis avec un agent réducteur, en présence d'un solvant, pour réduire le carbonyle en position 3 et obtenir le composé de formule (IIβ) : 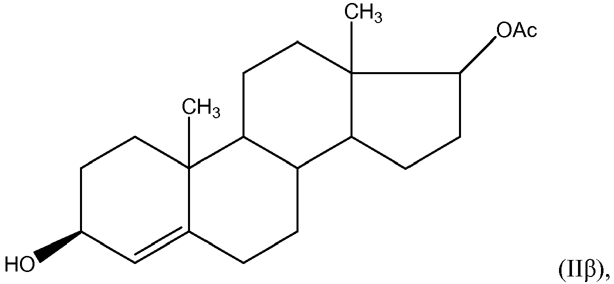 dans laquelle Ac signifie -CO-CH₃,
(2) mettre à réagir le composé de formule (IIβ) ainsi obtenu avec le composé de formule (III) : N₂CH-(CH₂)ₙ₋₁-COOC₂H₅, puis mettre à réagir avec une base, en présence d'un solvant, pour obtenir le composé de formule (Iaβ) : 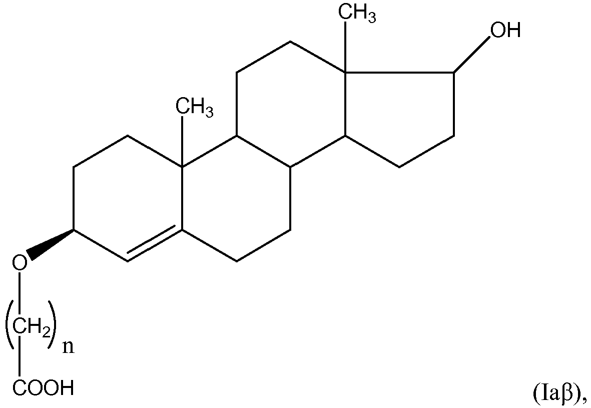
(3) mettre à réagir le composé de formule (Iaβ) ainsi obtenu avec du N-hydroxysuccinimide, en présence d'un dérivé de carbodiimide, pour produire le composé de formule (IVβ) : 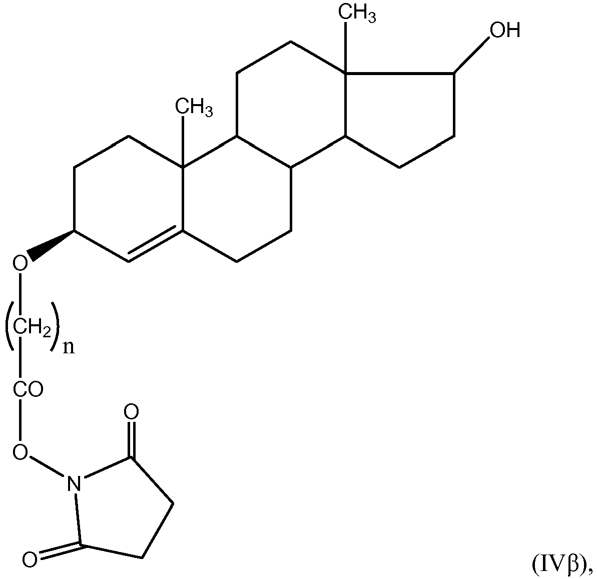
(4) mettre à réagir le composé de formule (IVβ) ainsi obtenu avec le composé de formule (V) : H₂N-(CH₂)m-COOR₁, dans laquelle R₁ est un groupement alkyle ou aryle, pour produire le composé de formule (VIβ) : 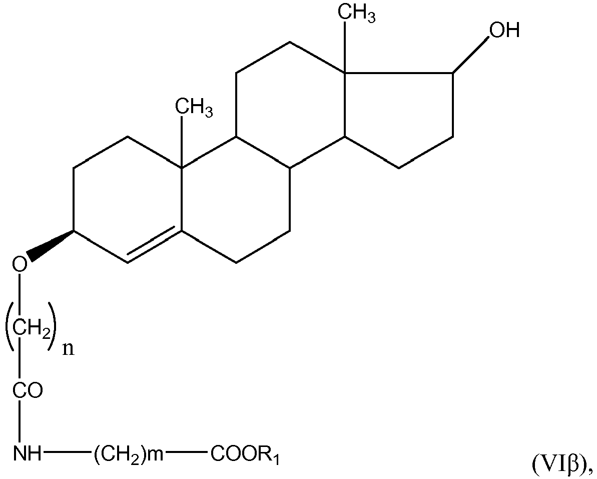
(5) mettre à réagir le composé de formule (VIβ) ainsi obtenu avec une base, en présence d'un solvant, pour obtenir le composé de formule (Ibβ) : 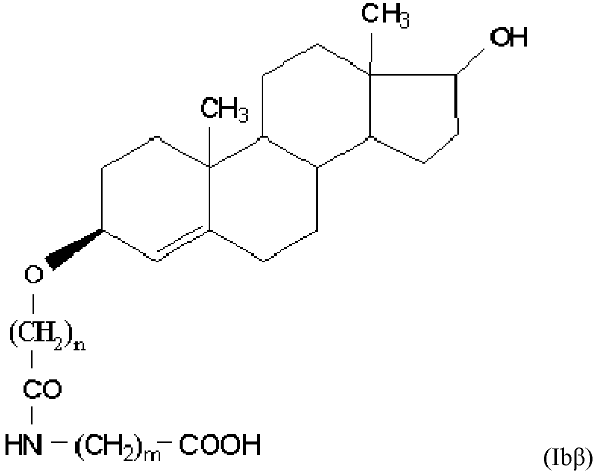
(6) mettre à réagir le composé de formule (Ibβ) ainsi obtenu, en présence d'un dérivé de carbodiimide, avec un réactif choisi, selon le groupement Y final, parmi les composés suivants, pour obtenir les composés de formule (Iβ) :

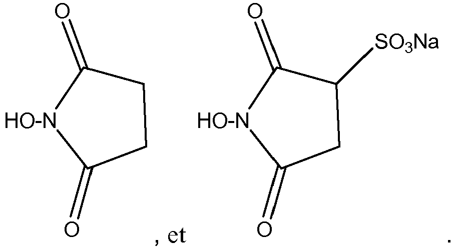

18. Procédé de préparation d'un dérivé de testostérone, sous forme d'isomère β en position 3, de formule générale (Icβ) suivante : 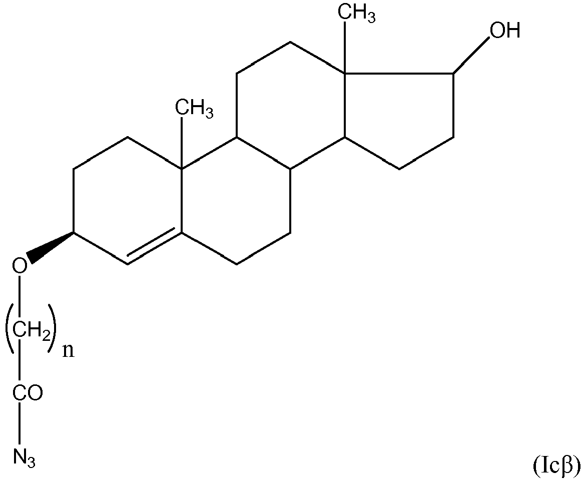 dans laquelle n est un nombre entier compris entre 1 et 10, comprenant les étapes consistant à :
(1) mettre à réagir la testostérone avec de l'anhydride acétique pour protéger la fonction OH de la position 17, puis avec un agent réducteur, en présence d'un solvant, pour réduire le carbonyle en position 3 et obtenir le composé de formule (IIβ) : 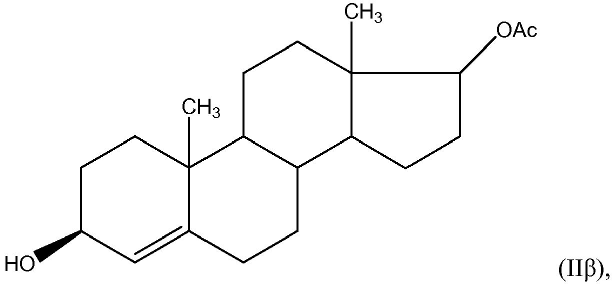 dans laquelle Ac signifie -CO-CH₃,
(2) mettre à réagir le composé de formule (IIβ) ainsi obtenu avec le composé de formule (III) : N₂-CH-(CH)ₙ₋₁COOC₂H₅, puis mettre à réagir avec une base, en présence d'un solvant, pour obtenir le composé de formule (Iaβ) : 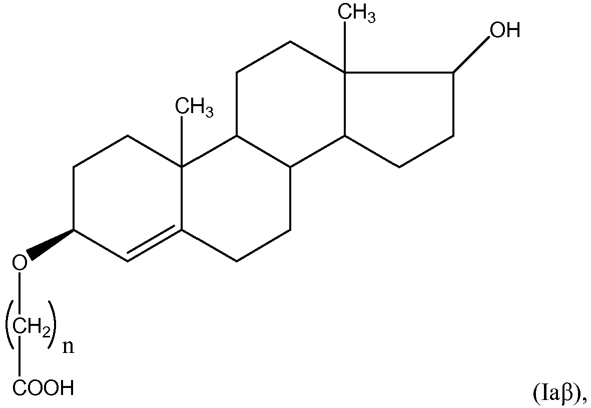
(3) mettre à réagir le composé de formule (Iaβ) ainsi obtenu, en présence d'un dérivé de carbodiimide, avec du H₂NNH₂, pour obtenir le composé de formule (VIIβ) : 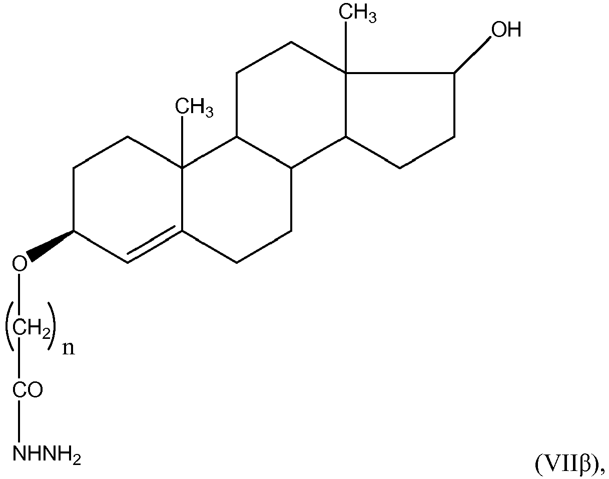
(4) mettre à réagir le composé de formule (VIIβ) ainsi obtenu avec du HONO pour obtenir le composé de formule (Icβ).

19. Procédé de préparation d'un dérivé de testostérone, sous forme d'isomère α en position 3, de formule générale (Iaα) suivante : 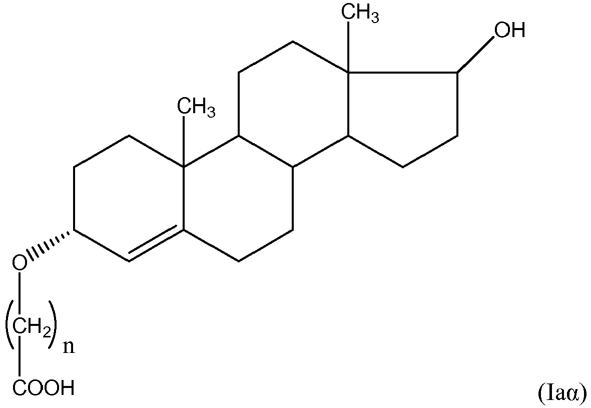 dans laquelle n est un nombre entier compris entre 1 et 10, comprenant les étapes consistant à :
(1) mettre à réagir la testostérone avec du t-butyldiméthylchlorosilane pour protéger la fonction OH de la position 17, puis avec un agent réducteur, en présence d'un solvant, pour réduire le carbonyle en position 3 et obtenir le composé de formule (VIIIβ) : 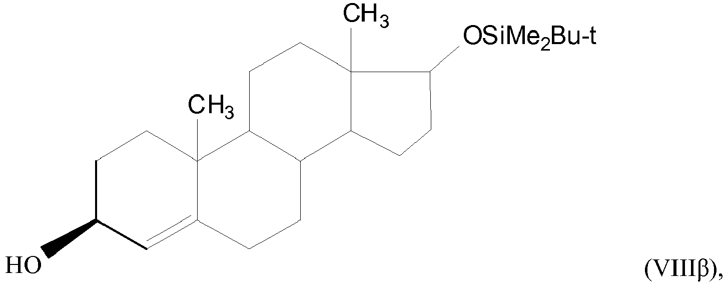 dans laquelle -SiMe₂Bu-t signifie t-butyldiméthylsilanyle,
(2) mettre à réagir le composé de formule (VIIIβ) ainsi obtenu avec de la triphénylphosphine, de l'acide benzoïque et de l'azodicarboxylate de diéthyle, puis avec une base, en présence d'un solvant, pour transformer, en position 3, l'isomère β en isomère α et obtenir ainsi le composé de formule (VIIIα) : 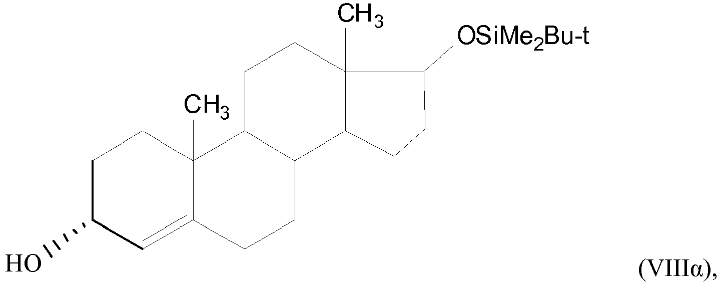 dans laquelle -SiMe₂Bu-t signifie t-butyldiméthylsilanyle,
(3) mettre à réagir le composé de formule (VIIIα) ainsi obtenu avec le composé de formule (III) : N₂CH-(CH)ₙ₋₁COOC₂H₅, puis mettre à réagir avec une base, en présence d'un solvant, pour obtenir le composé de formule (Iaα).

20. Procédé de préparation d'un dérivé de testostérone, sous forme d'isomère α en position 3, de formule générale (Ibα) suivante : 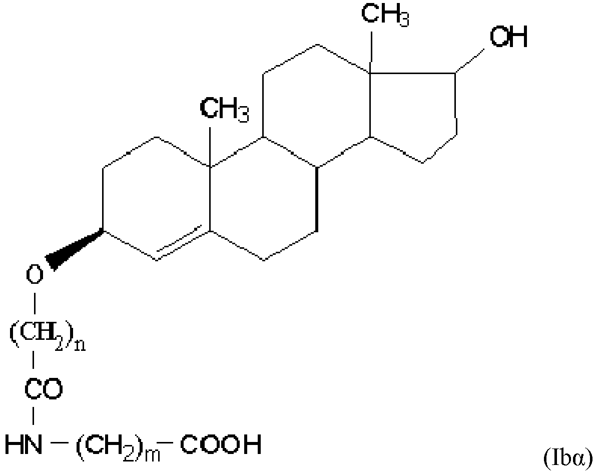 dans laquelle n est un nombre entier compris entre 1 et 10, m est un nombre entier compris entre 1 et 10, comprenant les étapes consistant à :
(1) mettre à réagir la testostérone avec du t-butyldiméthylchlorosilane pour protéger la fonction OH de la position 17, puis avec un agent réducteur, en présence d'un solvant, pour réduire le carbonyle en position 3 et obtenir le composé de formule (VIIIβ) : 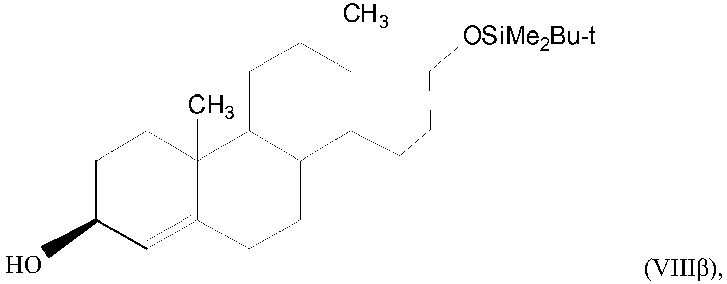 dans laquelle -SiMe₂Bu-t signifie t-butyldiméthylsilanyle,
(2) mettre à réagir le composé de formule (VIIIβ) ainsi obtenu avec de la triphénylphosphine, de l'acide benzoïque et de l'azodicarboxylate de diéthyle, puis avec une base, en présence d'un solvant, pour transformer, en position 3, l'isomère β en isomère α et obtenir ainsi le composé de formule (VIIIα) : 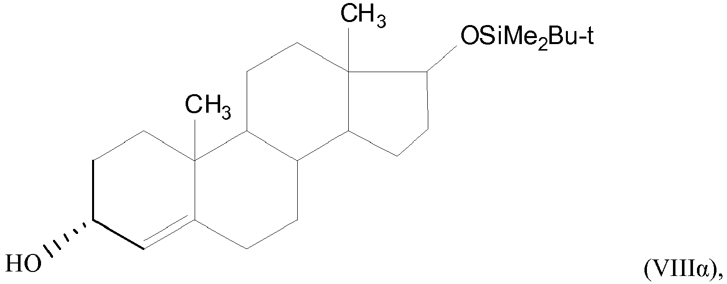 dans laquelle -SiMe₂Bu-t signifie t-butyldiméthylsilanyle,
(3) mettre à réagir le composé de formule (VIIIα) ainsi obtenu avec le composé de formule (III) : N₂CH-(CH₂)ₙ₋₁-COOC₂H₅, puis mettre à réagir avec une base, en présence d'un solvant, pour obtenir le composé de formule (Iaα) : 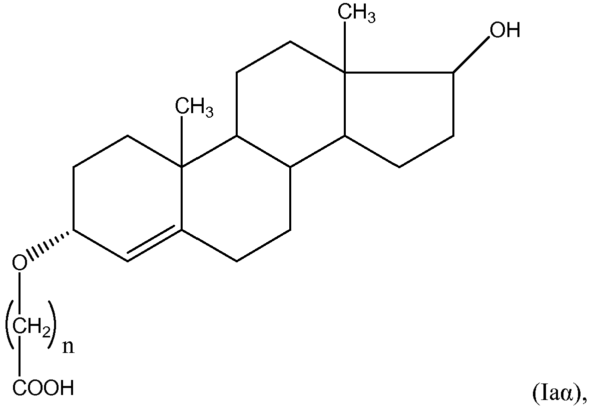
(4) mettre à réagir le composé de formule (Iaα) ainsi obtenu avec du N-hydroxysuccinimide, en présence d'un dérivé de carbodiimide, pour produire le composé de formule (IVα) : 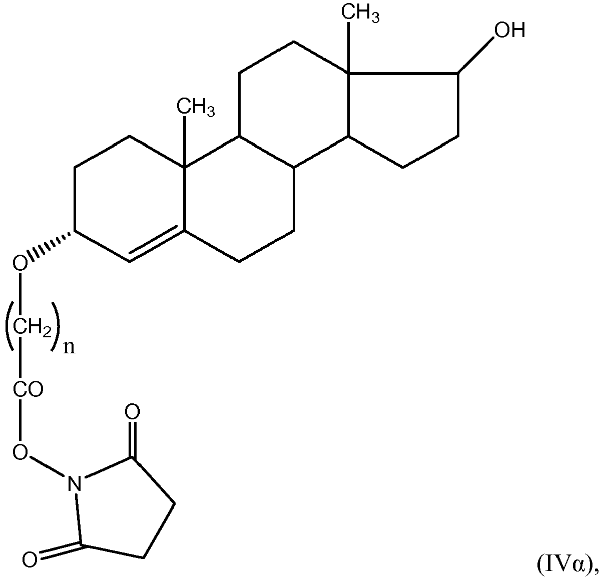
(5) mettre à réagir le composé de formule (IVα) ainsi obtenu avec le composé de formule (V) : H₂N-(CH₂)m-COOR₁, dans laquelle R₁ est un groupement alkyle ou aryle, pour produire le composé de formule (VIα) : 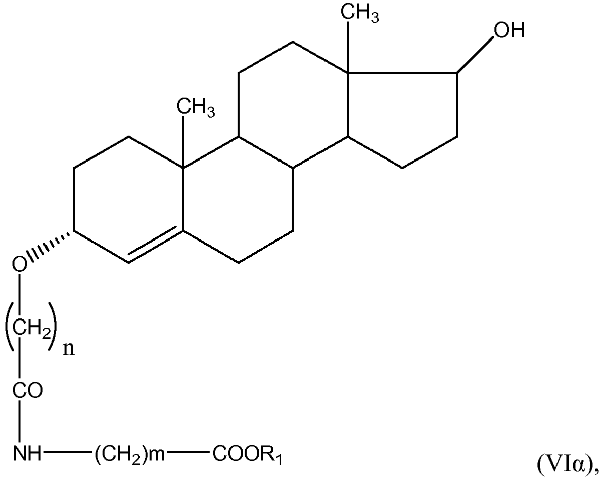
(6) mettre à réagir le composé de formule (VIα) ainsi obtenu avec une base, en présence d'un solvant, pour obtenir le composé de formule (Ibα).

21. Procédé de préparation d'un dérivé de testostérone, sous forme d'isomère α en position 3, de formule générale (Iα) suivante : 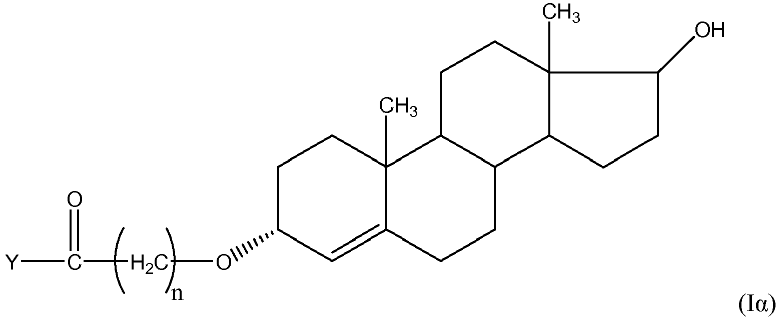 dans laquelle n est un nombre entier compris entre 1 et 10 et Y représente : 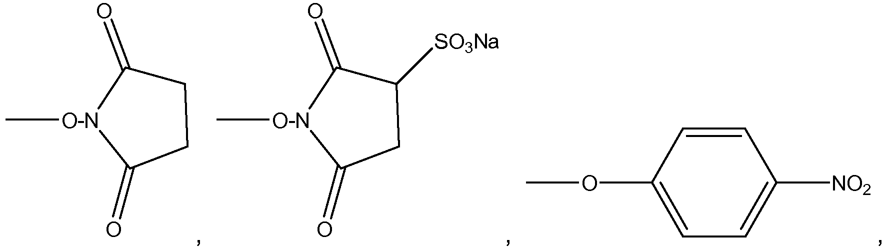 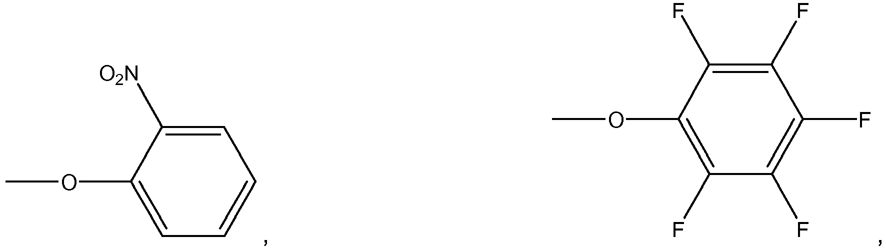 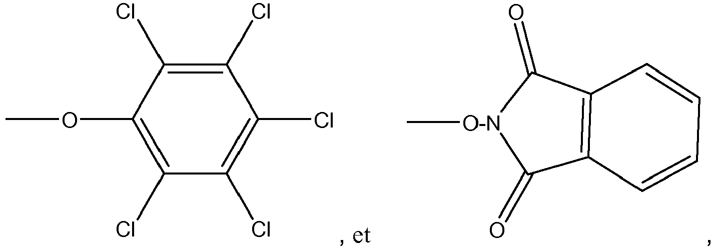 comprenant les étapes consistant à :
(1) mettre à réagir la testostérone avec du t-butyldiméthylchlorosilane pour protéger la fonction OH de la position 17, puis avec un agent réducteur, en présence d'un solvant, pour réduire le carbonyle en position 3 et obtenir le composé de formule (VIIIβ) : 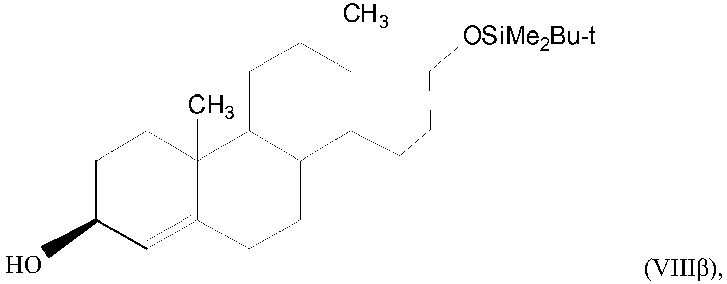 dans laquelle -SiMe₂Bu-t signifie t-butyldiméthylsilanyle,
(2) mettre à réagir le composé de formule (VIIIβ) ainsi obtenu avec de la triphénylphosphine, de l'acide benzoïque et de l'azodicarboxylate de diéthyle, puis avec une base, en présence d'un solvant, pour transformer, en position 3, l'isomère β en isomère α et obtenir ainsi le composé de formule (VIIIα) : 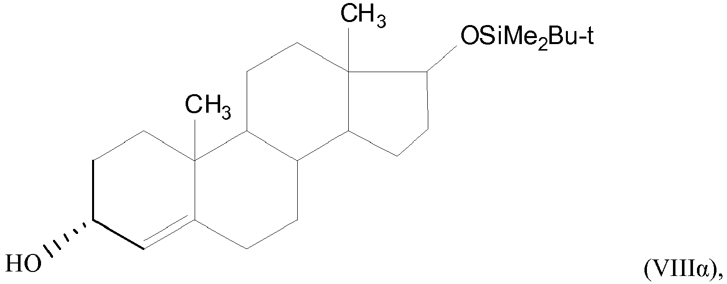 dans laquelle -SiMe₂Bu-t signifie t-butyldiméthylsilanyle,
(3) mettre à réagir le composé de formule (VIIIα) ainsi obtenu avec le composé de formule (III) : N₂CH-(CH₂)ₙ₋₁-COOC₂H₅, puis mettre à réagir avec une base, en présence d'un solvant, pour obtenir le composé de formule (Iaα) : 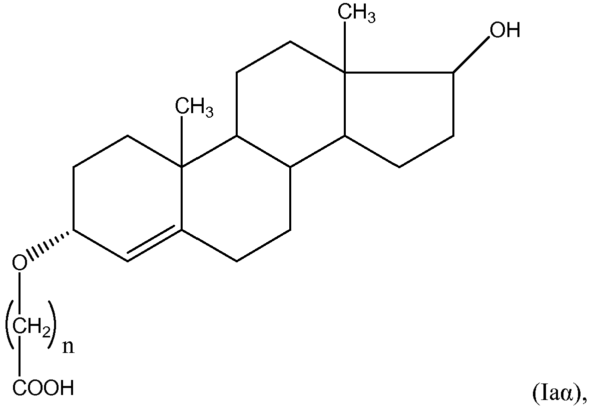
(4) mettre à réagir le composé de formule (Iaα) ainsi obtenu, en présence d'un dérivé de carbodiimide, avec un réactif choisi, selon le groupement Y final, parmi les composés suivants, pour obtenir un composé de formule (Iα) :

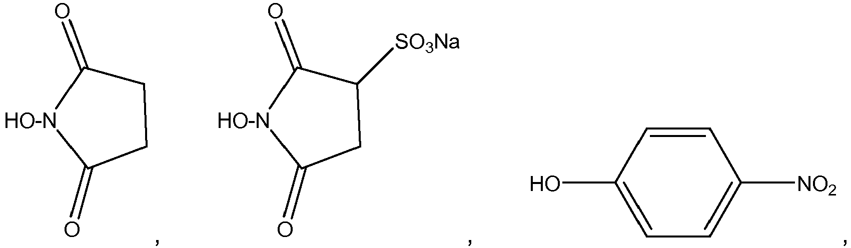

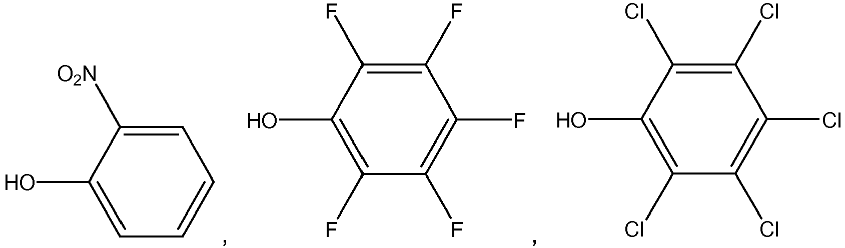

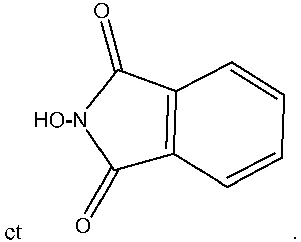

22. Procédé de préparation d'un dérivé de testostérone, sous forme d'isomère α en position 3, de formule générale (Iα) suivante : 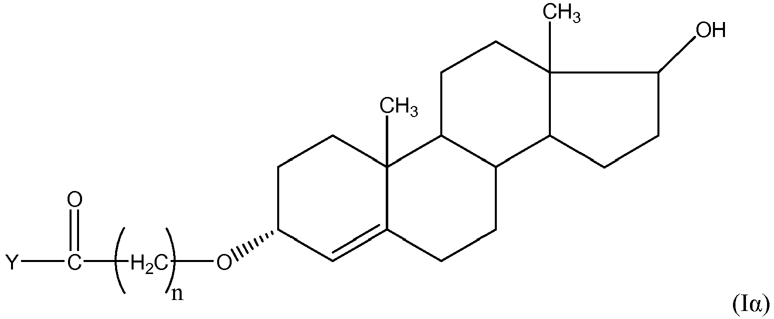 dans laquelle n est un nombre entier compris entre 1 et 10 et Y représente : 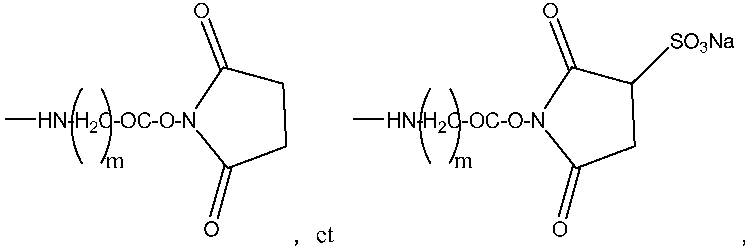 m étant un nombre entier compris entre 1 et 10, comprenant les étapes consistant à :
(1) mettre à réagir la testostérone avec du t-butyldiméthylchlorosilane pour protéger la fonction OH de la position 17, puis avec un agent réducteur, en présence d'un solvant, pour réduire le carbonyle en position 3 et obtenir le composé de formule (VIIIβ) : 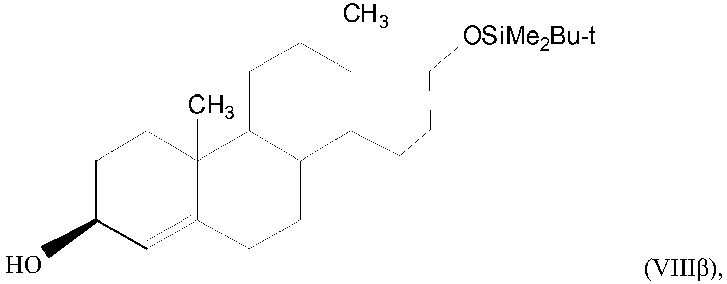 dans laquelle -SiMe₂Bu-t signifie t-butyldiméthylsilanyle,
(2) mettre à réagir le composé de formule (VIIIβ) ainsi obtenu avec de la triphénylphosphine, de l'acide benzoïque et de l'azodicarboxylate de diéthyle, puis avec une base, en présence d'un solvant, pour transformer, en position 3, l'isomère β en isomère α et obtenir ainsi le composé de formule (VIIIα) : 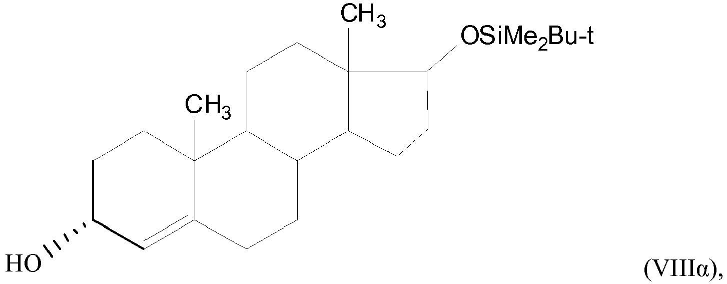 dans laquelle -SiMe₂Bu-t signifie t-butyldiméthylsilanyle,
(3) mettre à réagir le composé de formule (VIIIα) ainsi obtenu avec le composé de formule (III) : N₂CH-(CH₂)ₙ₋₁-COOC₂H₅, puis mettre à réagir avec une base, en présence d'un solvant, pour obtenir le composé de formule (Iaα) : 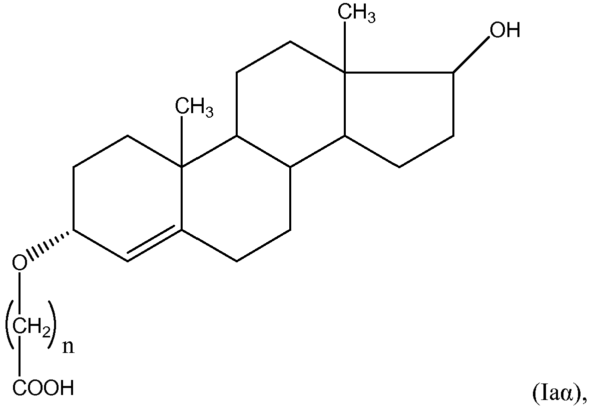
(4) mettre à réagir le composé de formule (Iaα) ainsi obtenu avec du N-hydroxysuccinimide, en présence d'un dérivé de carbodiimide, pour produire le composé de formule (IVα) : 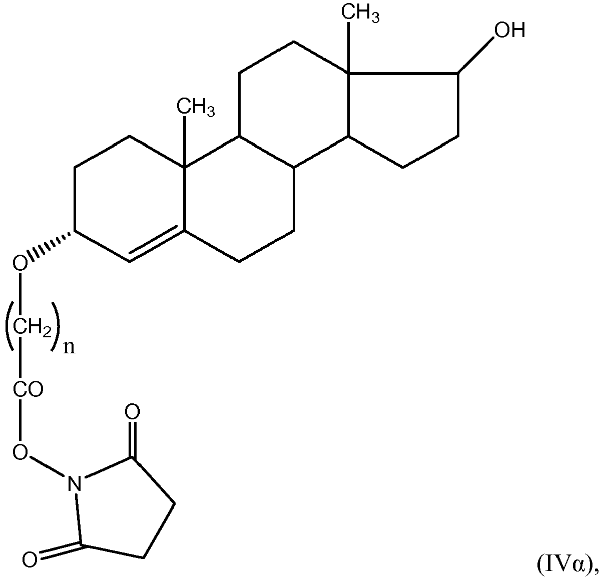
(5) mettre à réagir le composé de formule (IVα) ainsi obtenu avec le composé de formule (V) : H₂N-(CH₂)m-COOR₁, dans laquelle R₁ est un groupement alkyle ou aryle, pour produire le composé de formule (VIα) : 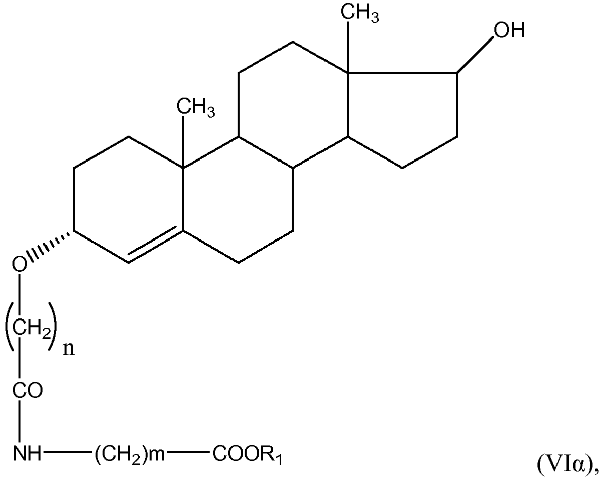
(6) mettre à réagir le composé de formule (VIα) ainsi obtenu avec une base, en présence d'un solvant, pour obtenir le composé de formule (Ibα) : 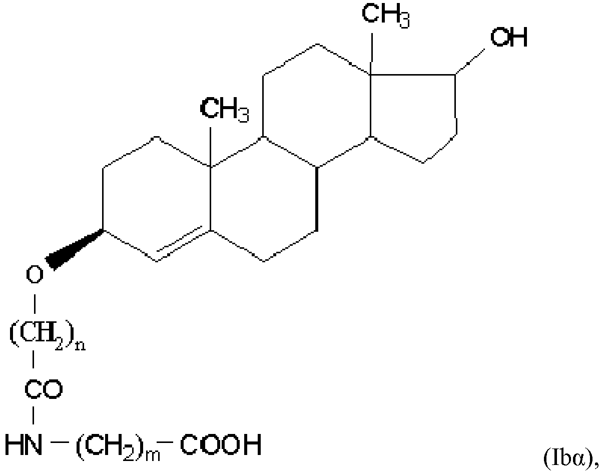
(7) mettre à réagir le composé de formule (Ibα) ainsi obtenu, en présence d'un dérivé de carbodiimide, avec un réactif choisi, selon le groupement Y final, parmi les composés suivants, pour obtenir un composé de formule (Iα) :

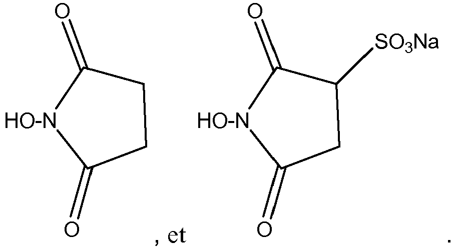

23. Procédé de préparation d'un dérivé de testostérone, sous forme d'isomère α en position 3, de formule générale (Icα) suivante : 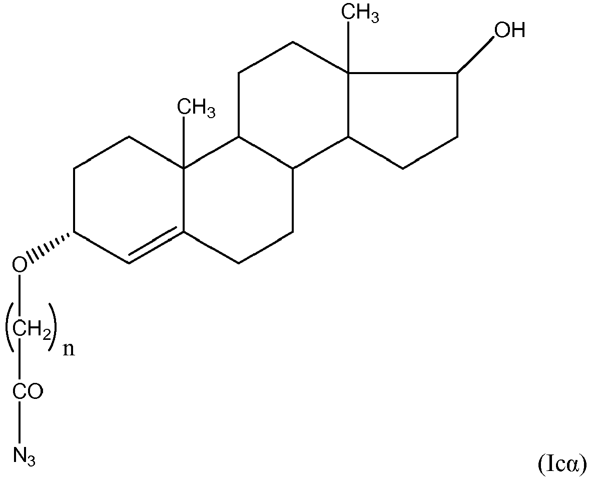 dans laquelle n est un nombre entier compris entre 1 et 10, comprenant les étapes consistant à :
(1) mettre à réagir la testostérone avec du t-butyldiméthylchlorosilane pour protéger la fonction OH de la position 17, puis avec un agent réducteur, en présence d'un solvant, pour réduire le carbonyle en position 3 et obtenir le composé de formule (VIIIβ) : 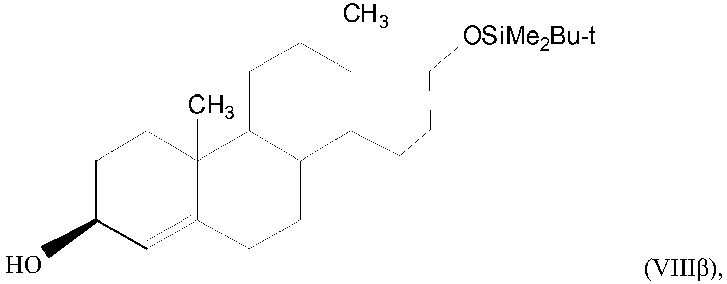 dans laquelle -SiMe₂Bu-t signifie t-butyldiméthylsilanyle,
(2) mettre à réagir le composé de formule (VIIIβ) ainsi obtenu avec de la triphénylphosphine, de l'acide benzoïque et de l'azodicarboxylate de diéthyle, puis avec une base, en présence d'un solvant, pour transformer, en position 3, l'isomère β en isomère α et obtenir ainsi le composé de formule (VIIIα) : 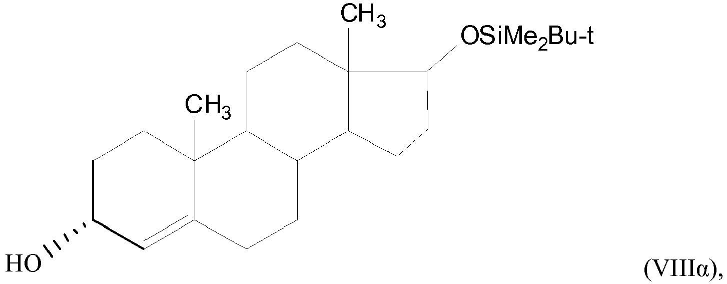 dans laquelle -SiMe₂Bu-t signifie t-butyldiméthylsilanyle,
(3) mettre à réagir le composé de formule (VIIIα) ainsi obtenu avec le composé de formule (III) : N₂CH-(CH₂)ₙ₋₁-COOC₂H₅, puis mettre à réagir avec une base, en présence d'un solvant, pour obtenir le composé de formule (Iaα) : 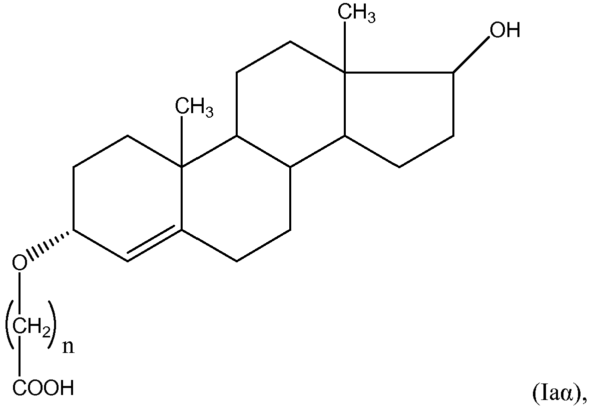
(4) mettre à réagir le composé de formule (Iaα) ainsi obtenu, en présence d'un dérivé de carbodiimide, avec du H₂NNH₂, pour obtenir le composé de formule (VIIα) : 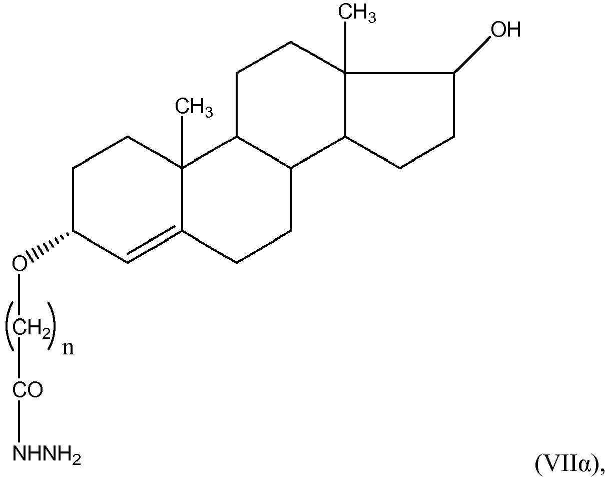
(5) mettre à réagir le composé de formule (VIIα) ainsi obtenu avec du HONO pour obtenir le composé de formule (Icα).

## Patentansprüche

1. Testosteronderivat der folgenden allgemeinen Formel (I): 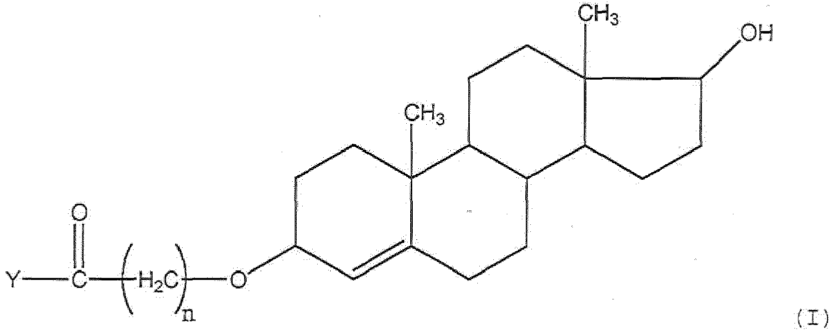 in der n für eine ganze Zahl zwischen 1 und 10 steht und Y für eine aktivierte oder aktivierungsbereite Gruppe, die die Bildung einer Amidbindung mit einem primären Amin eines Moleküls erlaubt, steht.

2. Testosteronderivat nach Anspruch 1, **dadurch gekennzeichnet, dass** n zwischen 1 und 5 liegt.

3. Testosteronderivat nach Anspruch 1 oder 2, **dadurch gekennzeichnet, dass** Y aus -OH, -NH-(CH₂)ₘ-COOH, -N₃, 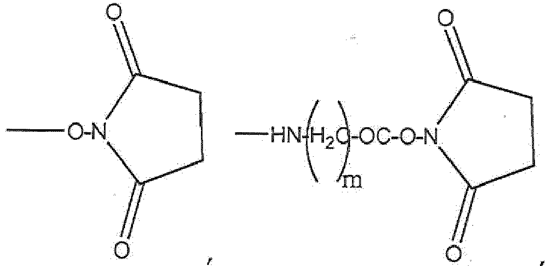 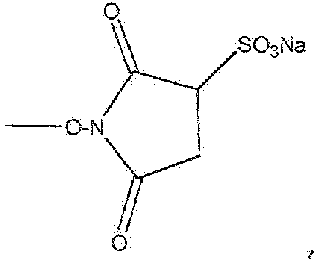 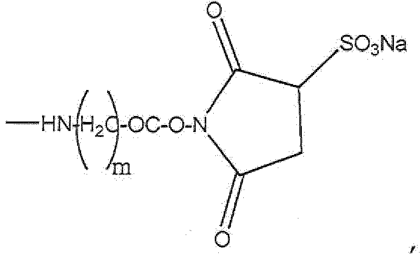 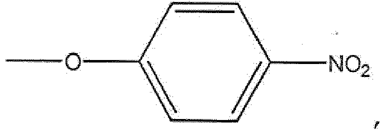 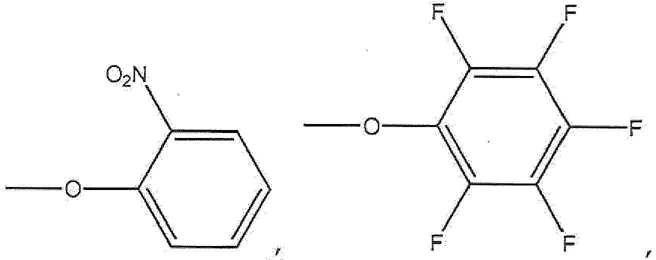 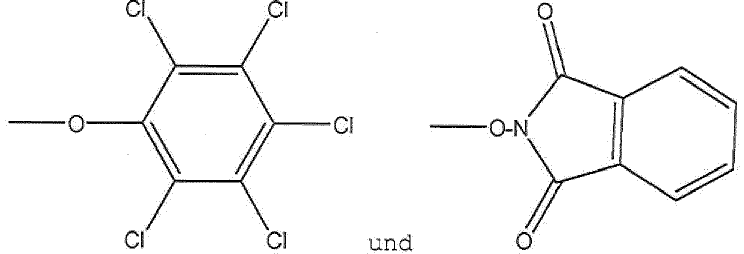 ausgewählt ist, wobei m für eine ganze Zahl zwischen 1 und 10 steht.

4. Testosteronderivat nach Anspruch 3, **dadurch gekennzeichnet, dass** m zwischen 1 und 5 liegt.

5. Testosteronderivat nach Anspruch 1 oder 2, **dadurch gekennzeichnet, dass** Y aus -OH und -NH-(CH₂)ₘ-COOH und 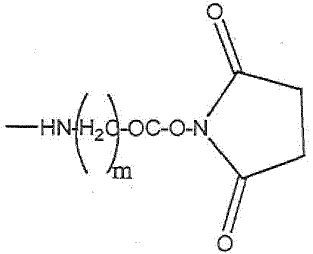 ausgewählt ist, wobei m zwischen 1 und 10 liegt und vorzugsweise gleich 1 ist.

6. Testosteronderivat nach einem der Ansprüche 1 bis 5, **dadurch gekennzeichnet, dass** es in Form eines β-Derivats an dem Kohlenstoff in 3-Position vorliegt.

7. Testosteronderivat nach einem der Ansprüche 1 bis 5, **dadurch gekennzeichnet, dass** es in Form eines α-Derivats an dem Kohlenstoff in 3-Position vorliegt.

8. Konjugat, bestehend aus einem Testosteronderivat gemäß einem der Ansprüche 1 bis 7 und einem Marker.

9. Verwendung eines Testosteronderivats gemäß einem der Ansprüche 1 bis 7 oder eines Konjugats gemäß Anspruch 8 zur Bestimmung der Konzentration von Testosteron in einer biologischen Probe.

10. Verfahren zur Bestimmung der Konzentration von Testosteron durch einen kompetitiven Immunassay in einer biologischen Probe, das folgende Schritte umfasst:
a) Inberührungbringen von (i) einem Testosteron-Bindungspartner und (ii) einer aus einem Testosteronderivat gemäß einem der Ansprüche 1 bis 7 und einem Konjugat gemäß Anspruch 8 ausgewählten Verbindung in der Probe, wobei eine der Komponenten (i) und (ii) dafür ausgelegt ist, ein Signal zu emittieren,
b) gegebenenfalls Warten über einen ausreichenden Zeitraum zum Ablaufenlassen der kompetitiven Reaktion und
c) Messen der Intensität des Signals und Ableiten der Konzentration von Testosteron daraus durch Vergleich mit einer Kalibrierkurve, die einen Zusammenhang zwischen der Intensität des gemessenen Signals und der Konzentration von Testosteron herstellt.

11. Verfahren zur Bestimmung der Konzentration von Testosteron nach Anspruch 10, **dadurch gekennzeichnet, dass** es sich bei der Verbindung (ii) um ein Konjugat gemäß Anspruch 8 handelt.

12. Diagnosekit zur Durchführung des Verfahrens nach Anspruch 10 oder 11, umfassend ein Testosteronderivat gemäß einem der Ansprüche 1 bis 7 oder ein Konjugat gemäß Anspruch 8.

13. Verwendung einer Verbindung der Formel (Ia) zur Herstellung eines Testosteronderivats der Formel (I) gemäß einem der Ansprüche 1 bis 7, wobei es sich bei der Formel (Ia) um: 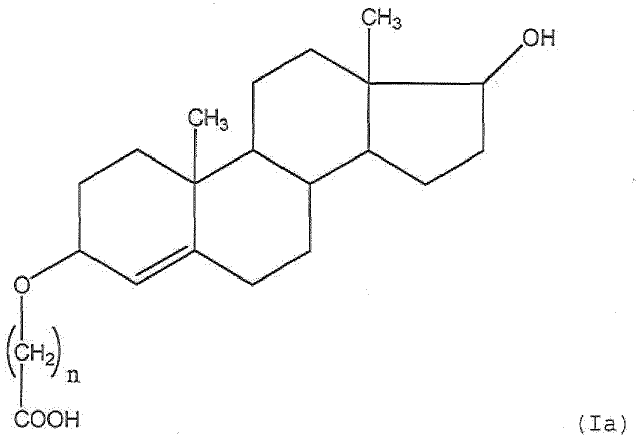 handelt, wobei n für eine ganze Zahl zwischen 1 und 10 steht.

14. Verfahren zur Herstellung eines Testosteronderivats in Form eines β-Isomers in 3-Position der folgenden allgemeinen Formel (Iaβ) : 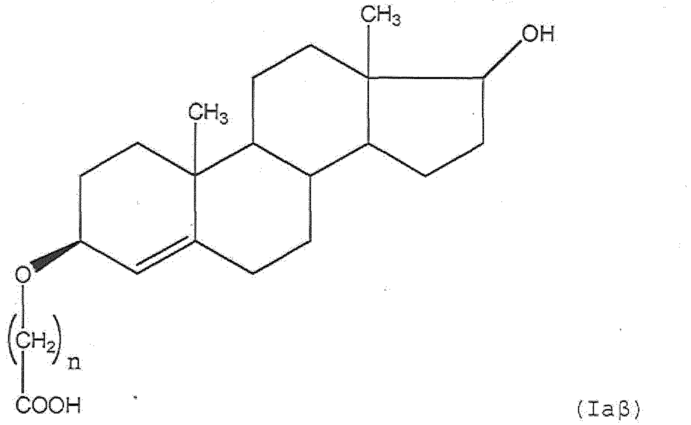 in der n für eine ganze Zahl zwischen 1 und 10 steht, das folgende Schritte umfasst:
(1) Umsetzen von Testosteron mit Essigsäureanhydrid zum Schützen der OH-Funktion in 17-Position und dann mit einem Reduktionsmittel in Gegenwart eines Lösungsmittels zur Reduktion des Carbonyls in 3-Position und zum Erhalt der Verbindung der Formel (IIβ): 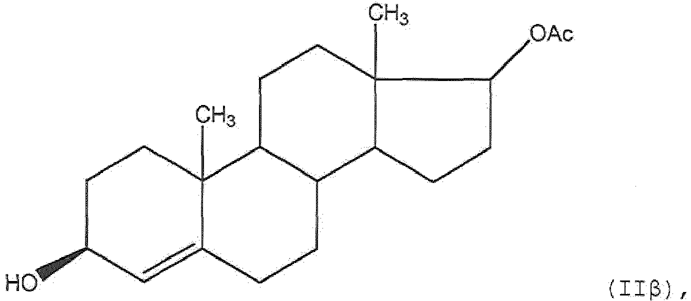 in der Ac für -CO-CH₃ steht,
(2) Umsetzen der so erhaltenen Verbindung der Formel (IIβ) mit der Verbindung der Formel (III) : N₂CH-(CH₂)ₙ₋₁-COOC₂H₅ und anschließendes Umsetzen mit einer Base in Gegenwart eines Lösungsmittels zum Erhalt der Verbindung der Formel (Iaβ).

15. Verfahren zur Herstellung eines Testosteronderivats in Form eines β-Isomers in 3-Position der folgenden allgemeinen Formel (Ibβ): 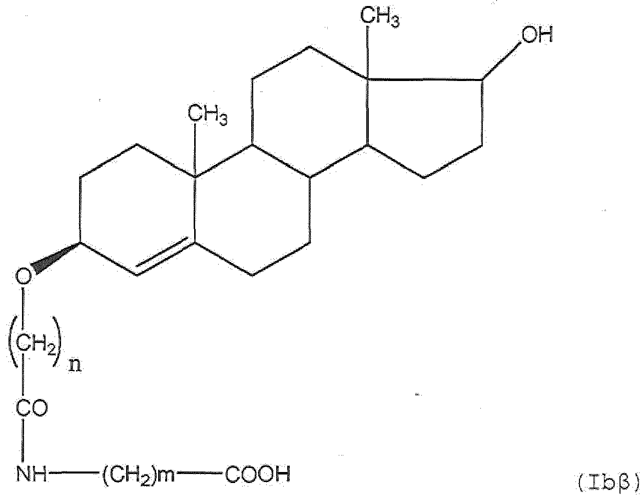 in der n für eine ganze Zahl zwischen 1 und 10 steht, m für eine ganze Zahl zwischen 1 und 10 steht, das folgende Schritte umfasst:
(1) Umsetzen von Testosteron mit Essigsäureanhydrid zum Schützen der OH-Funktion in 17-Position und dann mit einem Reduktionsmittel in Gegenwart eines Lösungsmittels zur Reduktion des Carbonyls in 3-Position und zum Erhalt der Verbindung der Formel (IIβ): 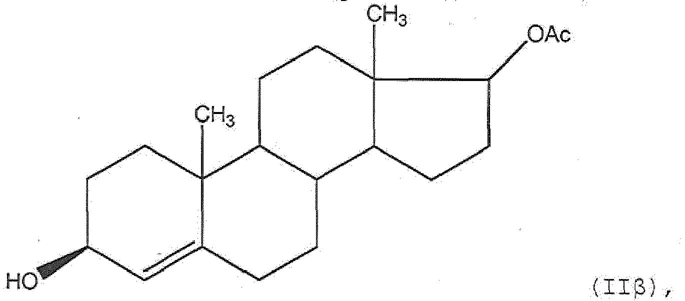 in der Ac für -CO-CH₃ steht,
(2) Umsetzen der so erhaltenen Verbindung der Formel (IIβ) mit der Verbindung der Formel (III) : N₂CH-(CH₂)ₙ₋₁-COOC₂H₅ und dann Umsetzen mit einer Base in Gegenwart eines Lösungsmittels zum Erhalt der Verbindung der Formel (Iaβ) : 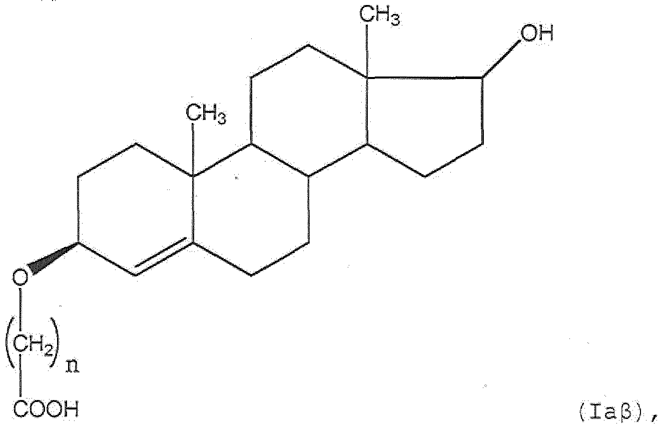
(3) Umsetzen der so erhaltenen Verbindung der Formel (Iaβ) mit N-Hydroxysuccinimid in Gegenwart eines Carbodiimid-Derivats zur Herstellung der Verbindung der Formel (IVβ) : 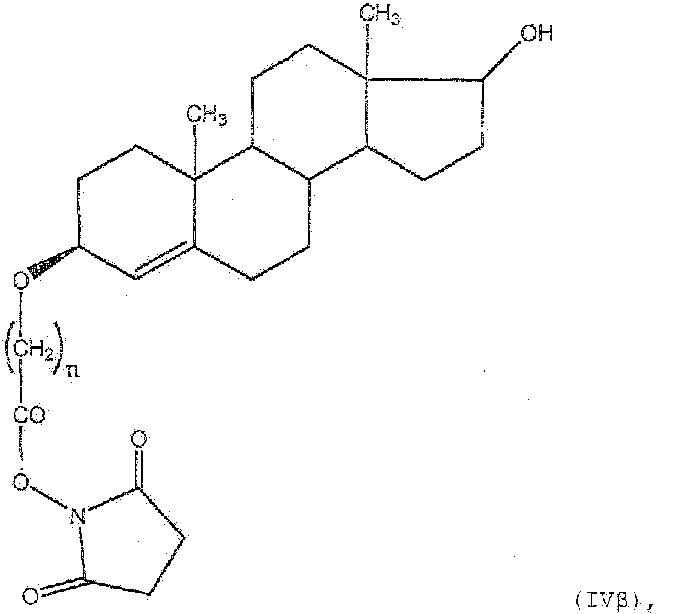
(4) Umsetzen der so erhaltenen Verbindung der Formel (IVβ) mit der Verbindung der Formel (V): H₂N-(CH₂)m-COOR₁, in der R₁ für eine Alkyl- oder Arylgruppe steht, zur Herstellung der Verbindung der Formel (VIβ): 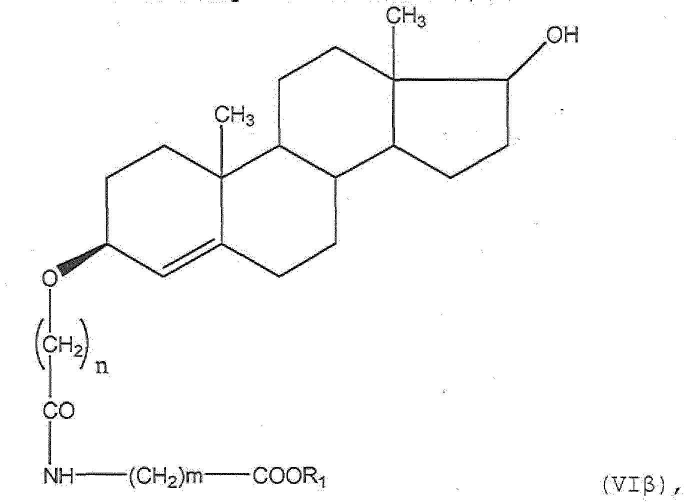
(5) Umsetzen der so erhaltenen Verbindung der Formel (VIβ) mit einer Base in Gegenwart eines Lösungsmittels zum Erhalt der Verbindung der Formel (Ibβ).

16. Verfahren zur Herstellung eines Testosteronderivats in Form eines β-Isomers in 3-Position der folgenden allgemeinen Formel (Iβ): 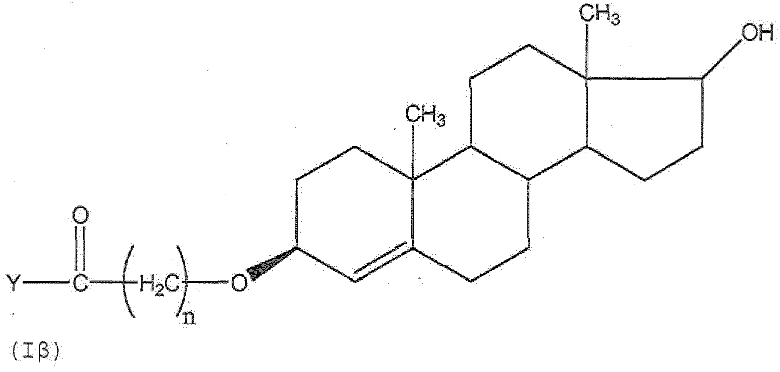 in der n für eine ganze Zahl zwischen 1 und 10 steht und Y für: 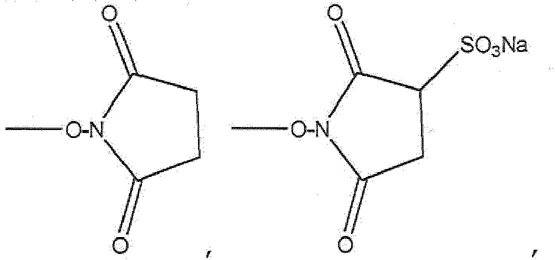 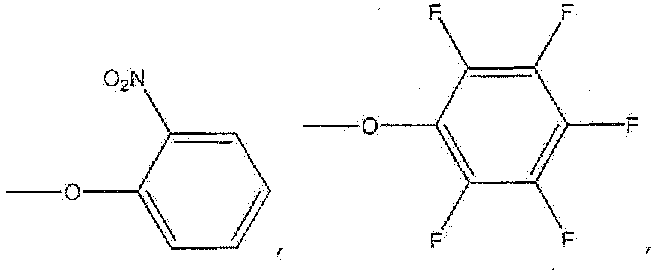 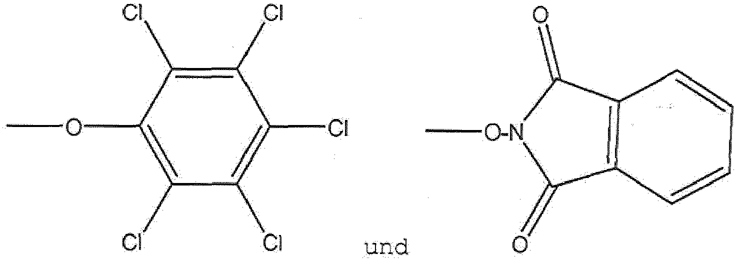 steht, das folgende Schritte umfasst:
(1) Umsetzen von Testosteron mit Essigsäureanhydrid zum Schützen der OH-Funktion in 17-Position und dann mit einem Reduktionsmittel in Gegenwart eines Lösungsmittels zur Reduktion des Carbonyls in 3-Position und zum Erhalt der Verbindung der Formel (IIβ) : 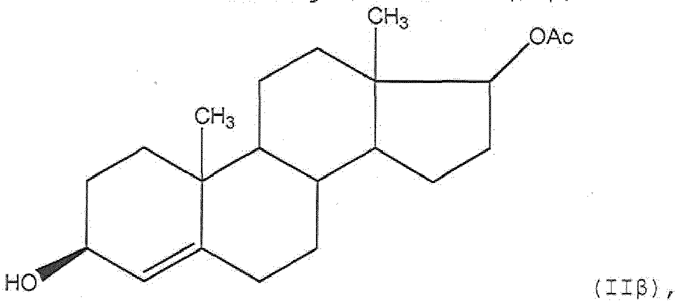 in der Ac für -CO-CH₃ steht,
(2) Umsetzen der so erhaltenen Verbindung der Formel (IIβ) mit der Verbindung der Formel (III) : N₂CH-(CH₂)ₙ₋₁-COOC₂H₅ und dann Umsetzen mit einer Base in Gegenwart eines Lösungsmittels zum Erhalt der Verbindung der Formel (Iaβ) : 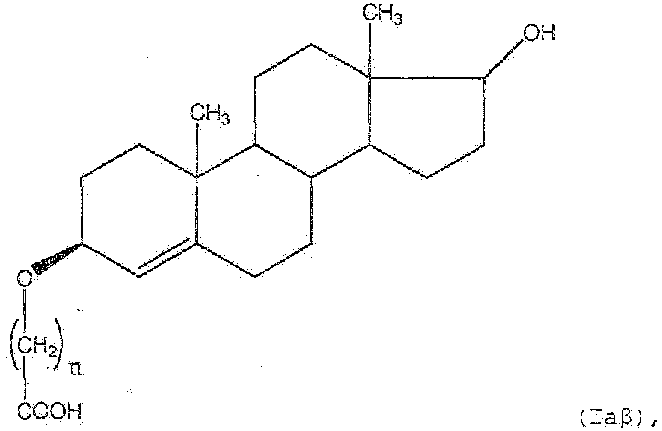
(3) Umsetzen der so erhaltenen Verbindung der Formel (Iaβ) in Gegenwart eines Carbodiimid-Derivats mit einem Reagenz, das gemäß der letztendlichen Gruppe Y aus den folgenden Verbindungen ausgewählt wird, zum Erhalt der Verbindungen der Formel (Iβ) :

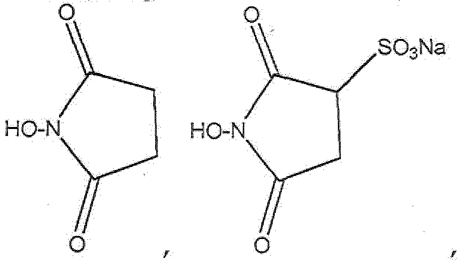

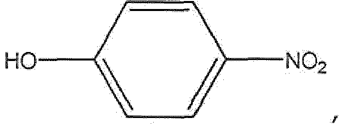

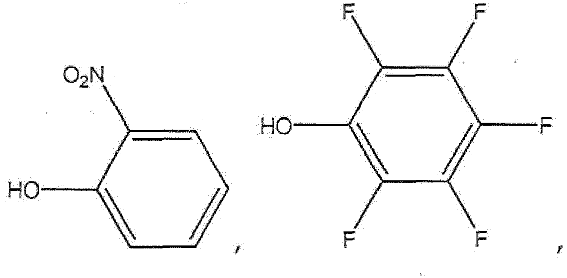

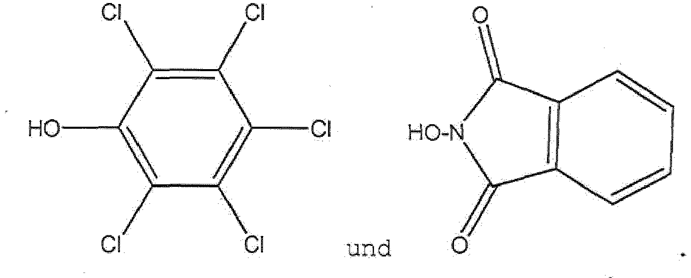

17. Verfahren zur Herstellung eines Testosteronderivats in Form eines β-Isomers in 3-Position der folgenden allgemeinen Formel (Iβ) : 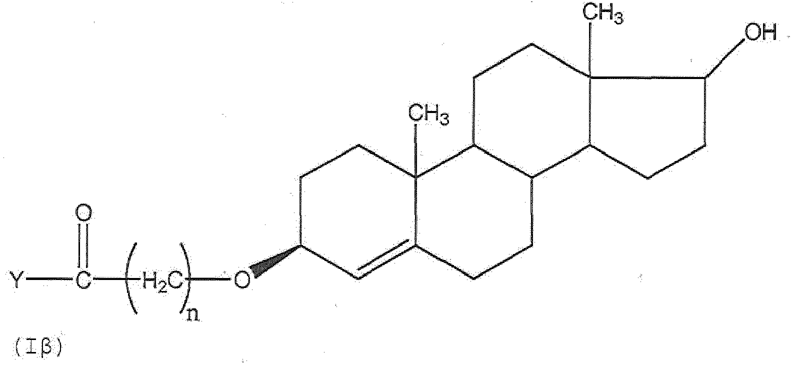 in der n für eine ganze Zahl zwischen 1 und 10 steht und Y für: 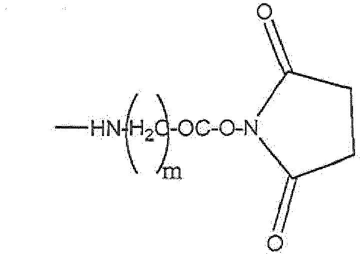 und 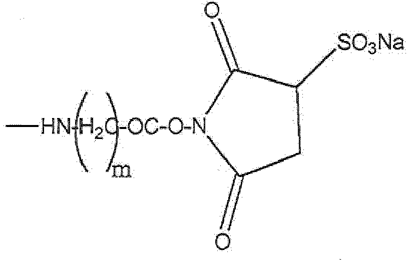 steht, wobei m für eine ganze Zahl zwischen 1 und 10 steht, das folgende Schritte umfasst:
(1) Umsetzen von Testosteron mit Essigsäureanhydrid zum Schützen der OH-Funktion in 17-Position und dann mit einem Reduktionsmittel in Gegenwart eines Lösungsmittels zur Reduktion des Carbonyls in 3-Position und zum Erhalt der Verbindung der Formel (IIβ) : 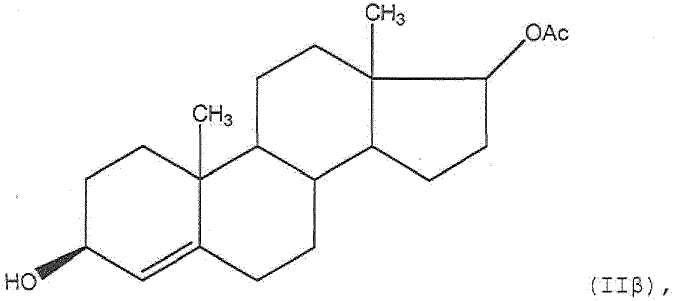 in der Ac für -CO-CH₃ steht,
(2) Umsetzen der so erhaltenen Verbindung der Formel (IIβ) mit der Verbindung der Formel (III) : N₂CH-(CH₂)ₙ₋₁COOC₂H₅ und dann Umsetzen mit einer Base in Gegenwart eines Lösungsmittels zum Erhalt der Verbindung der Formel (Iaβ) : 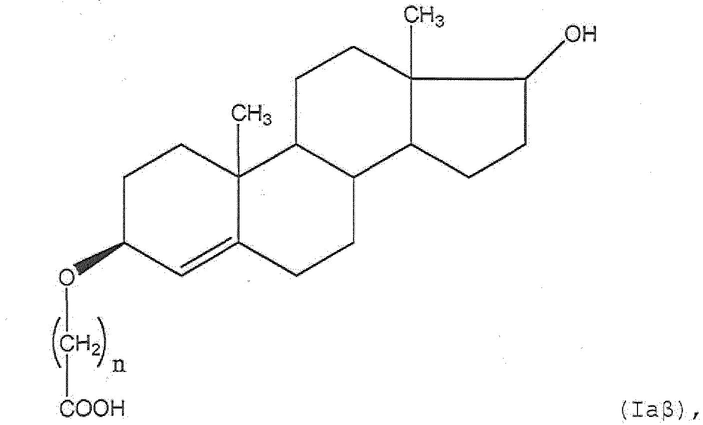
(3) Umsetzen der so erhaltenen Verbindung der Formel (Iaβ) mit N-Hydroxysuccinimid in Gegenwart eines Carbodiimid-Derivats zur Herstellung der Verbindung der Formel (IVβ) : 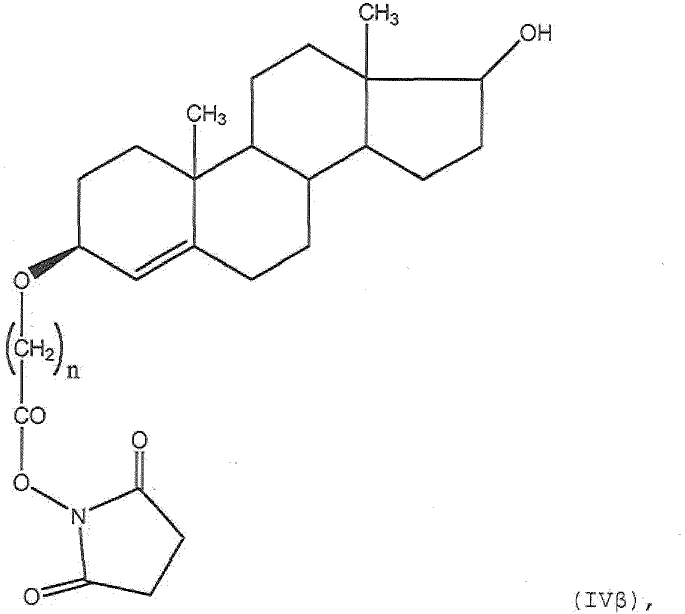
(4) Umsetzen der so erhaltenen Verbindung der Formel (IVβ) mit der Verbindung der Formel (V): H₂N-(CH₂)m-COOR₁, in der R₁ für eine Alkyl- oder Arylgruppe steht, zur Herstellung der Verbindung der Formel (VIβ): 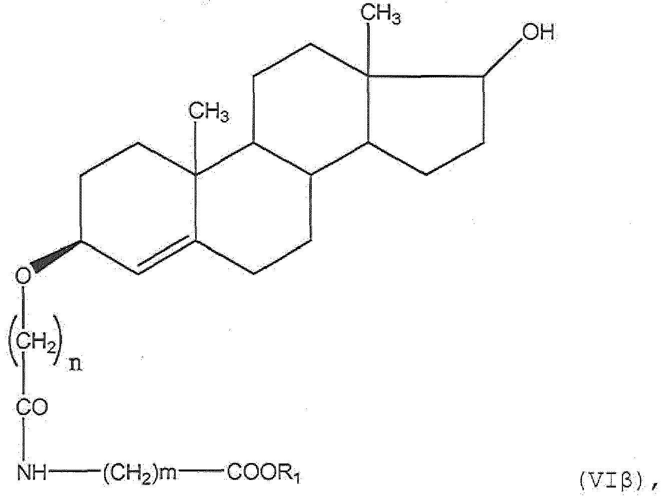
(5) Umsetzen der so erhaltenen Verbindung der Formel (VIβ) mit einer Base in Gegenwart eines Lösungsmittels zum Erhalt der Verbindung der Formel (Ibβ): 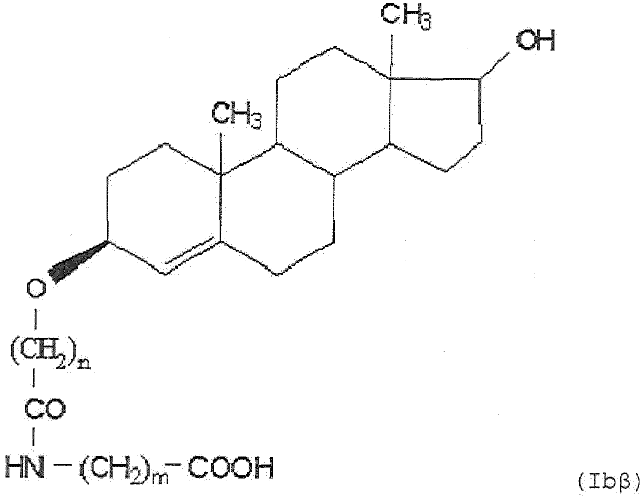
(6) Umsetzen der so erhaltenen Verbindung der Formel (Ibβ) in Gegenwart eines Carbodiimid-Derivats mit einem Reagenz, das gemäß der letztendlichen Gruppe Y aus den folgenden Verbindungen ausgewählt wird, zum Erhalt der Verbindungen der Formel (Iβ):

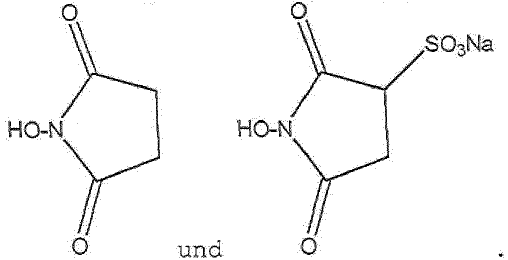

18. Verfahren zur Herstellung eines Testosteronderivats in Form eines β-Isomers in 3-Position der folgenden allgemeinen Formel (Icβ): 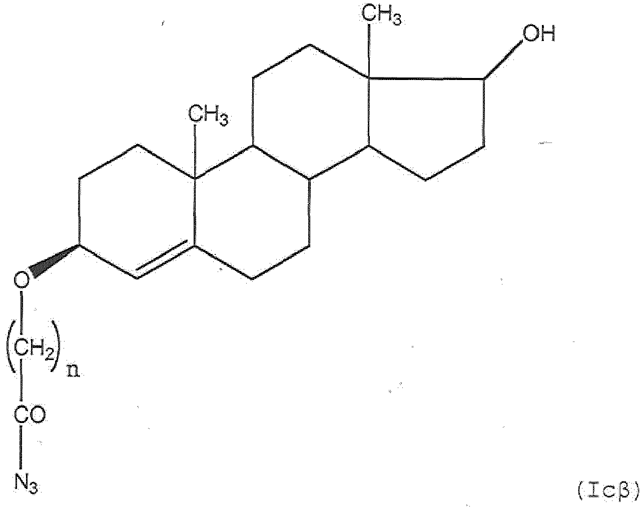 in der n für eine ganze Zahl zwischen 1 und 10 steht, das folgende Schritte umfasst:
(1) Umsetzen von Testosteron mit Essigsäureanhydrid zum Schützen der OH-Funktion in 17-Position und dann mit einem Reduktionsmittel in Gegenwart eines Lösungsmittels zur Reduktion des Carbonyls in 3-Position und zum Erhalt der Verbindung der Formel (IIβ): 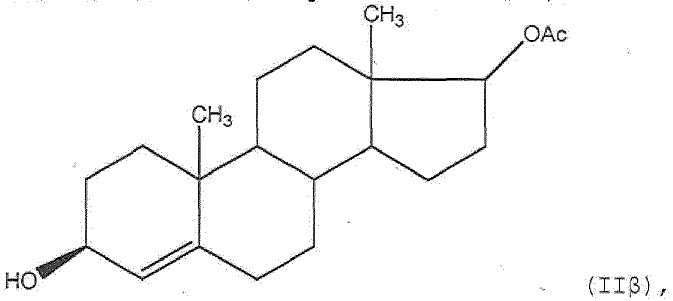 in der Ac für -CO-CH₃ steht,
(2) Umsetzen der so erhaltenen Verbindung der Formel (IIβ) mit der Verbindung der Formel (III) : N₂-CH-(CH)ₙ₋₁COOC₂H₅ und dann Umsetzen mit einer Base in Gegenwart eines Lösungsmittels zum Erhalt der Verbindung der Formel (Iaβ): 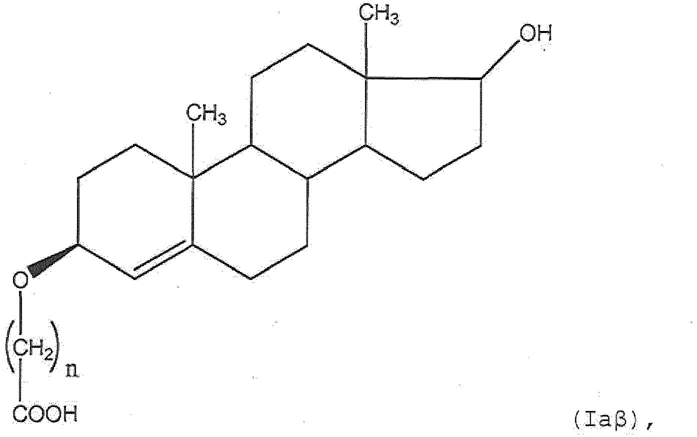
(3) Umsetzen der so erhaltenen Verbindung der Formel (Iaβ) in Gegenwart eines Carbodiimid-Derivats mit H₂NNH₂ zum Erhalt der Verbindung der Formel (VIIβ): 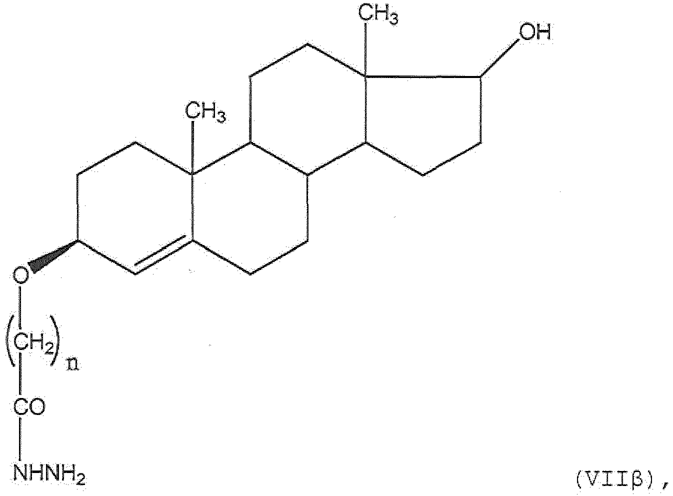
(4) Umsetzen der so erhaltenen Verbindung der Formel (VIIβ) mit HOMO zum Erhalt der Verbindung der Formel (Icβ).

19. Verfahren zur Herstellung eines Testosteronderivats in Form eines α-Isomers in 3-Position der folgenden allgemeinen Formel (Iaα): 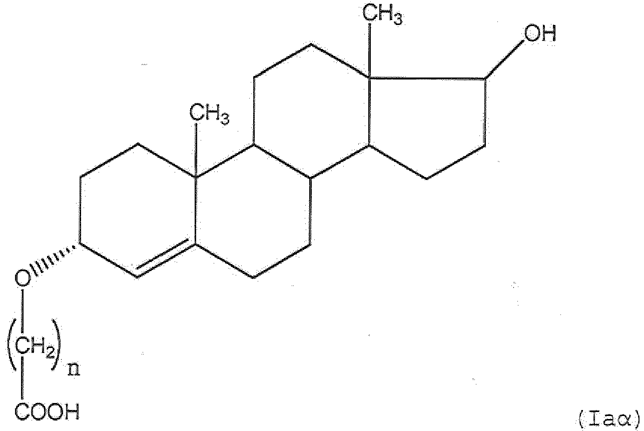 in der n für eine ganze Zahl zwischen 1 und 10 steht, das folgende Schritte umfasst:
(1) Umsetzen von Testosteron mit t-Butyldimethylchlorsilan zum Schützen der OH-Funktion in 17-Position und dann mit einem Reduktionsmittel in Gegenwart eines Lösungsmittels zur Reduktion des Carbonyls in 3-Position und zum Erhalt der Verbindung der Formel (VIIIβ): 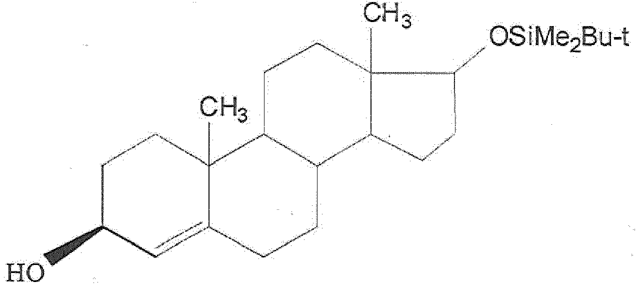 (VIIIβ), in der -SiMe₂Bu-t für t-Butyldimethylsilanyl steht,
(2) Umsetzen der so erhaltenen Verbindung der Formel (VIIIβ) mit Triphenylphosphin, Benzoesäure und Diethylazodicarboxylat und dann mit einer Base in Gegenwart eines Lösungsmittels zur Umwandlung des β-Isomers in das α-Isomer in 3-Position und somit zum Erhalt der Verbindung der Formel (VIIIα): 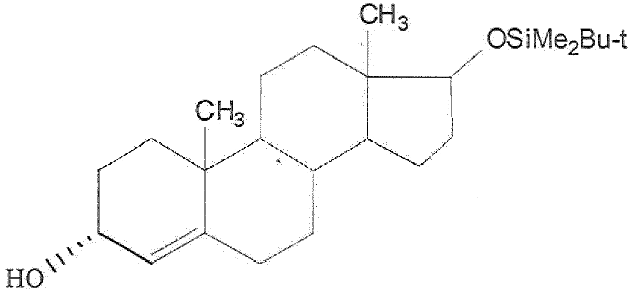 (VIIIα), in der -SiMe₂Bu-t für t-Butyldimethylsilanyl steht,
(3) Umsetzen der so erhaltenen Verbindung der Formel (VIIIα) mit der Verbindung der Formel (III): N₂CH-(CH)ₙ₋₁COOC₂H₅ und dann Umsetzen mit einer Base in Gegenwart eines Lösungsmittels zum Erhalt der Verbindung der Formel (Iaα).

20. Verfahren zur Herstellung eines Testosteronderivats in Form eines α-Isomers in 3-Position der folgenden allgemeinen Formel (Ibα): 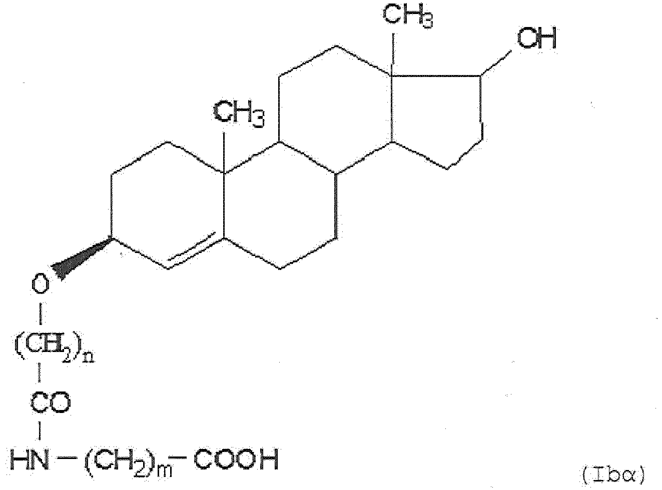 in der n für eine ganze Zahl zwischen 1 und 10 steht, m für eine ganze Zahl zwischen 1 und 10 steht, das folgende Schritte umfasst:
(1) Umsetzen von Testosteron mit t-Butyldimethylchlorsilan zum Schützen der OH-Funktion in 17-Position und dann mit einem Reduktionsmittel in Gegenwart eines Lösungsmittels zur Reduktion des Carbonyls in 3-Position und zum Erhalt der Verbindung der Formel (VIIIβ): 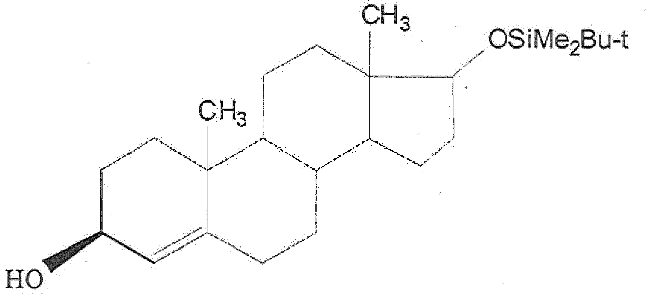 (VIIIβ), in der -SiMe₂Bu-t für t-Butyldimethylsilanyl steht,
(2) Umsetzen der so erhaltenen Verbindung der Formel (VIIIβ) mit Triphenylphosphin, Benzoesäure und Diethylazodicarboxylat und dann mit einer Base in Gegenwart eines Lösungsmittels zur Umwandlung des β-Isomers in das α-Isomer in 3-Position und somit zum Erhalt der Verbindung der Formel (VIIIα): 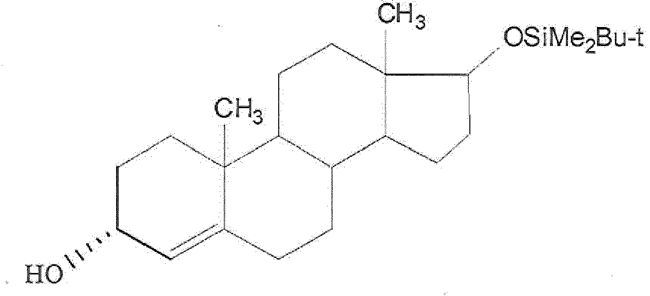 (VIIIα), in der -SiMe₂Bu-t für t-Butyldimethylsilanyl steht,
(3) Umsetzen der so erhaltenen Verbindung der Formel (VIIIα) mit der Verbindung der Formel (III): N₂CH-(CH₂)ₙ₋₁-COOC₂H₅ und dann Umsetzen mit einer Base in Gegenwart eines Lösungsmittels zum Erhalt der Verbindung der Formel (Iaα) : 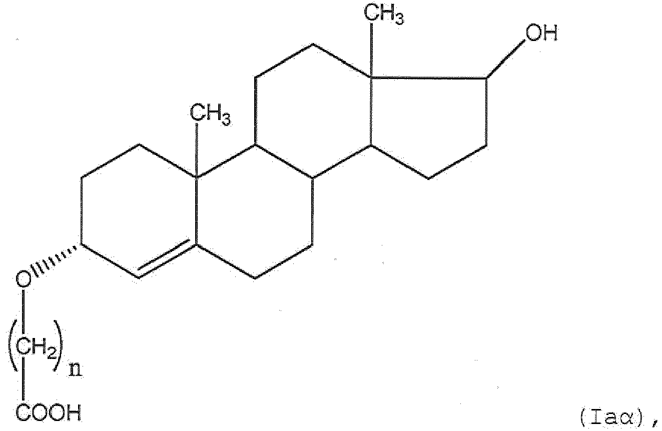
(4) Umsetzen der so erhaltenen Verbindung der Formel (Iaα) mit N-Hydroxysuccinimid in Gegenwart eines Carbodiimid-Derivats zur Herstellung der Verbindung der Formel (IVα): 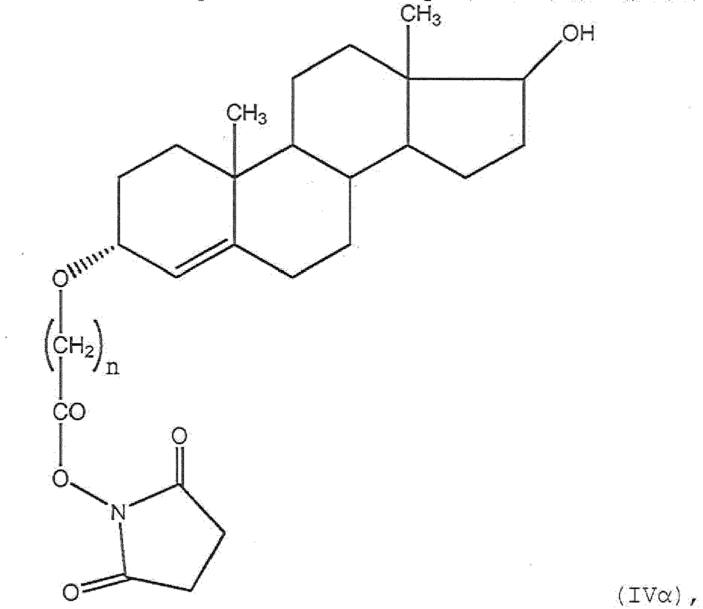
(5) Umsetzen der so erhaltenen Verbindung der Formel (IVα) mit der Verbindung der Formel (V): H₂N-(CH₂)m-COOR₁, in der R₁ für eine Alkyl- oder Arylgruppe steht, zur Herstellung der Verbindung der Formel (VIα): 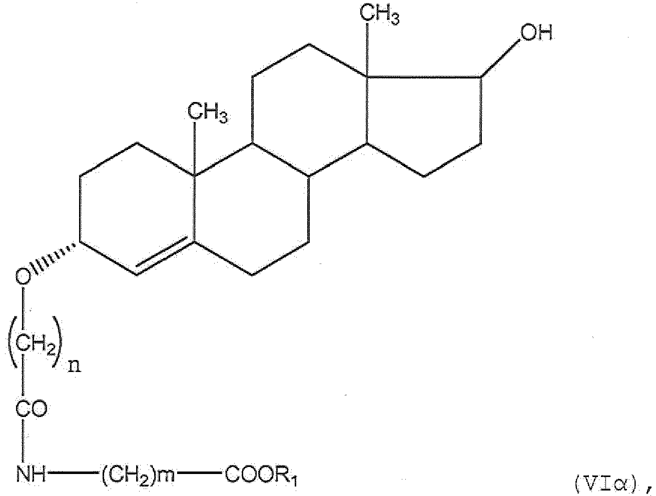
(6) Umsetzen der so erhaltenen Verbindung der Formel (VIα) mit einer Base in Gegenwart eines Lösungsmittels zum Erhalt der Verbindung der Formel (Ibα).

21. Verfahren zur Herstellung eines Testosteronderivats in Form eines α-Isomers in 3-Position der folgenden allgemeinen Formel (Iα): 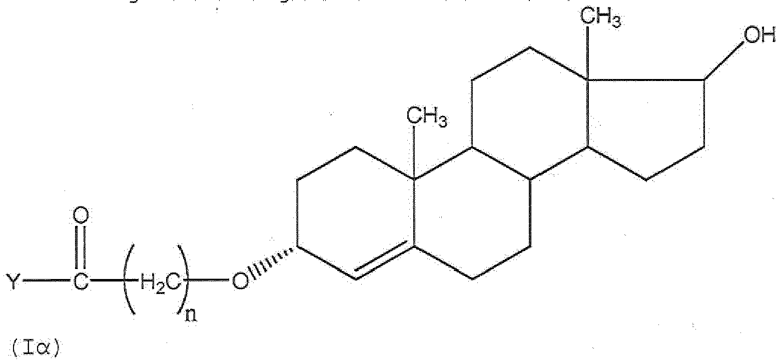 in der n für eine ganze Zahl zwischen 1 und 10 steht und Y für: 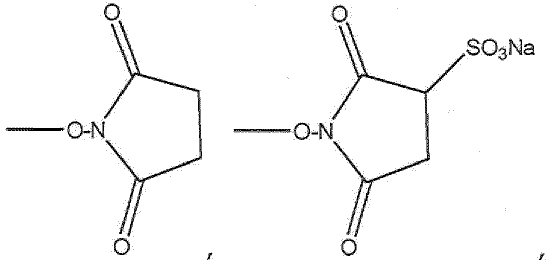 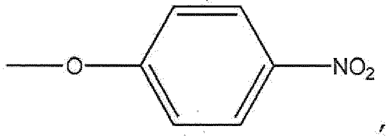 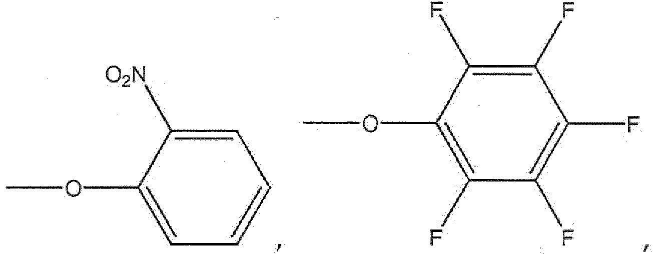 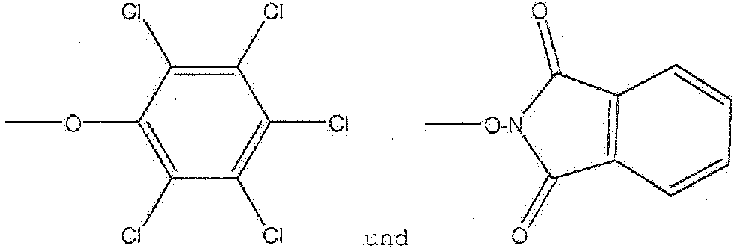 steht, das folgende Schritte umfasst:
(1) Umsetzen von Testosteron mit t-Butyldimethylchlorsilan zum Schützen der OH-Funktion in 17-Position und dann mit einem Reduktionsmittel in Gegenwart eines Lösungsmittels zur Reduktion des Carbonyls in 3-Position und zum Erhalt der Verbindung der Formel (VIIIβ) : 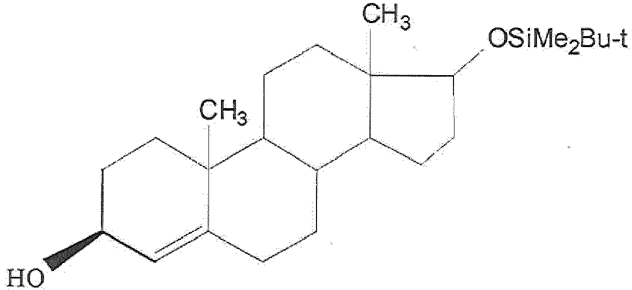 (VIIIβ), in der -SiMe₂Bu-t für t-Butyldimethylsilanyl steht,
(2) Umsetzen der so erhaltenen Verbindung der Formel (VIIIβ) mit Triphenylphosphin, Benzoesäure und Diethylazodicarboxylat und dann mit einer Base in Gegenwart eines Lösungsmittels zur Umwandlung des β-Isomers in das α-Isomer in 3-Position und somit zum Erhalt der Verbindung der Formel (VIIIα): 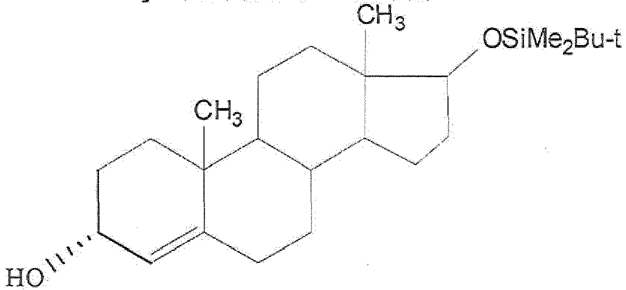 (VIIIα), in der -SiMe₂Bu-t für t-Butyldimethylsilanyl steht,
(3) Umsetzen der so erhaltenen Verbindung der Formel (VIIIα) mit der Verbindung der Formel (III): N₂CH-(CH₂)ₙ₋₁COOC₂H₅ und dann Umsetzen mit einer Base in Gegenwart eines Lösungsmittels zum Erhalt der Verbindung der Formel Iaα) : 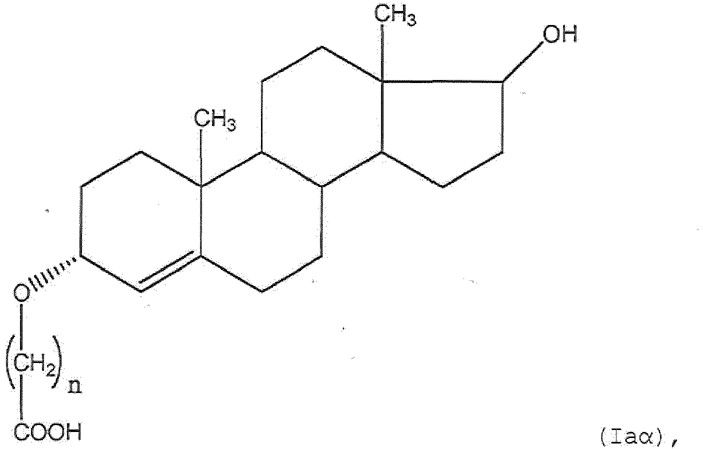
(4) Umsetzen der so erhaltenen Verbindung der Formel (Iaα) in Gegenwart eines Carbodiimid-Derivats mit einem Reagenz, das gemäß der letztendlichen Gruppe Y aus den folgenden Verbindungen ausgewählt wird, zum Erhalt einer Verbindung der Formel (Iα):

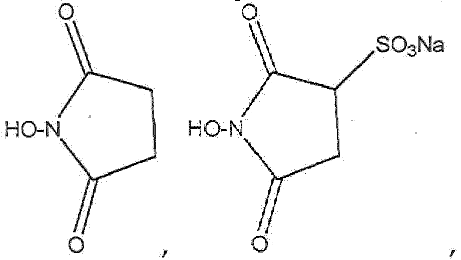

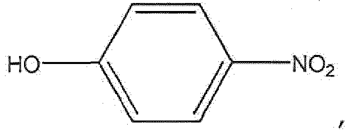

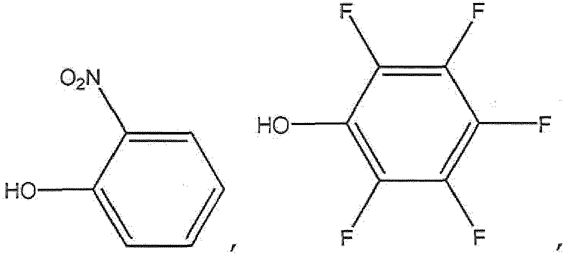

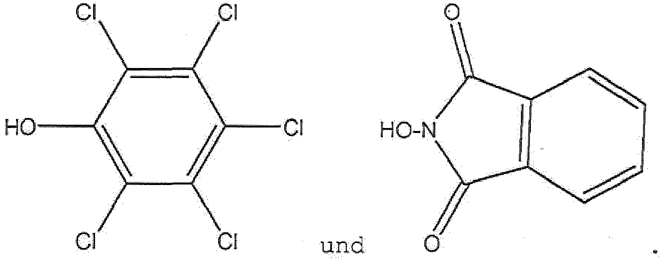

22. Verfahren zur Herstellung eines Testosteronderivats in Form eines α-Isomers in 3-Position der folgenden allgemeinen Formel (Iα) : 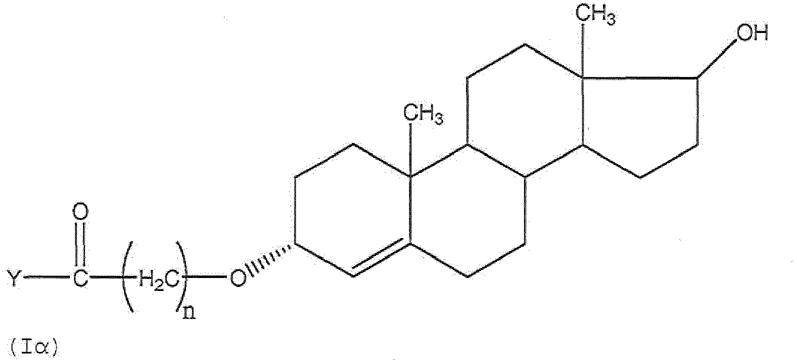 in der n für eine ganze Zahl zwischen 1 und 10 steht und Y für: 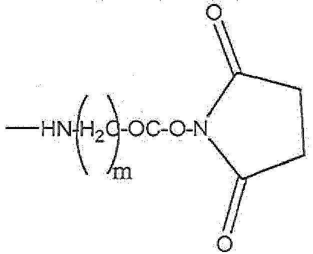 und 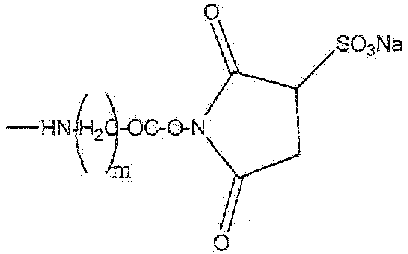 steht, wobei m für eine ganze Zahl zwischen 1 und 10 steht, das folgende Schritte umfasst:
(1) Umsetzen von Testosteron mit t-Butyldimethylchlorsilan zum Schützen der OH-Funktion in 17-Position und dann mit einem Reduktionsmittel in Gegenwart eines Lösungsmittels zur Reduktion des Carbonyls in 3-Position und zum Erhalt der Verbindung der Formel (VIIIβ) : 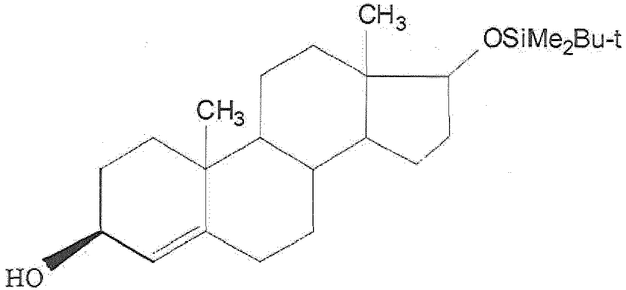 (VIIIβ), in der -SiMe₂Bu-t für t-Butyldimethylsilanyl steht,
(2) Umsetzen der so erhaltenen Verbindung der Formel (VIIIβ) mit Triphenylphosphin, Benzoesäure und Diethylazodicarboxylat und dann mit einer Base in Gegenwart eines Lösungsmittels zur Umwandlung des β-Isomers in das α-Isomer in 3-Position und somit zum Erhalt der Verbindung der Formel (VIIIα): 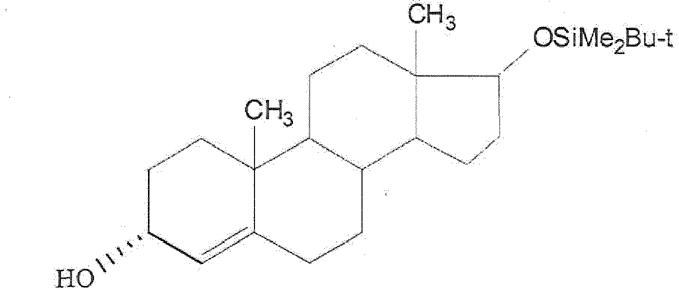 (VIIIα), in der -SiMe₂Bu-t für t-Butyldimethylsilanyl steht,
(3) Umsetzen der so erhaltenen Verbindung der Formel (VIIIα) mit der Verbindung der Formel (III): N₂CH-(CH₂)ₙ₋₁-COOC₂H₅ und dann Umsetzen mit einer Base in Gegenwart eines Lösungsmittels zum Erhalt der Verbindung der Formel (Iaα) : 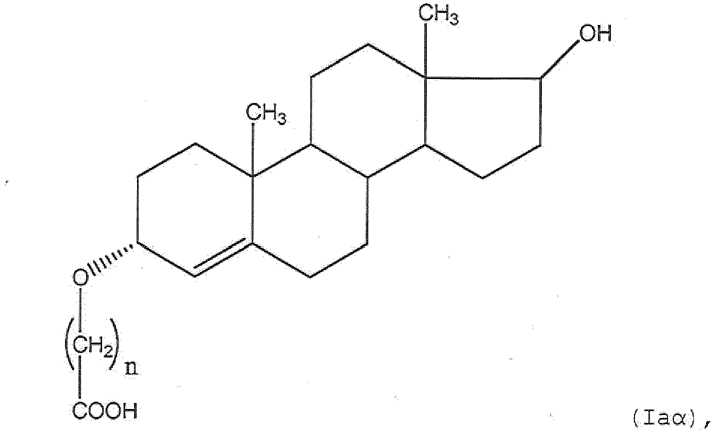
(4) Umsetzen der so erhaltenen Verbindung der Formel (Iaα) mit N-Hydroxysuccinimid in Gegenwart eines Carbodiimid-Derivats zur Herstellung der Verbindung der Formel (IVα): 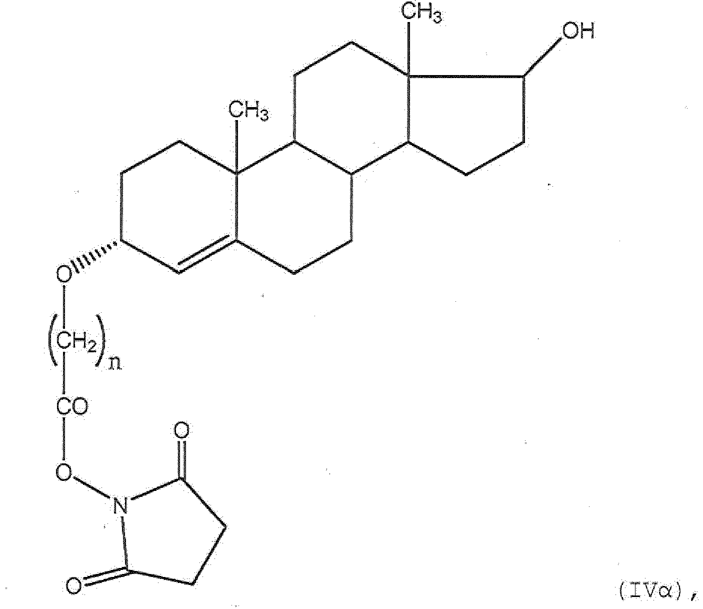
(5) Umsetzen der so erhaltenen Verbindung der Formel (IVα) mit der Verbindung der Formel (V): H₂N-(CH₂)m-COOR₁, in der R₁ für eine Alkyl- oder Arylgruppe steht, zur Herstellung der Verbindung der Formel (VIα): 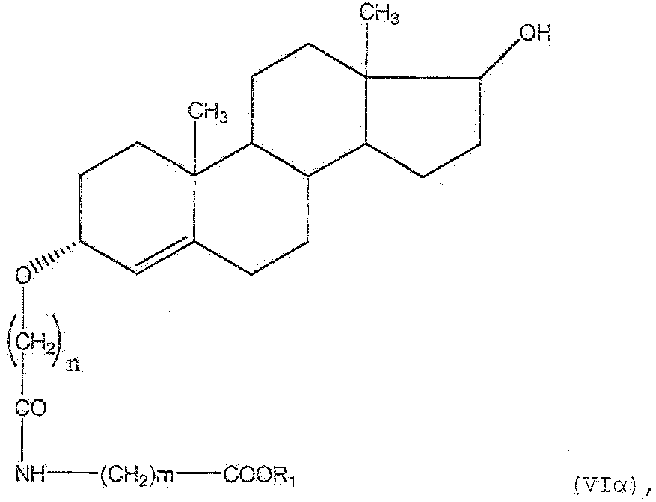
(6) Umsetzen der so erhaltenen Verbindung der Formel (VIα) mit einer Base in Gegenwart eines Lösungsmittels zum Erhalt der Verbindung der Formel (Ibα): 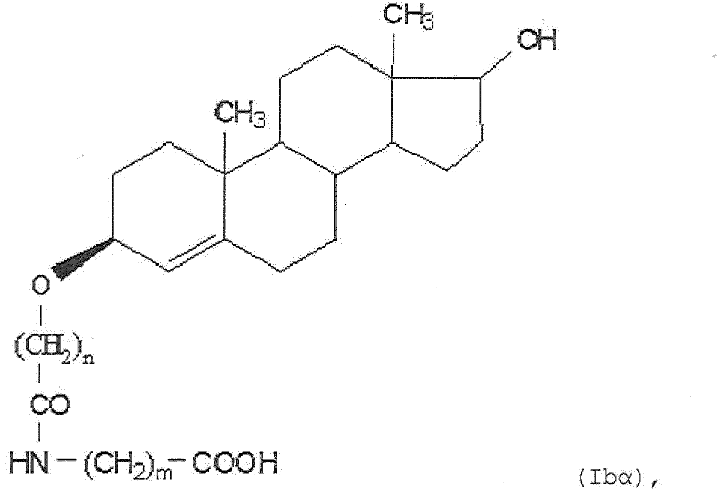
(7) Umsetzen der so erhaltenen Verbindung der Formel (Ibα) in Gegenwart eines Carbodiimid-Derivats mit einem Reagenz, das gemäß der letztendlichen Gruppe Y aus den folgenden Verbindungen ausgewählt wird, zum Erhalt einer Verbindung der Formel (Iα):

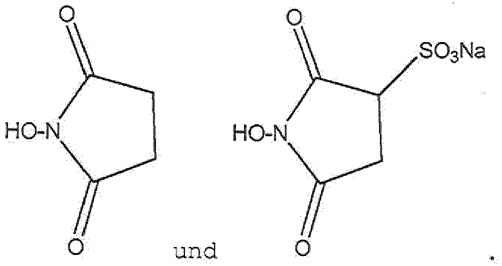

23. Verfahren zur Herstellung eines Testosteronderivats in Form eines α-Isomers in 3-Position der folgenden allgemeinen Formel (Icα): 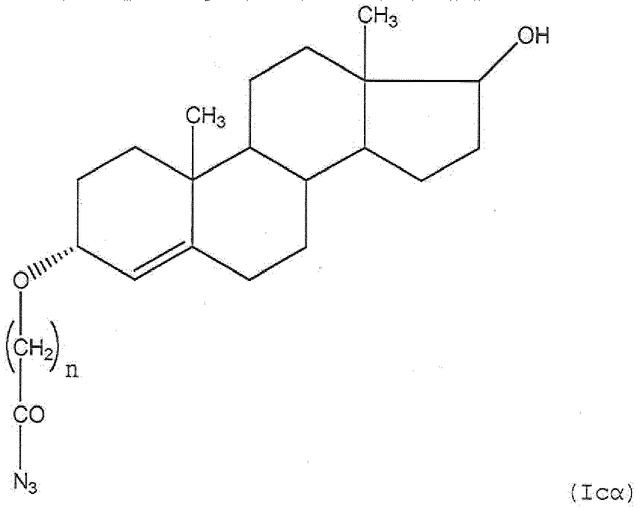 in der n für eine ganze Zahl zwischen 1 und 10 steht, das folgende Schritte umfasst:
(1) Umsetzen von Testosteron mit t-Butyldimethylchlorsilan zum Schützen der OH-Funktion in 17-Position und dann mit einem Reduktionsmittel in Gegenwart eines Lösungsmittels zur Reduktion des Carbonyls in 3-Position und zum Erhalt der Verbindung der Formel (VIIIβ) : 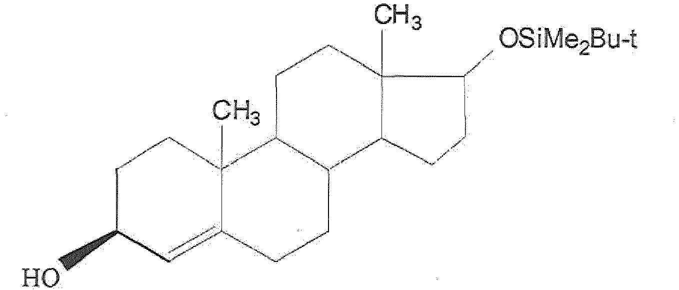 (VIIIβ), in der -SiMe₂Bu-t für t-Butyldimethylsilanyl steht,
(2) Umsetzen der so erhaltenen Verbindung der Formel (VIIIβ) mit Triphenylphosphin, Benzoesäure und Diethylazodicarboxylat und dann mit einer Base in Gegenwart eines Lösungsmittels zur Umwandlung des β-Isomers in das α-Isomer in 3-Position und somit zum Erhalt der Verbindung der Formel (VIIIα): 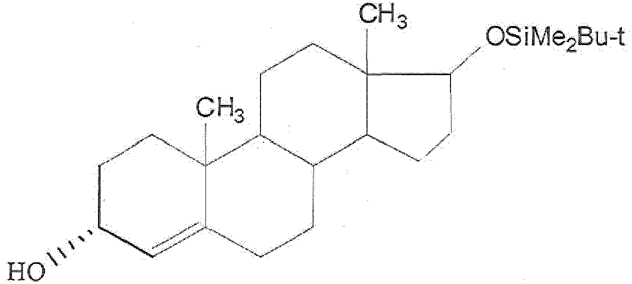 (VIIIα), in der -SiMe₂Bu-t für t-Butyldimethylsilanyl steht,
(3) Umsetzen der so erhaltenen Verbindung der Formel (VIIIα) mit der Verbindung der Formel (III) : N₂CH-(CH₂)ₙ₋₁-COOC₂H₅ und dann Umsetzen mit einer Base in Gegenwart eines Lösungsmittels zum Erhalt der Verbindung der Formel (Iaα) : 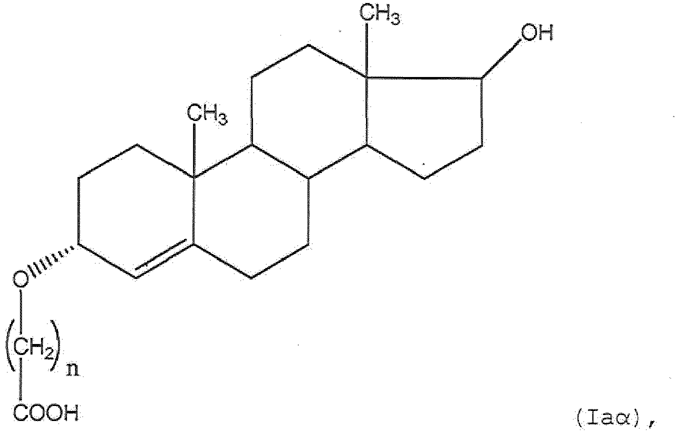
(4) Umsetzen der so erhaltenen Verbindung der Formel (Iaα) in Gegenwart eines carbodiimid-Derivats mit H₂NNH₂ zum Erhalt der Verbindung der Formel (VIIα) : 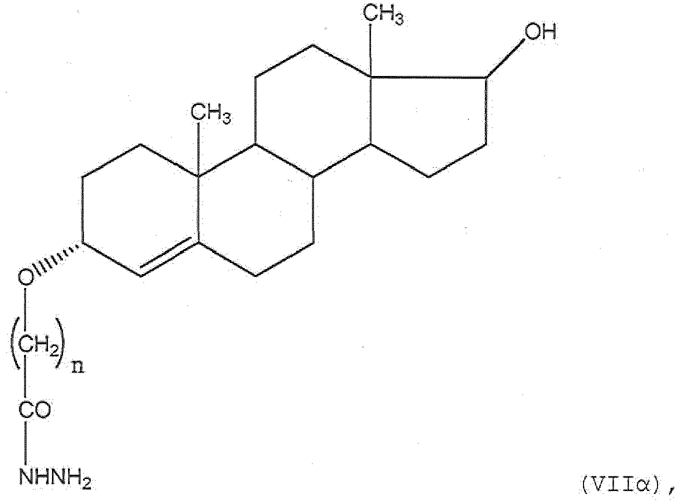
(5) Umsetzen der so erhaltenen Verbindung der Formel (VIIα) mit HONO zum Erhalt der Verbindung der Formel (Icα).

## Claims

1. A testosterone derivative of the following general formula (I): 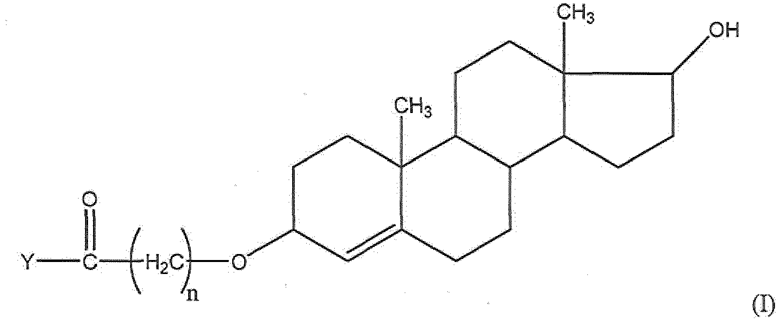 in which n is an integer between 1 and 10 and Y represents an activated or ready-to-be-activated group allowing formation of an amide bond with a primary amine of a molecule.

2. The testosterone derivative as claimed in claim 1, **characterized in that** n is between 1 and 5.

3. The testosterone derivative as claimed in claim 1 or 2, **characterized in that** Y is selected from selected from -OH, -NH-(CH₂)ₘ-COOH, -N₃, 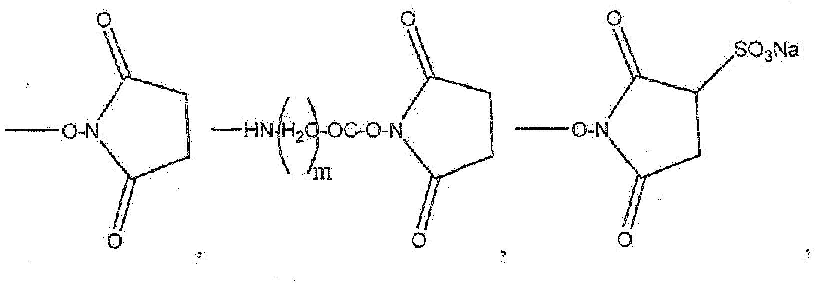 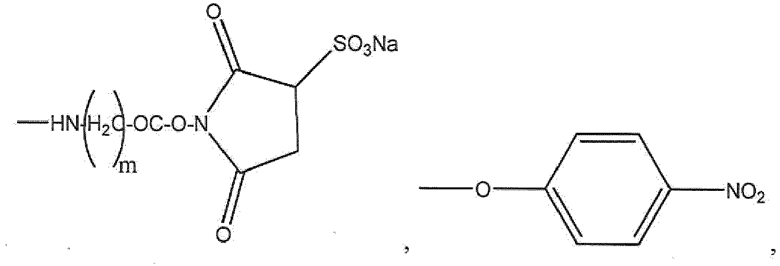 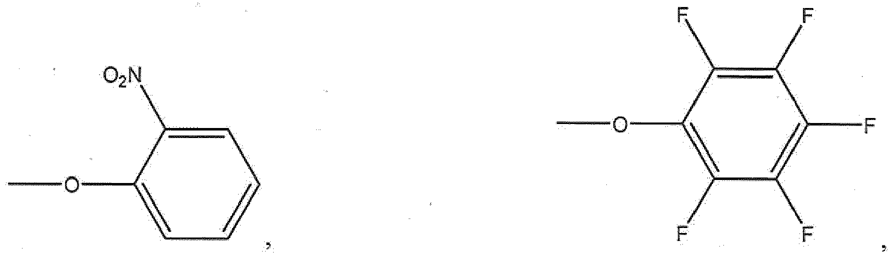 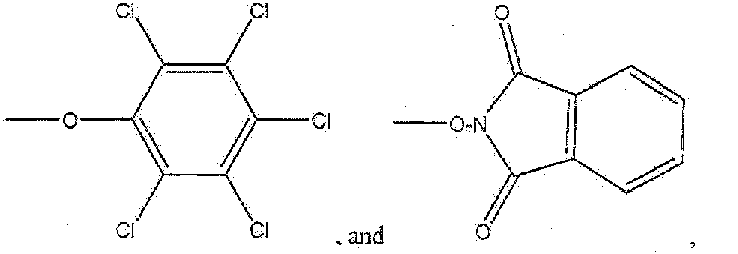 m being an integer between 1 and 10.

4. The testosterone derivative as claimed in claim 3, **characterized in that** m is between 1 and 5.

5. The testosterone derivative as claimed in claim 1 or 2, **characterized in that** Y is selected from -OH and -NH-(CH₂)ₘ-COOH and 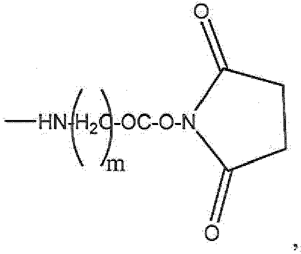 m being between 1 and 10 and preferably m being equal to 1.

6. The testosterone derivative as claimed in any one of claims 1 to 5, **characterized in that** it is in the form of a β derivative at the carbon in position 3.

7. The testosterone derivative as claimed in any one of claims 1 to 5, **characterized in that** it is in the form of an α derivative at the carbon in position 3.

8. A conjugate consisting of a testosterone derivative as described in any one of claims 1 to 7, and a marker.

9. The use of a testosterone derivative as described in any one of claims 1 to 7 or of a conjugate as described in claim 8 for determining the concentration of testosterone in a biological sample.

10. A method for determining the concentration of testosterone by competitive immunoassay in a biological sample, comprising the steps consisting of:
a) bringing into contact, within said sample, (i) a testosterone binding partner and (ii) a compound selected from a testosterone derivative as described in any one of claims 1 to 7 and a conjugate as described in claim 8, one of said components (i) and (ii) being adapted to emit a signal,
b) optionally waiting a sufficient time to allow the competition reaction and
c) measuring the intensity of the signal and deducing therefrom the concentration of testosterone by comparing with a calibration curve establishing a relationship between the measured signal intensity and the testosterone concentration.

11. The method for determining the concentration of testosterone as claimed in claim 10, **characterized in that** the compound (ii) is a conjugate as described in claim 8.

12. A diagnostic kit for implementing the method as claimed in claim 10 or 11, comprising a testosterone derivative as described in any one of claims 1 to 7 or a conjugate as described in claim 8.

13. The use of a compound of formula (Ia) for preparing a testosterone derivative of formula (I) as described in any one of claims 1 to 7, formula (Ia) being: 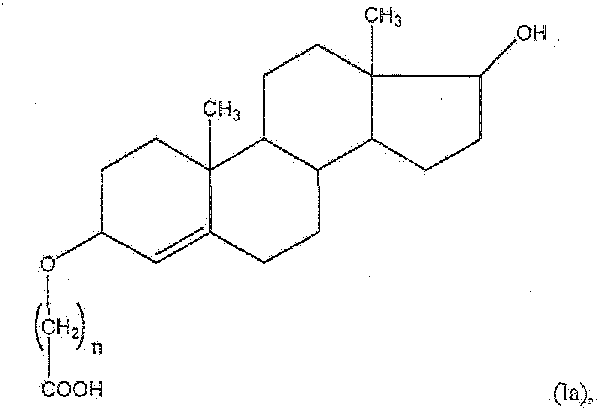 n being an integer between 1 and 10.

14. A method for preparing a testosterone derivative, in the form of β isomer in position 3, of the following general formula (Iaβ): 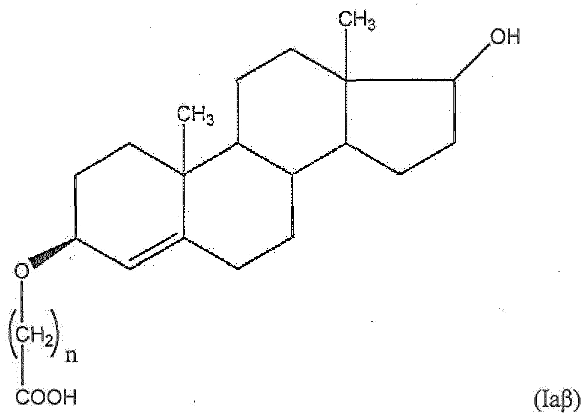 in which n is an integer between 1 and 10, comprising the steps consisting of:
(1) reacting testosterone with acetic anhydride to protect the OH function of position 17, and then with a reducing agent, in the presence of a solvent, to reduce the carbonyl in position 3 and obtain the compound of formula (IIβ): 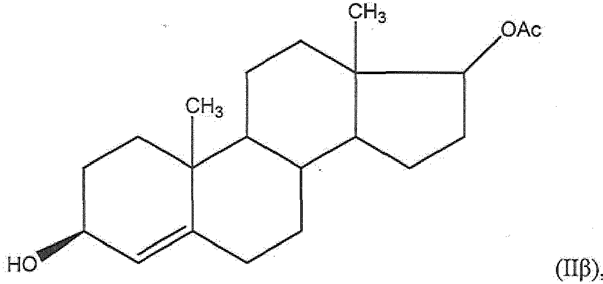 in which Ac means -CO-CH₃,
(2) reacting the compound of formula (IIβ) thus obtained with the compound of formula (III): N₂CH-(CH₂)ₙ₋₁-COOC₂H₅, and then reacting with a base, in the presence of a solvent, to obtain the compound of formula (Iaβ).

15. A method for preparing a testosterone derivative, in the form of β isomer in position 3, of the following general formula (Ibβ): 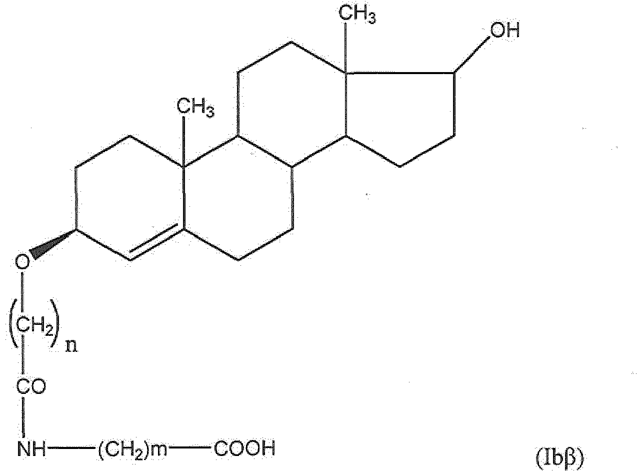 in which n is an integer between 1 and 10, m is an integer between 1 and 10, comprising the steps consisting of:
(1) reacting testosterone with acetic anhydride to protect the OH function of position 17, and then with a reducing agent, in the presence of a solvent, to reduce the carbonyl in position 3 and obtain the compound of formula (IIβ): 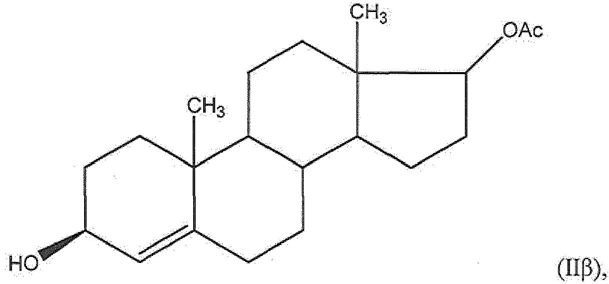 in which Ac means -CO-CH₃,
(2) reacting the compound of formula (IIβ) thus obtained with the compound of formula (III): N₂CH-(CH₂)ₙ₋₁-COOC₂H₅, and then reacting with a base, in the presence of a solvent, to obtain the compound of formula (Iaβ): 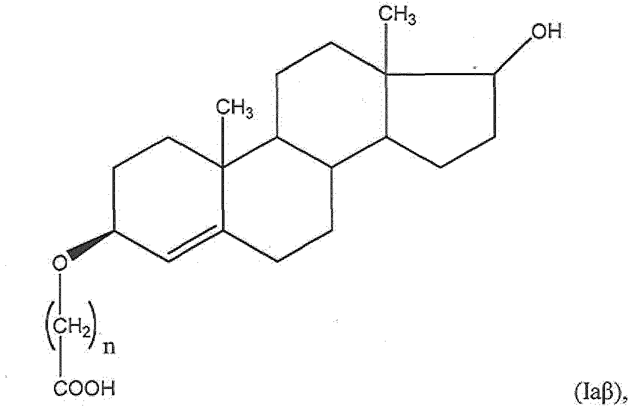
(3) reacting the compound of formula (Iaβ) thus obtained with N-hydroxysuccinimide, in the presence of a carbodiimide derivative, to produce the compound of formula (IVβ): 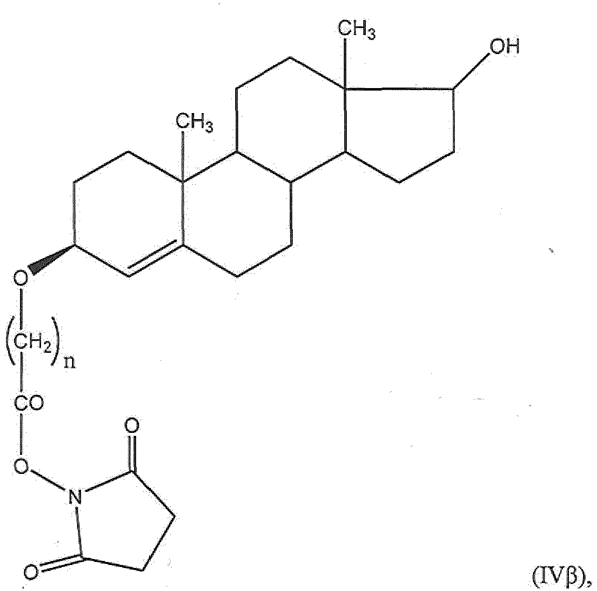
(4) reacting the compound of formula (IVβ) thus obtained with the compound of formula (V): H₂N-(CH₂)m-COOR₁, in which R₁ is an alkyl or aryl group, to produce the compound of formula (VIβ): 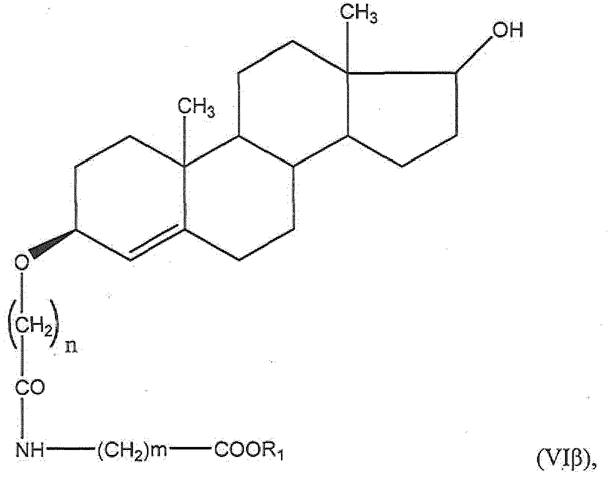
(5) reacting the compound of formula (VIβ) thus obtained with a base, in the presence of a solvent, to obtain the compound of formula (Ibβ).

16. A method for preparing a testosterone derivative, in the form of β isomer in position 3, of the following general formula (Iβ): 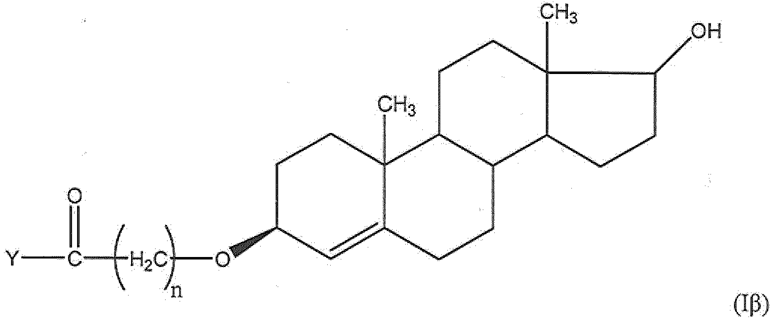 in which n is an integer between 1 and 10 and Y represents: 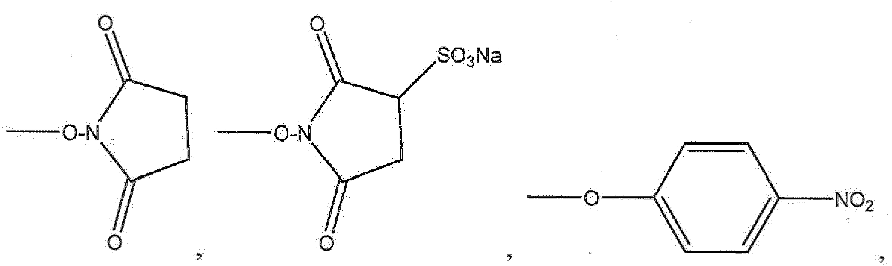 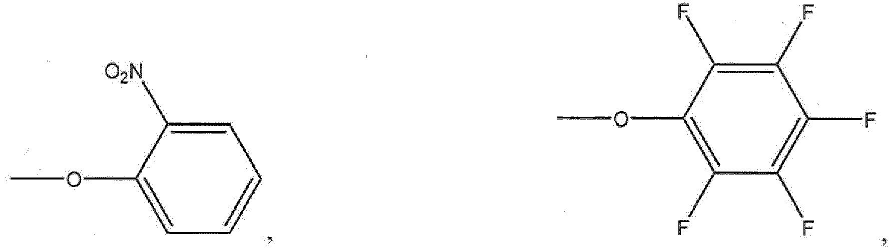 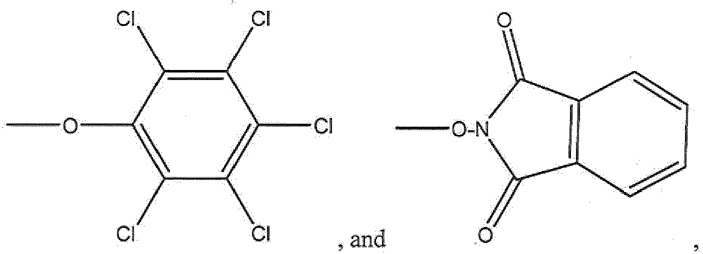 comprising the steps consisting of:
(1) reacting testosterone with acetic anhydride to protect the OH function of position 17, and then with a reducing agent, in the presence of a solvent, to reduce the carbonyl in position 3 and obtain the compound of formula (IIβ): 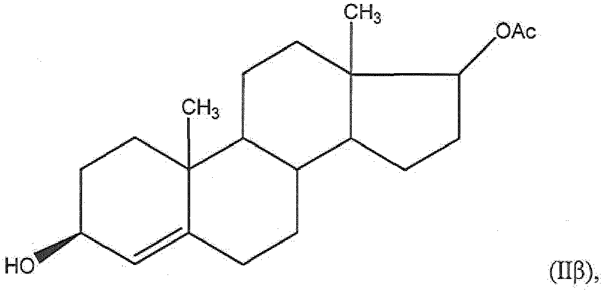 in which Ac means -CO-CH₃
(2) reacting the compound of formula (IIβ) thus obtained with the compound of formula (III): N₂CH-(CH₂)ₙ₋₁-COOC₂H₅, and then reacting with a base, in the presence of a solvent, to obtain the compound of formula (Iaβ): 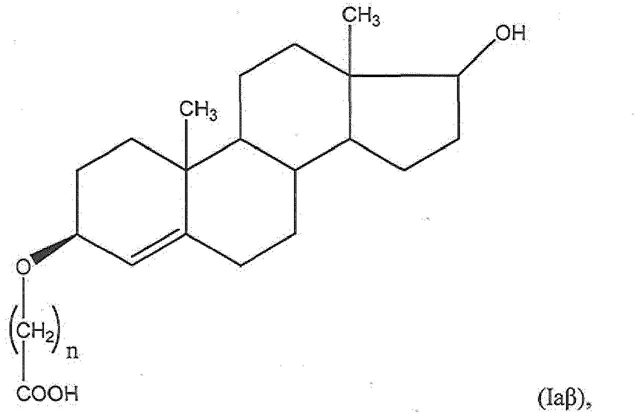
(3) reacting the compound of formula (Iaβ) thus obtained, in the presence of a carbodiimide derivative, with a reagent selected, depending on the final group Y, from the following compounds, to obtain the compounds of formula (Iβ):

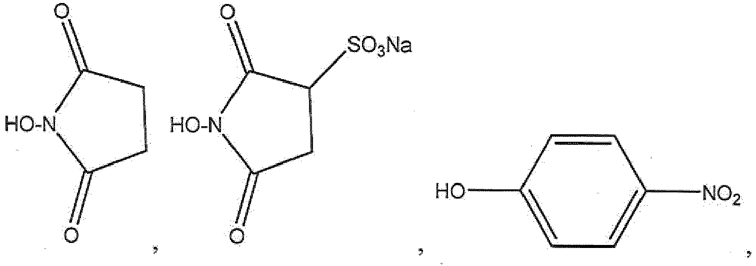

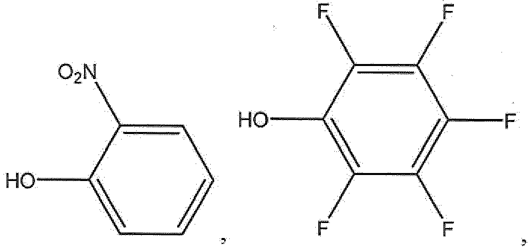

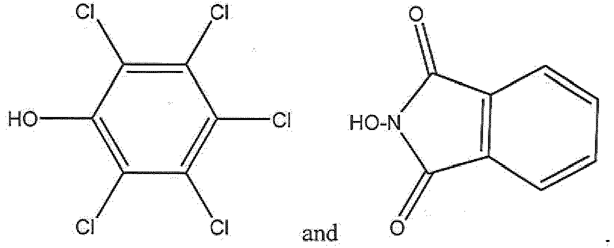

17. A method for preparing a testosterone derivative, in the form of β isomer in position 3, of the following general formula (Iβ): 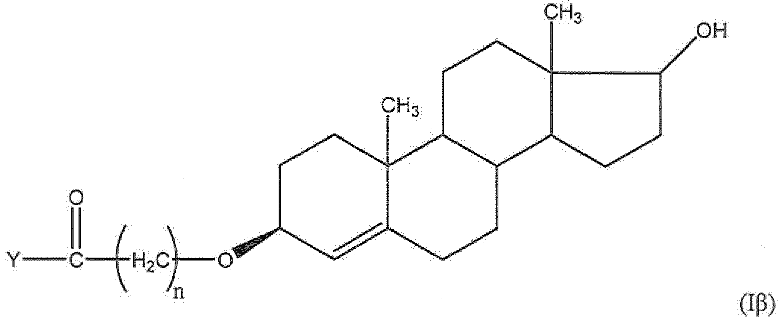 in which n is an integer between 1 and 10 and Y represents: 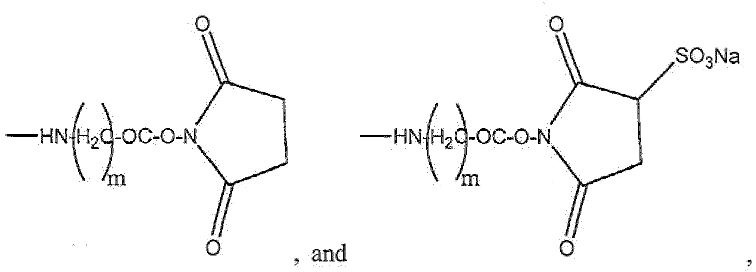 m being an integer between 1 and 10, comprising the steps consisting of:
(1) reacting testosterone with acetic anhydride to protect the OH function of position 17, and then with a reducing agent, in the presence of a solvent, to reduce the carbonyl in position 3 and obtain the compound of formula (IIβ): 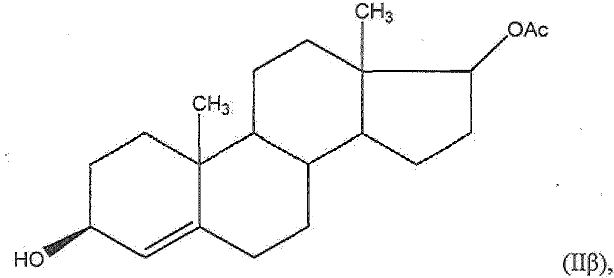 in which Ac means -CO-CH₃,
(2) reacting the compound of formula (IIβ) thus obtained with the compound of formula (III): N₂CH-(CH₂)ₙ₋₁-COOC₂H₅, and then reacting with a base, in the presence of a solvent, to obtain the compound of formula (Iaβ): 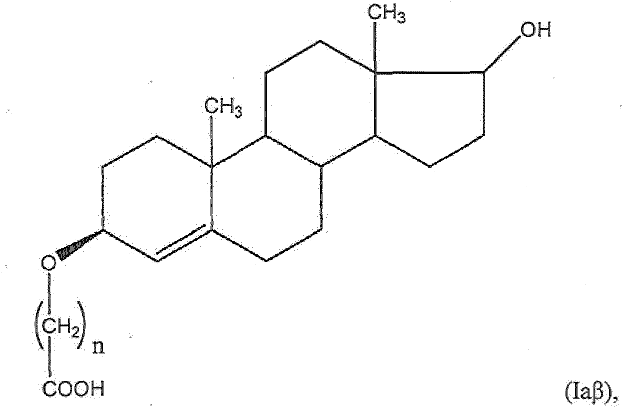
(3) reacting the compound of formula (Iaβ) thus obtained with N-hydroxysuccinimide, in the presence of a carbodiimide derivative, to produce the compound of formula (IVβ): 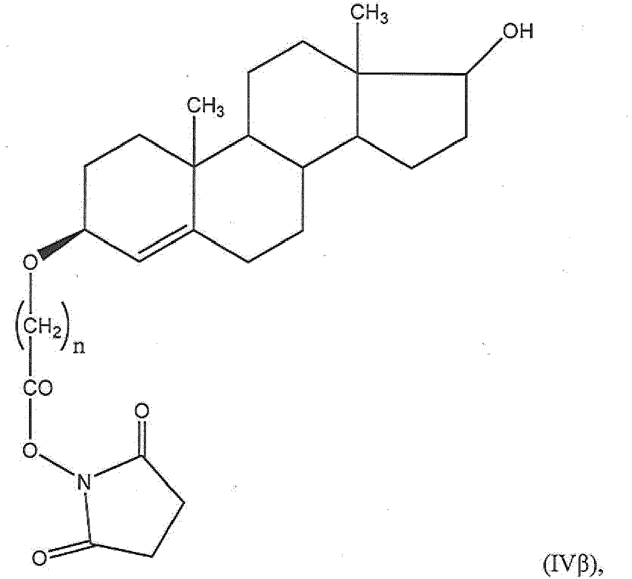
(4) reacting the compound of formula (IVβ) thus obtained with the compound of formula (V): H₂N-(CH₂)m-COOR₁, in which R₁ is an alkyl or aryl group, to produce the compound of formula (VIβ): 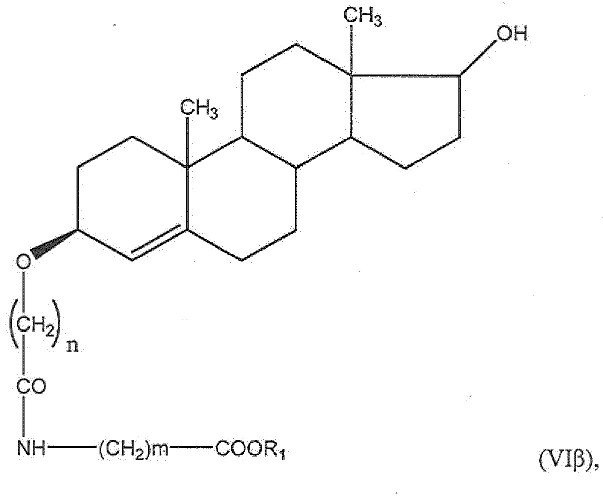
(5) reacting the compound of formula (VIβ) thus obtained with a base, in the presence of a solvent, to obtain the compound of formula (Ibβ): 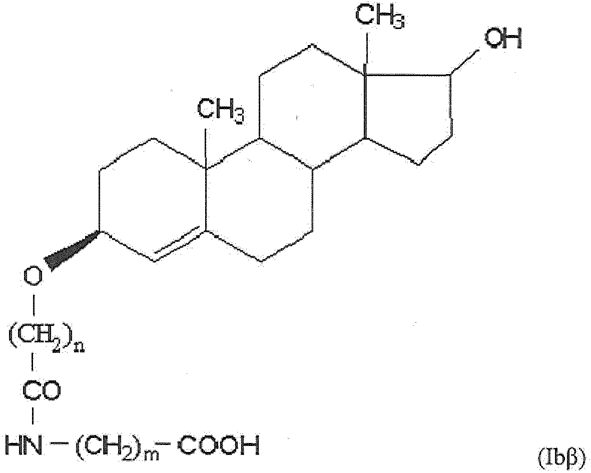
(6) reacting the compound of formula (Ibβ) thus obtained, in the presence of a carbodiimide derivative, with a reagent selected, depending on the final group Y, from the following compounds, to obtain the compounds of formula (Iβ):

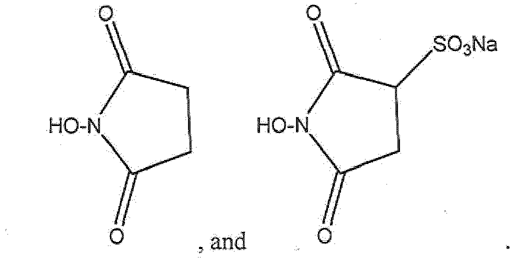

18. A method for preparing a testosterone derivative, in the form of β isomer in position 3, of the following general formula (Icβ): 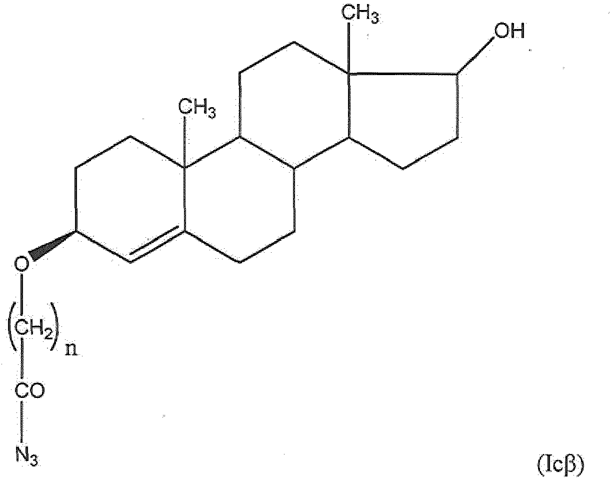 in which n is an integer between 1 and 10, comprising the steps consisting of:
(1) reacting testosterone with acetic anhydride to protect the OH function of position 17, and then with a reducing agent, in the presence of a solvent, to reduce the carbonyl in position 3 and obtain the compound of formula (IIβ): 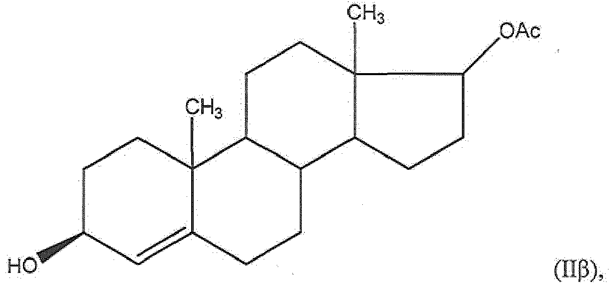 in which Ac means -CO-CH₃,
(2) reacting the compound of formula (IIβ) thus obtained with the compound of formula (III): N2-CH-(CH)ₙ₋₁COOC₂H₅, and then reacting with a base, in the presence of a solvent, to obtain the compound of formula (Iaβ): 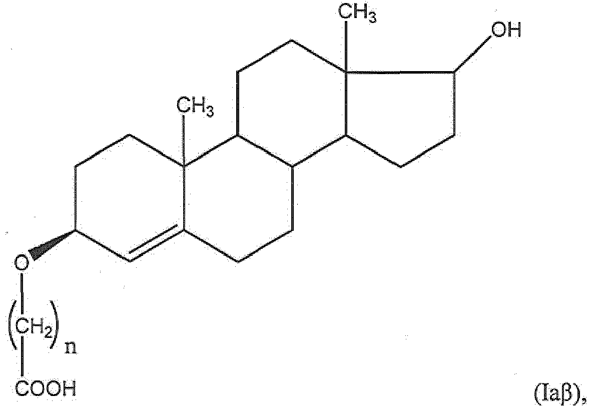
(3) reacting the compound of formula (Iaβ) thus obtained, in the presence of a carbodiimide derivative, with H₂NNH₂, to obtain the compound of formula (VIIβ): 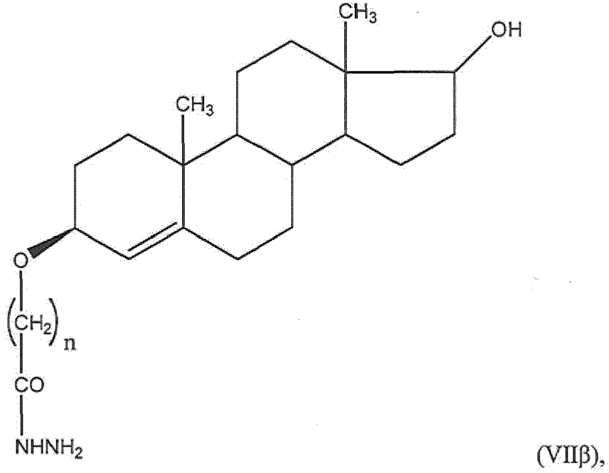
(4) reacting the compound of formula (VIIβ) thus obtained with HONO to obtain the compound of formula (Icβ).

19. A method for preparing a testosterone derivative, in the form of α isomer in position 3, of the following general formula (Iaα): 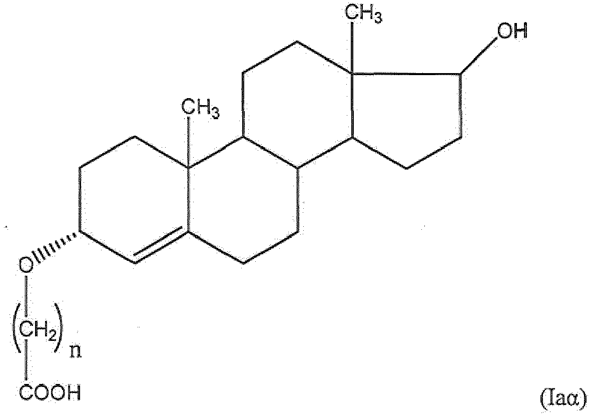 in which n is an integer between 1 and 10, comprising the steps consisting of:
(1) reacting testosterone with t-butyldimethylchlorosilane to protect the OH function of position 17, and then with a reducing agent, in the presence of a solvent, to reduce the carbonyl in position 3 and obtain the compound of formula (VIIIβ): 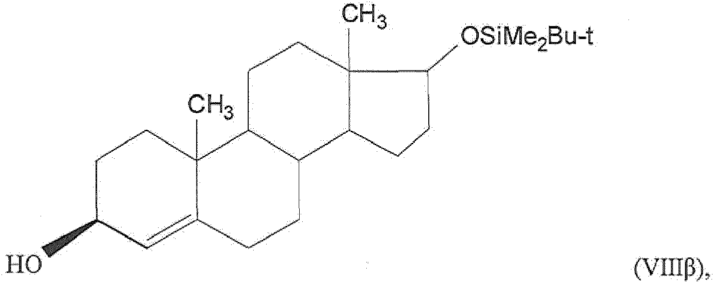 in which -SiMe₂Bu-t means t-butyldimethylsilanyl,
(2) reacting the compound of formula (VIIIβ) thus obtained with triphenylphosphine, benzoic acid and diethyl azodicarboxylate, and then with a base, in the presence of a solvent, to transform, in position 3, the β isomer to α isomer and thus obtain the compound of formula (VIIIα): 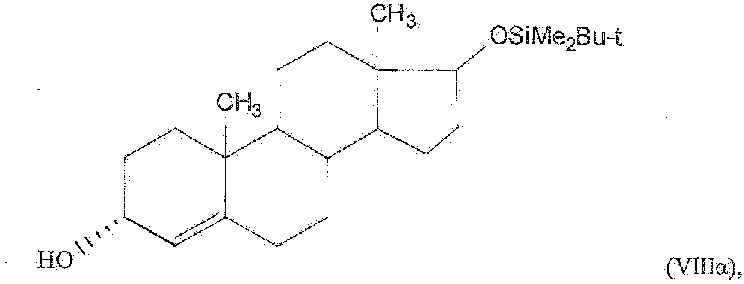 in which -SiMe₂Bu-t means t-butyldimethylsilanyl,
(3) reacting the compound of formula (VIIIα) thus obtained with the compound of formula (III): N₂CH-(CH)ₙ₋₁COOC₂H₅, and then reacting with a base, in the presence of a solvent, to obtain the compound of formula (Iaα).

20. A method for preparing a testosterone derivative, in the form of a isomer in position 3, of the following general formula (Ibα): 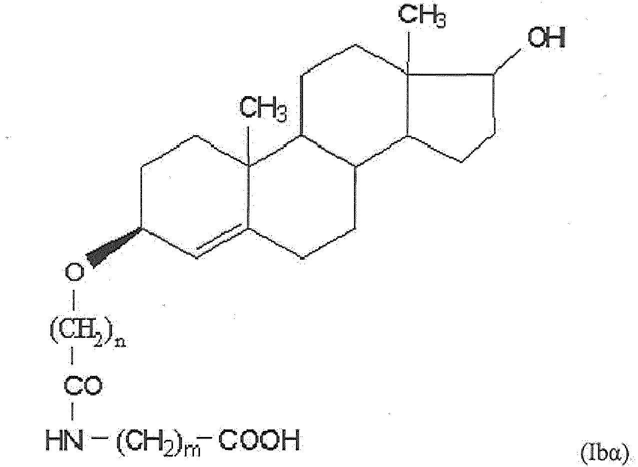 in which n is an integer between 1 and 10, m is an integer between 1 and 10, comprising the steps consisting of:
(1) reacting testosterone with t-butyldimethylchlorosilane to protect the OH function of position 17, and then with a reducing agent, in the presence of a solvent, to reduce the carbonyl in position 3 and obtain the compound of formula (VIIIβ): 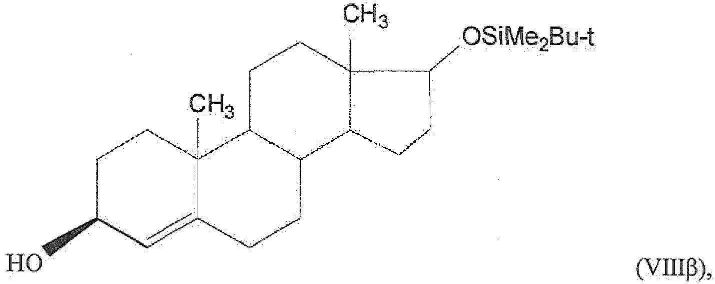 in which -SiMe₂Bu-t means t-butyldimethylsilanyl,
(2) reacting the compound of formula (VIIIβ) thus obtained with triphenylphosphine, benzoic acid and diethyl azodicarboxylate, and then with a base, in the presence of a solvent, to transform, in position 3, the β isomer to α isomer and thus obtain the compound of formula (VIIIα): 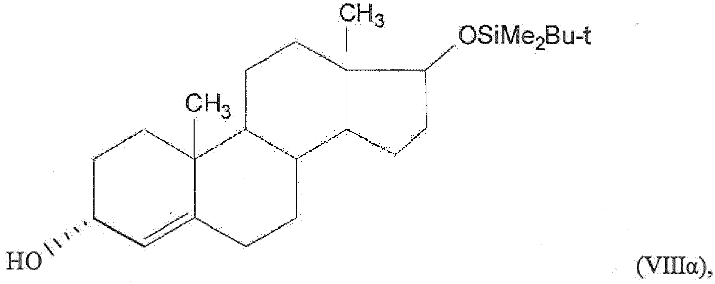 in which -SiMe₂Bu-t means t-butyldimetllylsrlanyl,
(3) reacting the compound of formula (VIIIα) thus obtained with the compound of formula (III): N₂CH-(CH₂)ₙ₋₁-COOC₂H₅, and then reacting with a base, in the presence of a solvent, to obtain the compound of formula (Iaα): 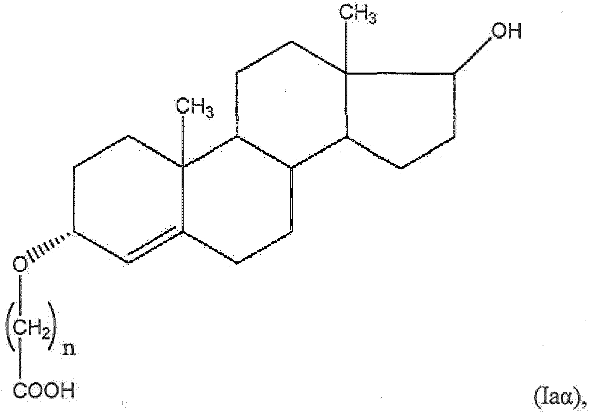
(4) reacting the compound of formula (Iaα) thus obtained with N-hydroxysuccinimide, in the presence of a carbodiimide derivative, to produce the compound of formula (IVα): 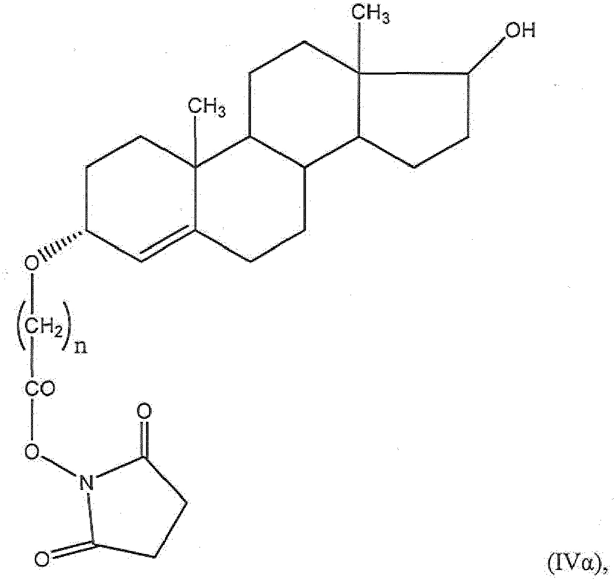
(5) reacting the compound of formula (IVα) thus obtained with the compound of formula (V): H₂N-(CH₂)m-COOR₁, in which R₁ is an alkyl or aryl group, to produce the compound of formula (VIα): 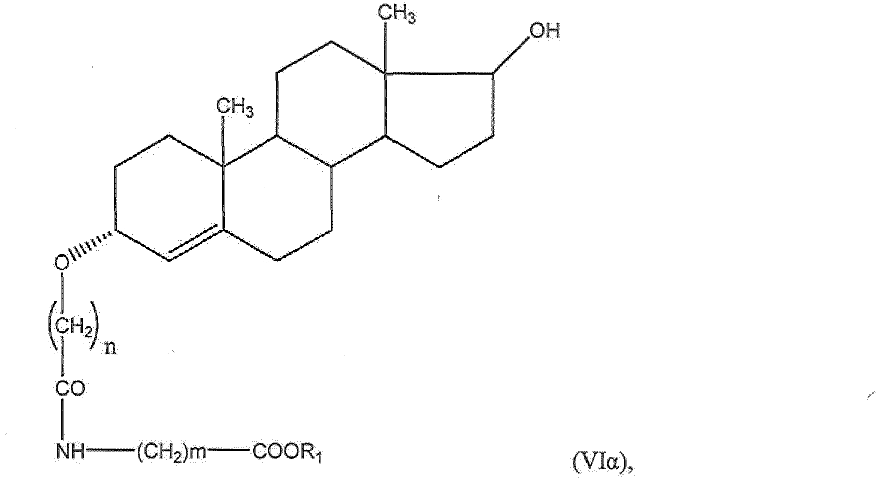
(6) reacting the compound of formula (VIα) thus obtained with a base, in the presence of a solvent, to obtain the compound of formula (Ibα).

21. A method for preparing a testosterone derivative, in the form of α isomer in position 3, of the following general formula (Iα): 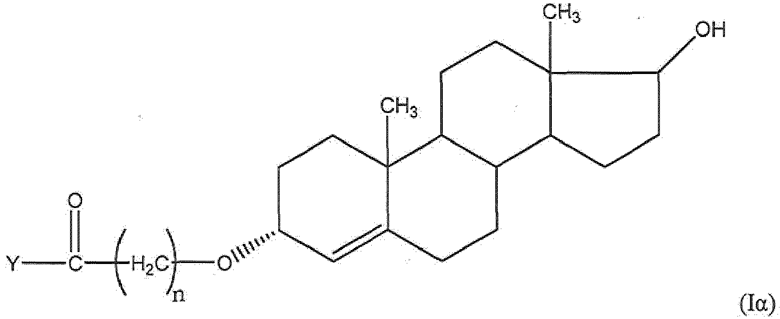 in which n is an integer between 1 and 10 and Y represents: 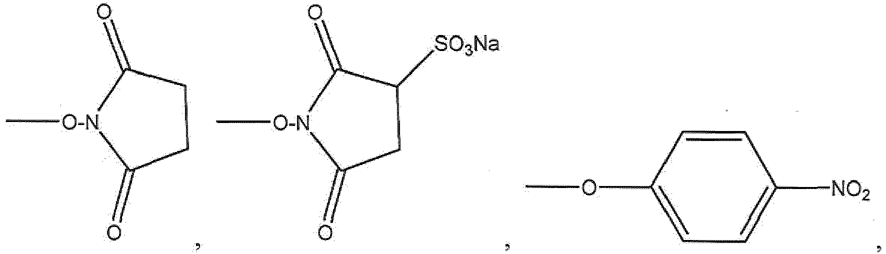 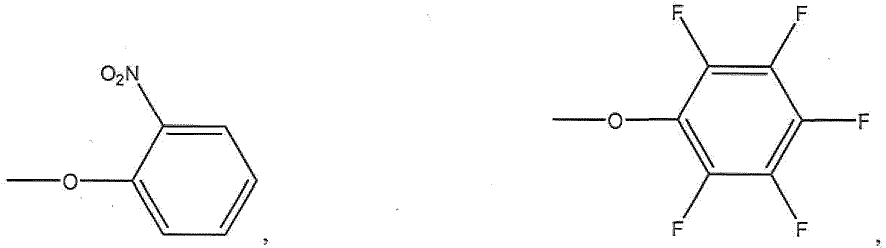 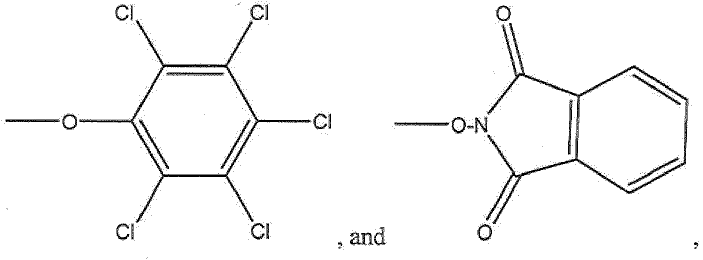 comprising the steps consisting of:
(1) reacting testosterone with t-butyldimethylchlorosilane to protect the OH function of position 17, and then with a reducing agent, in the presence of a solvent, to reduce the carbonyl in position 3 and obtain the compound of formula (VIIIβ): 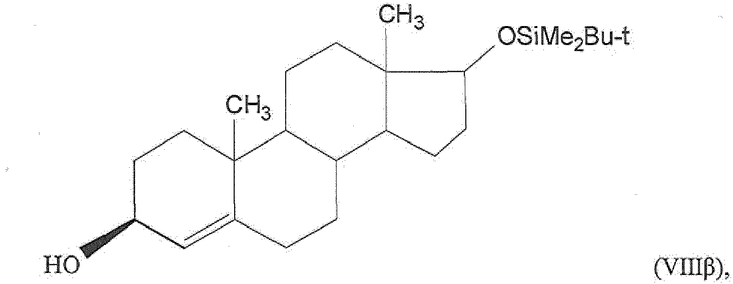 in which -SiMe₂Bu-t means t-butyldimethylsilanyl,
(2) reacting the compound of formula (VIIIβ) thus obtained with triphenylphosphine, benzoic acid and diethyl azodicarboxylate, and then with a base, in the presence of a solvent, to transform, in position 3, the β isomer to α isomer and thus obtain the compound of formula (VIIIα): 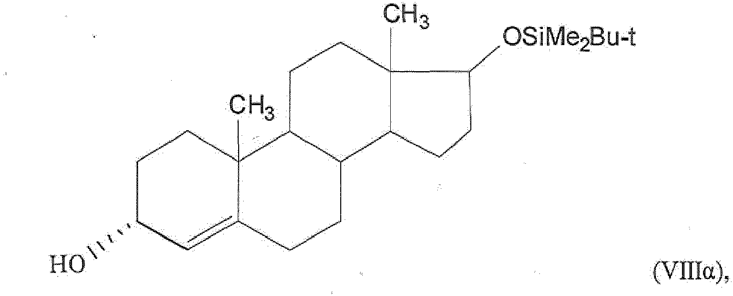 in which - SiMe₂Bu-t means t-butyldirnetlzylsilanyl,
(3) reacting the compound of formula (VIIIα) thus obtained with the compound of formula (III): N₂CH-(CH₂)_{n-I}-COOC₂H₅, and then reacting with a base, in the presence of a solvent, to obtain the compound of formula (Iaα): 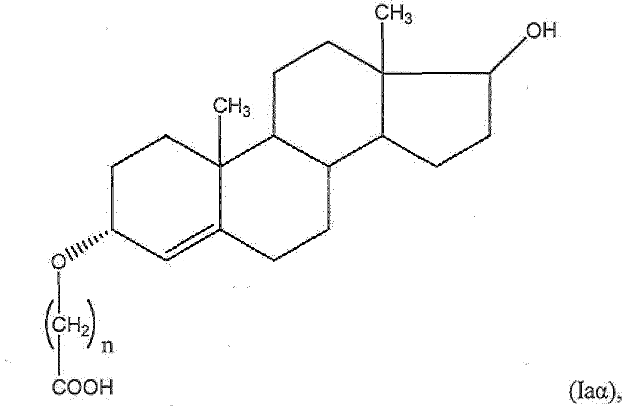
(4) reacting the compound of formula (Iaα) thus obtained, in the presence of a carbodiimide derivative, with a reagent selected, depending on the final group Y, from the following compounds, to obtain a compound of formula (Iα): 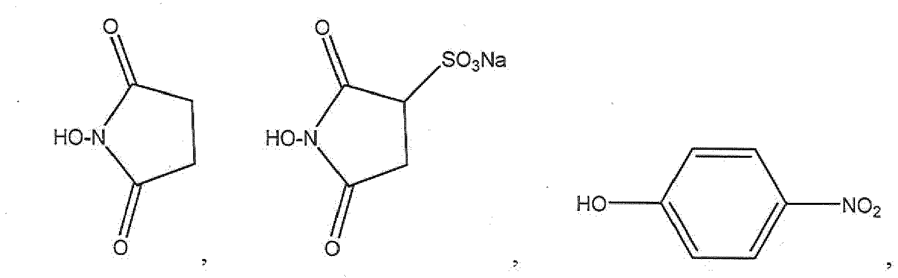 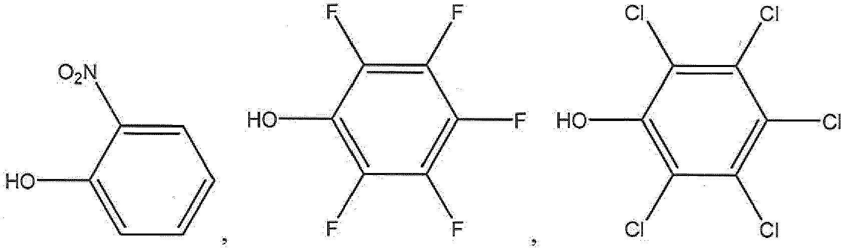 and

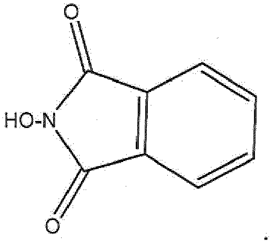

22. A method for preparing a testosterone derivative, in the form of α isomer in position 3, of the following general formula (Iα): 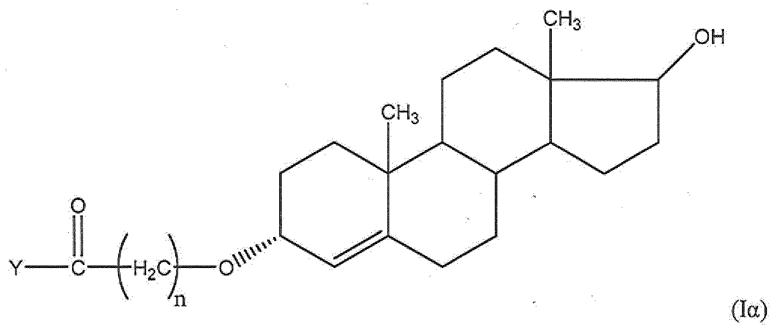 in which n is an integer between 1 and 10 and Y represents: 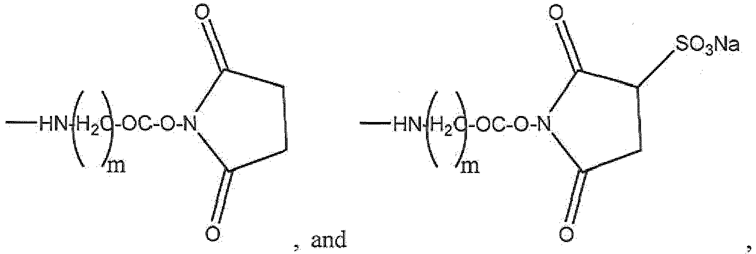 m being an integer between 1 and 10, comprising the steps consisting of:
(1) reacting testosterone with t-butyldimethylchlorosilane to protect the OH function of position 17, and then with a reducing agent, in the presence of a solvent, to reduce the carbonyl in position 3 and obtain the compound of formula (VIIIβ): 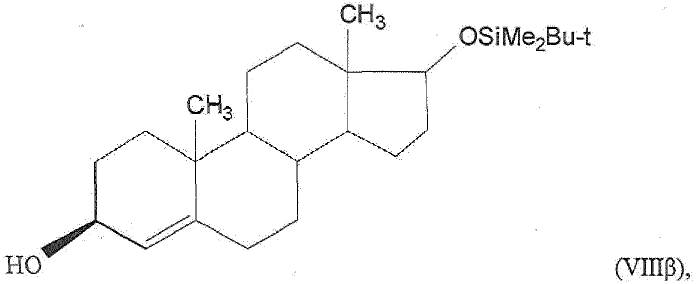 in which -SiMe₂Bu-t means t-butyldimethylsilanyl,
(2) reacting the compound of formula (VIIIβ) thus obtained with triphenylphosphine, benzoic acid and diethyl azodicarboxylate, and then with a base, in the presence of a solvent, to transform, in position 3, the β isomer to α isomer and thus obtain the compound of formula (VIIIα): 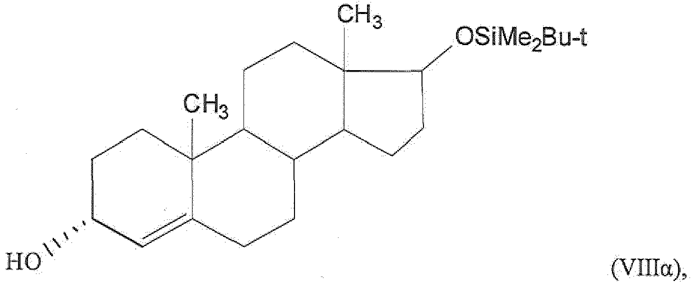 in which -SiMe₂Bu-t means t-butyldimethylsilanyl,
(3) reacting the compound of formula (VIIIα) thus obtained with the compound of formula (III): N₂CH-(CH₂)ₙ₋₁-COOC₂H₅, and then reacting with a base, in the presence of a solvent, to obtain the compound of formula (Iaα): 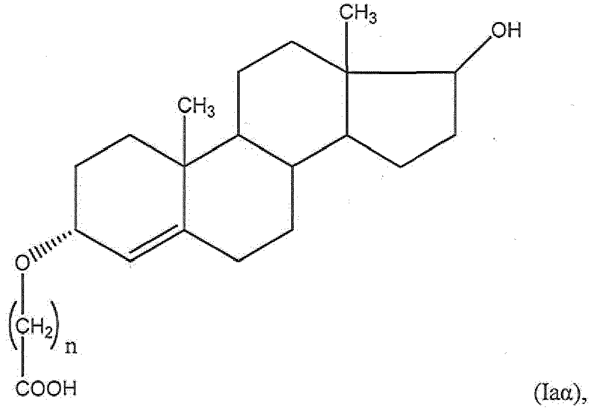
(4) reacting the compound of formula (Iaα) thus obtained with N-hydroxysuccinimide, in the presence of a carbodiimide derivative, to produce the compound of formula (IVα): 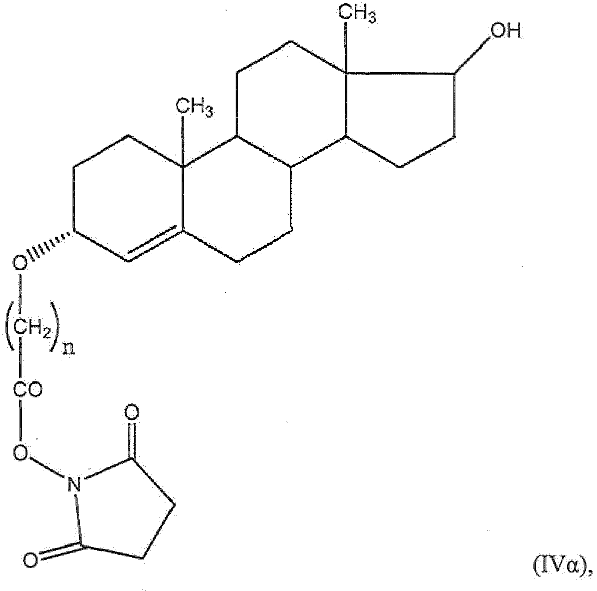
(5) reacting the compound of formula (IVα) thus obtained with the compound of formule (V): H₂N-(CH₂)m-COOR₁, in which R₁ is an alkyl or aryl group, to produce the compound of formula (VIα): 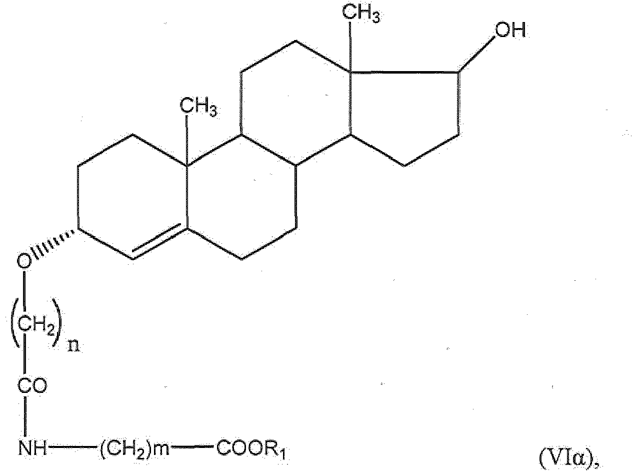
(6) reacting the compound of formula (VIα) thus obtained with a base, in the presence of a solvent, to obtain the compound of formula (Ibα): 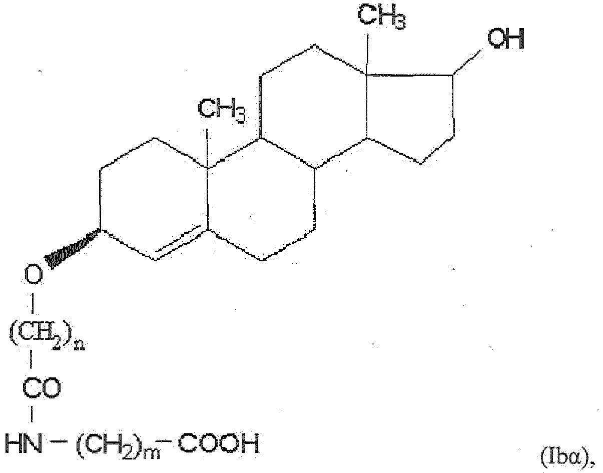
(7) reacting the compound of formula (Ibα) thus obtained, in the presence of a carbodiimide derivative, with a reagent selected, depending on the final group Y, from the following compounds, to obtain a compound of formula (Iα):

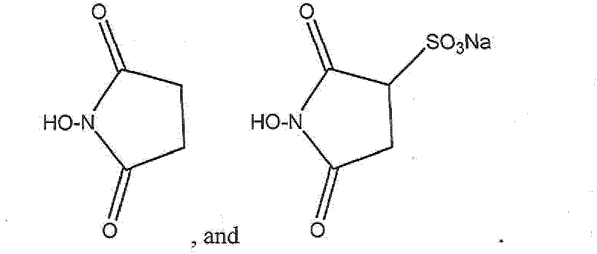

23. A method for preparing a testosterone derivative, in the form of α isomer in position 3, of the following general formula (Icα): 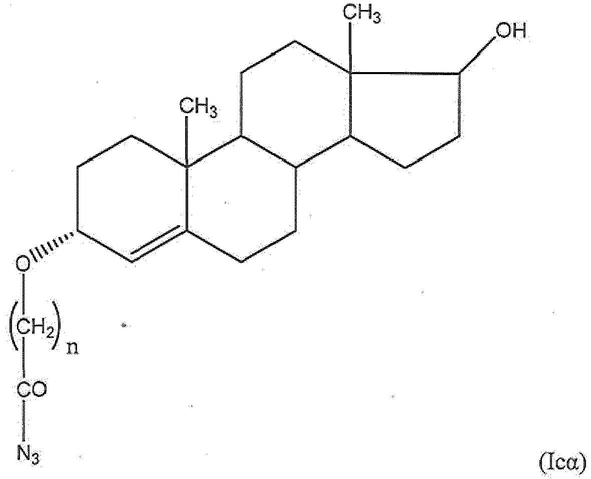 in which n is an integer between 1 and 10, comprising the steps consisting of:
(1) reacting testosterone with t-butyldimethylchlorosilane to protect the OH function of position 17, and then with a reducing agent, in the presence of a solvent, to reduce the carbonyl in position 3 and obtain the compound of formula (VIIIβ): 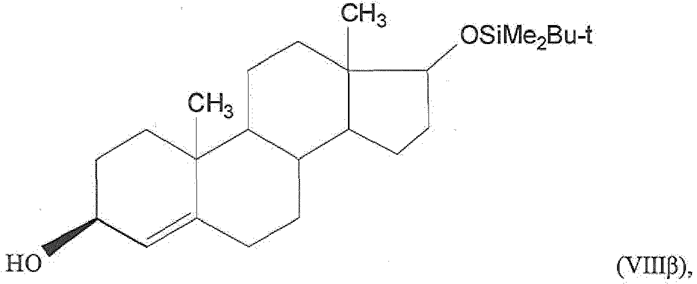 in which -SiMe₂Bu-t means t-butyldimethylsilanyl,
(2) reacting the compound of formula (VIIIβ) thus obtained with triphenylphosphine, benzoic acid and diethyl azodicarboxylate, and then with a base, in the presence of a solvent, to transform, in position 3, the β isomer to α isomer and thus obtain the compound of formula (VIIIα): 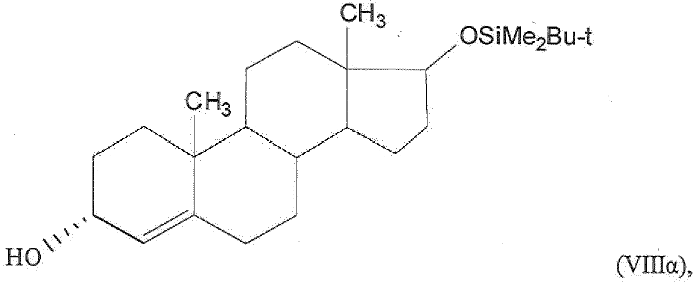 in which -SiMe₂Bu-t means t-butyldimethylsilanyl,
(3) reacting the compound of formula (VIIIα) thus obtained with the compound of formula (III): N₂CH-(CH₂)ₙ₋₁-COOC₂H₅, and then reacting with a base, in the presence of a solvent, to obtain the compound of formula (Iaα): 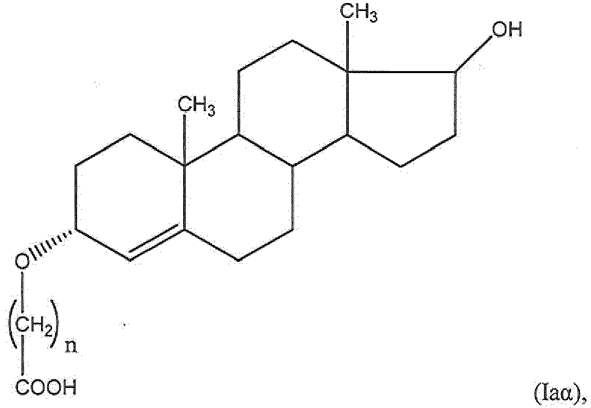
(4) reacting the compound of formula (Iaα) thus obtained, in the presence of a carbodiimide derivative, with H₂NNH₂, to obtain the compound of formula (VIIα): 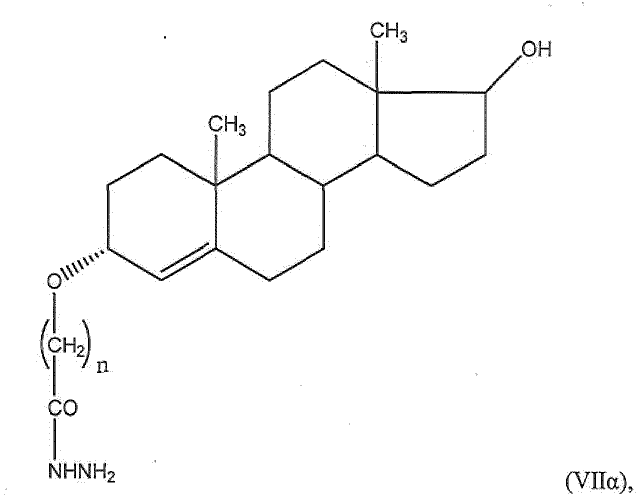
(5) reacting the compound of formula (VIIα) thus obtained with HONO to obtain the compound of formula (Icα).
